(19) Europäisches Patentamt
European Patent Office
Office européen des brevets

(11) EP 1 636 585 B2

(12) NEW EUROPEAN PATENT SPECIFICATION
After opposition procedure

(45) Date of publication and mention of the opposition decision:
**13.06.2012 Bulletin 2012/24**

(45) Mention of the grant of the patent:
**16.01.2008 Bulletin 2008/03**

(21) Application number: **04752642.1**

(22) Date of filing: **19.05.2004**

(51) Int Cl.:
***G01N 33/574*** *(2006.01)*

(86) International application number:
**PCT/US2004/015655**

(87) International publication number:
**WO 2004/113274 (29.12.2004 Gazette 2004/53)**

# (54) DIARYL UREAS WITH KINASE INHIBITING ACTIVITY

DIARYL-HARNSTOFFE MIT KINASEHEMMENDER WIRKUNG

DIARYL-UREES PRESENTANT UNE ACTIVITE D'INHIBITION DES KINASES

(84) Designated Contracting States:
**AT BE BG CH CY CZ DE DK EE ES FI FR GB GR HU IE IT LI LU MC NL PL PT RO SE SI SK TR**

(30) Priority: **20.05.2003 US 471735 P**
**17.11.2003 US 520399 P**
**25.03.2004 US 556062 P**

(43) Date of publication of application:
**22.03.2006 Bulletin 2006/12**

(73) Proprietor: **Bayer HealthCare LLC**
**Tarrytown, NY 10591 (US)**

(72) Inventors:
- **WILHELM, Scott**
**Orange, CT 06477 (US)**
- **DUMAS, Jacques**
**Bethany, CT 06524 (US)**
- **LADOUCEUR, Gaetan**
**Guilford, CT 06437 (US)**
- **LYNCH, Mark**
**Madison, CT 06443 (US)**
- **SCOTT, William, J.**
**Guilford, CT 06437 (US)**

(74) Representative: **Stolmár, Matthias et al**
**Stolmár Scheele & Partner**
**Patentanwälte**
**Blumenstrasse 17**
**80331 München (DE)**

(56) References cited:
**WO-A-02/25286**
**WO-A-03/068228**
**WO-A1-00//042012**

- **SCOTT W. ET AL.: 'BAY 43-9006: Preclinical Data' CURRENT PHARMACEUTICAL DESIGN vol. 8, 2002, pages 2255 - 2257**
- **T LEE J. ET AL.: 'BAY-43-9006 Bayer/Onyx' CURRENT OPINION IN INVESTIGATIONAL DRUGS vol. 4(6), 2003, ISSN 1472-4472 pages 757 - 763**
- **DEGRENDELE H. ET AL.: 'Activity of the Raf Kinase Inhibitor BAY 43-9006 in Patients with Advanced Solid Tumors' CLINICAL COLORECTAL CANCER May 2003, pages 16 - 18**
- **BHAGWAT S.V. ET AL.: 'The angiogenic regulator CD13/APN is a transcriptional target of Ras signaling pathways in endothelial morphogenesis' BLOOD vol. 101, no. 5, 01 March 2003, pages 1818 - 1826**
- **RAK J. ET AL.: 'Oncogenes as inducers of tumor angiogenesis' CANCER AND METASTASIS REVIEWS vol. 14, 1995, pages 263 - 277**
- **RAK J. ET AL.: 'Oncogenes and Tumor Angiogenesis: Differential Modes of Vascular Endothelial Growth Factor Up-Regulation in ras-transformed Epithelial Cells and Fibroblasts' CANCER RESEARCH vol. 60, 15 January 2000, pages 490 - 498**
- **RAK J. ET AL.: 'Oncogenes and Angiogenesis: Signaling Three-Dimensional Tumor Growth' THE SOCIETY FOR INVESTIGATIVE DERMATOLOGY, INC. vol. 5, 2000, pages 24 - 33**
- **KONERDING M.A. ET AL.: 'Vascular Endothelium, Source and Target of Inflammatory Mediators' IOS PRESS 2001, ISSN 1566-7693 pages 151 - 165**

EP 1 636 585 B2

## Description

This application claims the benefit of U.S. Provisional Application Nos. 60/556,062, filed March 25, 2004, 60/520,399, filed November 17, 2003, and 60/471,735, filed May 20, 2003, each of which are hereby incorporated by reference in their entirety.

## Background of the Invention

Activation of the ras signal transduction pathway indicates a cascade of events that have a profound impact on cellular proliferation, differentiation, and transformation. Raf kinase, a downstream effector of ras, is a key mediator of these signals from cell surface receptors to the cell nucleus (Lowy, D.R. and Willumsen, B. M. Ann. Rev. Biochem. 1993, 62, 851; Bos, J. L. Cancer Res. 1989, 49, 4682). It has been shown that inhibiting the effect of active ras by inhibiting the raf kinase signaling pathway by administration of deactivating antibodies to raf kinase or by co-expression of dominant negative raf kinase or dominant negative MEK, the substrate of raf kinase, leads to the reversion of transformed cells to the normal growth phenotype (e.g., Daum et al. Trends Biochem. Sci. 1994, 19, 474-80; Fridman et al. J. Biol. Chem. 1994, 269, 30105-8). Kolch et al. (Nature 1991, 349, 426-28) have further shown that inhibition of raf expression by antisense RNA blocks cell proliferation in membrane-associated oncogenes. Similarly, inhibition of raf kinase (by antisense oligodeoxynucleotides) has been correlated in vitro and in vivo with inhibition of the growth of a variety of human tumor types (Monia et al., Nat. Med. 1996, 2, 668-75). Thus, small molecule inhibitors of Raf kinase activity are important agents for the treatment of cancer (Naumann, U.; Eisenmann-Tappe, I.; Rapp, U. R. Recent Results Cancer Res. 1997, 143, 237; Monia, B. P.; Johnston, J. F.; Geiger, T.; Muller, M.; Fabbro, D. Nature Medicine 1996, 2, 668).

To support progressive tumor growth beyond the size of 1-2 $mm^3$, tumor cells require a functional stroma, a support structure consisting of fibroblast, smooth muscle cells, endothelial cells, extracellular matrix proteins, and soluble factors (Folkman, J., Semin Oncol, 2002. 29(6 Suppl 16), 15-8). Tumors induce the formation of stromal tissues through the secretion of soluble growth factor such as PDGF and transforming growth factor-beta (TGF-beta), which in turn stimulate the secretion of complimentary factors by host cells such as fibroblast growth factor (FGF), epidermal growth factor (EGF), and vascular endothelial growth factor (VEGF). These stimulatory factors induce the formation of new blood vessels, or angiogenesis, which brings oxygen and nutrients to the tumor and allows it to grow and provides a route for metastasis.

There is a need of developing a novel agent with pluripotent activity against a number of key signaling pathways utilized by tumors to induce angiogenesis in the host stroma. These include PDGF, a potent stimulator of stroma formation (Ostman, A. and C.H. Heldin, Adv Cancer Res, 2001, 80, 1-38), FGF, a chemo-attractant and mitogen for fibroblasts and endothelial cells, and VEGF, a potent regulator of vascularization.

One of the key regulators of stromal formation is PDGF, which is secreted by many tumors in a paracrine fashion and promotes the growth of fibroblasts, smooth muscle and endothelial cells, promoting stroma formation and angiogenesis. PDGF was originally identified as the v-sis oncogene product of the simian sarcoma virus (Heldin, C.H., et al., J Cell Sci Suppl, 1985, 3, 65-76). The growth factor is made up of two peptide chains, referred to as A or B chains which share 60% homology in their primary amino acid sequence. The chains are disulfide cross linked to form the 30 kDa mature protein composed of either AA, BB or AB homo- or heterodimmers. PDGF is found at high levels in platelets, and is expressed by endothelial cells and vascular smooth muscle cells. In addition, the production of PDGF is up regulated under low oxygen conditions such as those found in poorly vascularized tumor tissue (Kourembanas, S., et al., Kidney Int, 1997, 51(2), 438-43). PDGF binds with high affinity to the PDGF receptor, a 1106 amino acid 124 kDa transmembrane tyrosine kinase receptor (Heldin, C.H.; A. Ostman, and L. Ronnstrand, Biochim Biophys Acta, 1998. 1378(1), 79-113). PDGFR is found as homo- or heterodimer chains which have 30% homology overall in their amino acid sequence and 64% homology between their kinase domains (Heldin, C.H., et al.. Embo J, 1988, 7(5), 1387-93). PDGFR is a member of a family of tyrosine kinase receptor with split kinase domains that includes VEGFR2 (KDR), c-Kit, and FLT3 which have all been found to have a role in promoting tumor angiogenesis, growth and survival. The PDGF receptor is expressed primarily on fibroblast, smooth muscle cells, and pericytes and to a lesser extent on neurons, kidney mesangial, Leydig, and Schwann cells of the central nervous system. Upon binding to the receptor, PDGF induces receptor dimerization and undergoes auto- and trans-phosphorylation of tyrosine residues which increase the receptors' kinase activity and promotes the recruitment of downstream effectors through the activation of SH2 protein binding domains. A number of signaling molecules form complexes with activated PDGFR including PI-3-kinase, phospholipase C-gamma, src and GAP (GTPase activating protein for p21-ras) (Soskic, V., et al. Biochemistry, 1999, 38(6), 1757-64). Through the activation of PI-3-kinase, PDGF activates the Rho signaling pathway inducing cell motility and migration, and through the activation of GAP, induces mitogenesis through the activation of p21-ras and the MAPK signaling pathway.

In adults, the major function of PDGF is to facilitate and increase the rate of wound healing and to maintain blood vessel homeostasis (Baker, E.A. and D.J. Leaper, Wound Repair Regen, 2000. 8(5), 392-8; Yu, J., A. Moon, and

H.R. Kim, Biochem Biophys Res Commun, 2001. 282(3), 697-700). PDGF is found at high concentrations in platelets and is a potent chemoattractant for fibroblast, smooth muscle cells, neutrophils and macrophages. In addition to its role in wound healing PDGF helps maintain vascular homeostasis. During the development of new blood vessels, PDGF recruits pericytes and smooth muscle cells that are needed for the structural integrity of the vessels. PDGF is thought to play a similar role during tumor neovascularization. As part of its role in angiogenesis PDGF controls interstitial fluid pressure, regulating the permeability of vessels through its regulation of the interaction between connective tissue cells and the extracellular matrix. Inhibiting PDGFR activity can lower interstitial pressure and facilitate the influx of cytotoxics into tumors improving the anti-tumor efficacy of these agents (Pietras, K., et al. Cancer Res, 2002. 62(19), 5476-84; Pietras, K., et al. Cancer Res, 2001. 61(7), 2929-34).

PDGF can promote tumor growth through either the paracrine or autocrine stimulation of PDGFR receptors on stromal cells or tumor cells directly, or through the amplification of the receptor or activation of the receptor by recombination. Over expressed PDGF can transform human melanoma cells and keratinocytes (Forsberg, K., et al. Proc Natl Acad Sci USA., 1993. 90(2), 393-7; Skobe, M. and N.E. Fusenig, Proc Natl Acad Sci USA, 1998. 95(3), 1050-5), two cell types that do not express PDGF receptors, presumably by the direct effect of PDGF on stroma formation and induction of angiogenesis. This paracrine stimulation of tumor stroma is also observed in carcinomas of the colon, lung, breast, and prostate (Bhardwaj, B., et al. Clin Cancer Res, 1996, 2(4), 773-82; Nakanishi, K., et al. Mod Pathol, 1997, 10(4), 341-7; Sundberg, C., et al. Am J Pathol, 1997, 151(2), 479-92; Lindmark, G., et al. Lab Invest, 1993, 69 (6), 682-9; Vignaud, J.M., et al, Cancer Res, 1994, 54(20), 5455-63) where the tumors express PDGF, but not the receptor. The autocrine stimulation of tumor cell growth, where a large faction of tumors analyzed express both the ligand PDGF and the receptor, has been reported in glioblastomas (Fleming, T.P., et al. Cancer Res, 1992, 52(16), 4550-3), soft tissue sarcomas (Wang, J., M.D. Coltrera, and A.M. Gown, Cancer Res, 1994, 54(2), 560-4) and cancers of the ovary (Henriksen, R., et al. Cancer Res, 1993, 53(19), 4550-4), prostate (Fudge, K., C.Y. Wang, and M.E. Stearns, Mod Pathol, 1994, 7(5), 549-54), pancreas (Funa, K., et al. Cancer Res, 1990, 50(3), 748-53) and lung (Antoniades, H.N., et al., Proc Natl Acad Sci USA, 1992, 89(9), 3942-6). Ligand independent activation of the receptor is found to a lesser extent but has been reported in chronic myelomonocytic leukemia (CMML) where a chromosomal translocation event forms a fusion protein between the Ets-like transcription factor TEL and the PDGF receptor. In addition, activating mutations in PDGFR have been found in gastrointestinal stromal tumors in which c-Kit activation is not involved (Heinrich; M.C., et al., Science, 2003, 9, 9). PDGFR inhibitors will interfere with tumor stromal development and inhibit tumor growth and metastasis without undue side effects. Additional factors such as VEGF and FGF, secreted by stromal cells in response to tumor secreted PDGF, play key roles in stromal formation, angiogenesis and tumor progression.

Another major regulator of angiogenesis and vasculogenesis in both embryonic development and some angiogenic-dependent diseases is vascular endothelial growth factor (VEGF; also called vascular permeability factor, VPF). VEGF represents a family of isoforms of mitogens existing in homodimeric forms due to alternative RNA splicing. The VEGF isoforms are highly specific for vascular endothelial cells (for reviews, see: Farrara et al. Endocr. Rev. 1992, 13, 18; Neufield et al. FASEB J. 1999, 13, 9).

VEGF expression is induced by hypoxia (Shweiki et al. Nature 1992, 359, 843), as well as by a variety of cytokines and growth factors, such as interleukin-1, interleukin-6, epidermal growth factor and transforming growth factor. To date, VEGF and the VEGF family members have been reported to bind to one or more of three transmembrane receptor tyrosine kinases (Mustonen et al. J. Cell Biol., 1995, 129, 895), VEGF receptor-1 (also known as flt-1 (fms-like tyrosine kinase-1)), VEGFR-2 (also known as kinase insert domain containing receptor (KDR); the murine analogue of KDR is known as fetal liver kinase-1 (flk-1)), and VEGFR-3 (also known as flt-4). KDR and flt-1 have been shown to have different signal transduction properties (Waltenberger et al. J. Biol. Chem. 1994, 269, 26988); Park et al. Oncogene 1995, 10, 135). Thus, KDR undergoes strong ligand-dependant tyrosine phosphorylation in intact cells, whereas flt-1 displays a weak response. Thus, binding to KDR is a critical requirement for induction of the full spectrum of VEGF-mediated biological responses.

*In vivo,* VEGF plays a central role in vasculogenesis, and induces angiogenesis and permeabilization of blood vessels. Deregulated VEGF expression contributes to the development of a number of diseases that are characterized by abnormal angiogenesis and/or hyperpermeability processes. Regulation of the VEGF-mediated signal transduction cascade will therefore provide a useful mode for control of abnormal angiogenesis and/or hyperpermeability processes.

Angiogenesis is regarded as an absolute prerequisite for growth of tumors beyond about 1-2 mm. Oxygen and nutrients may be supplied to cells in tumor smaller than this limit through diffusion. However, every tumor is dependent on angiogenesis for continued growth after it has reached a certain size. Tumorigenic cells within hypoxic regions of tumors respond by stimulation of VEGF production, which triggers activation of quiescent endothelial cells to stimulate new blood vessel formation. (Shweiki et al. Proc. Nat'l. Acad. Sci., 1995, 92, 768). In addition, VEGF production in tumor regions where there is no angiogenesis may proceed through the ras signal transduction pathway (Grugel et al. J. Biol. Chem., 1995, 270, 25915; Rak et al. Cancer Res. 1995, 55, 4575). In situ hybridization studies have demonstrated VEGF mRNA is strongly upregulated in a wide variety of human tumors, including lung (Mattern et al. Br. J. Cancer 1996, 73, 931), thyroid (Viglietto et al. Oncogene 1995, 11, 1569), breast (Brown et al. Human Pathol. 1995, 26, 86),

gastrointestional tract (Brown et al. Cancer Res. 1993, 53, 4727; Suzuki et al. Cancer Res. 1996, 56, 3004), kidney and bladder (Brown et al. Am. J. Pathol. 1993, 1431, 1255), ovary (Olson et al. Cancer Res. 1994, 54, 1255), and cervical (Guidi et al. J. Nat'l Cancer Inst. 1995, 87, 12137) carcinomas, as well as angiosacroma (Hashimoto et al. Lab. Invest. 1995, 73, 859*)* and several intracranial tumors (Plate et al. Nature 1992, 359, 845; Phillips et al. Int. J. Oncol. 1993, 2, 913; Berkman et al. J. Clin. Invest., 1993, 91, 153). Neutralizing monoclonal antibodies to KDR have been shown to be efficacious in blocking tumor angiogenesis (Kim et al. Nature 1993, 362, 841; Rockwell et al. Mol. Cell. Differ. 1995, 3, 315).

Overexpression of VEGF, for example under conditions of extreme hypoxia, can lead to intraocular angiogenesis, resulting in hyperproliferation of blood vessels, leading eventually to blindness. Such a cascade of events has been observed for a number of retinopathies, including diabetic retinopathy, ischemic retinal-vein occlusion, and retinopathy of prematurity (Aiello et al. New Engl. J. Med. 1994, 331, 1480; Peer et al. Lab. Invest. 1995, 72, 638), and age-related macular degeneration (AMD; see, Lopez et al. Invest. Opththalmol. Vis. Sci. 1996, 37, 855).

In rheumatoid arthritis (RA), the in-growth of vascular pannus may be mediated by production of angiogenic factors. Levels of immunoreactive VEGF are high in the synovial fluid of RA patients, while VEGF levels were low in the synovial fluid of patients with other forms of arthritis of with degenerative joint disease (Koch et al. J. Immunol. 1994, 152, 4149). The angiogenesis inhibitor AGM-170 has been shown to prevent neovascularization of the joint in the rat collagen arthritis model (Peacock et al. J. Exper. Med. 1992, 175, 1135).

Increased VEGF expression has also been shown in psoriatic skin, as well as bullous disorders associated with subepidermal blister formation, such as bullous pemphigoid, erythema multiforme, and dermatitis herpetiformis (Brown et al. J. Invest. Dermatol. 1995, 104, 744).

The vascular endothelial growth factors (VEGF, VEGF-C, VEGF-D) and their receptors (VEGFR2, VEGFR3) are not only key regulators of tumor angiogenesis, but also lymphangiogenesis. VEGF, VEGF-C and VEGF-D are expressed in most tumors, primarily during periods of tumor growth and, often at substantially increased levels. VEGF expression is stimulated by hypoxia, cytokines, oncogenes such as *ras,* or by inactivation of tumor suppressor genes (McMahon, G. Oncologist 2000, 5(Suppl. 1), 3-10; McDonald, N.Q.; Hendrickson, W.A. Cell 1993, 73,421-424)

The biological activities of the VEGFs are mediated through binding to their receptors. VEGFR3 (also called flt-4) is predominantly expressed on lymphatic endothelium in normal adult tissues. VEGFR3 function is needed for new lymphatic vessel formation, but not for maintenance of the pre-existing lymphatics. VEGFR3 is also upregulated on blood vessel endothelium in tumors. Recently VEGF-C and VEGF-D, ligands for VEGFR3, have been identified as regulators of lymphangiogenesis in mammals. Lymphangiogenesis induced by tumor-associated lymphangiogenic factors could promote the growth of new vessels into the tumor, providing tumor cells access to systemic circulation. Cells that invade the lymphatics could find their way into the bloodstream via the thoracic duct. Tumor expression studies have allowed a direct comparison of VEGF-C, VEGF-D and VEGFR3 expression with clinicopathological factors that relate directly to the ability of primary tumors to spread (e.g., lymph node involvement, lymphatic invasion, secondary metastases, and disease-free survival). In many instances, these studies demonstrate a statistical correlation between the expression of lymphangiogenic factors and the ability of a primary solid tumor to metastasize (Skobe, M. et al. Nature Med. 2001, 7(2), 192-198; Stacker, S.A. et al.. Nature Med. 2001, 7(2), 186-191; Makinen, T. et al. Nature Med. 2001, 7(2), 199-205; Mandriota, S.J. et al. EMBO J. 2001, 20(4), 672-82; Karpanen, T. et al. Cancer Res. 2001, 61 (5), 1786-90; Kubo, H. et al. Blood 2000, 96(2), 546-53).

Hypoxia appears to be an important stimulus for VEGF production in malignant cells. Activation of p38 MAP kinase is required for VEGF induction by tumor cells in response to hypoxia (Blaschke, F. et al. Biochem. Biophys. Res. Commun. 2002, 296, 890-896; Shemirani, B. et al. Oral Oncology 2002, 38, 251-257). In addition to its involvement in angiogenesis through regulation of VEGF secretion, p38 MAP kinase promotes malignant cell invasion, and migration of different tumor types through regulation of collagenase activity and urokinase plasminogen activator expression (Laferriere, J. et al. J. Biol. Chem. 2001, 276, 33762-33772; Westermarck, J. et al. Cancer Res. 2000, 60, 7156-7162; Huang, S. et al. J. Biol. Chem. 2000, 275, 12266-12272; Simon, C. et al. Exp. Cell Res. 2001, 271, 344-355). Therefore, inhibition of p38 kinase is expected to impact tumor growth by interfering with signaling cascades associated with both angiogenesis and malignant cell invasion.

Diarylureas are a class of serine-threonine kinase inhibitors as well as tyrosine kinase inhibitors well known in the art. The following publications illustrate their utility as active ingredient in pharmaceutical compositions for the treatment of cancer, angiogenesis disorders, and inflammatory disorders:

Redman et al., Bioorg. Med. Chem. Lett. 2001, 11, 9-12.
Smith et al., Bioorg. Med. Chem. Lett. 2001, 11, 2775-2778.
Dumas et al., Bioorg. Med. Chem. Lett. 2000, 10, 2047-2050.
Dumas et al., Bioorg. Med. Chem. Lett. 2000, 10, 2051-2054.
Ranges et al., Book of Abstracts, 220th ACS National Meeting, Washington, DC, USA, MEDI 149.
Dumas et al., Bioorg. Med. Chem. Lett. 2002, 12, 1559-1562.
Lowinger et al., Clin. Cancer Res. 2000, 6(suppl.), 335.

Lyons et al., Endocr.-Relat. Cancer 2001, 8, 219-225.
Riedl et al., Book of Abstracts, 92nd AACR Meeting, New Orleans, LA, USA, abstract 4956.
Khire et al., Book of Abstracts, 93rdAACR Meeting, San Francisco, CA, USA, abstract 4211.
Lowinger et al., Curr. Pharm. Design 2002, 8, 99-110.
Regan et al., J. Med. Chem. 2002, 45, 2994-3008.
Pargellis et al., Nature Struct. Biol. 2002, 9(4), 268-272.
Carter et al., Book of Abstracts ; 92ndAACR Meeting, New Orleans, LA, USA, abstract 4954.
Vincent et al., Book of Abstracts, 38th ASCO Meeting, Orlando, FL, USA, abstract 1900.
Hilger et al., Book of Abstracts, 38th ASCO Meeting, Orlando, FL, USA, abstract 1916.
Moore et al., Book of Abstracts, 38th ASCO Meeting, Orlando, FL, USA, abstract 1816.
Strumberg et al., Book ofAbstracts, 38th ASCO Meeting, Orlando, FL, USA, abstract 121.
Madwed JB: Book of Abstracts, Protein Kinases: Novel Target Identification and Validation for Therapeutic Development, San Diego, CA, USA, March 2002.
Roberts et al., Book ofAbstracts, 38th ASCO Meeting, Orlando, FL, USA, abstract 473.
Tolcher et al., Book ofAbstracts, 38th ASCO Meeting, Orlando, FL, USA, abstract 334.
Karp et al., Book of Abstracts, 38th AACR Meeting, San Francisco, CA, USA, abstract 2753.

## Brief Description of the Drawings

Fig. 1. Qualitative assessment consisted of assessing the staining intensity, identifying the positively staining cells and the intracellular compartments involved in staining, and evaluating the overall slide quality. Separate evaluations were performed on the tumor cells and the tumor cell stroma. Intensity of staining was graded based upon the following scale: Negative, ± (equivocal), 1+ (weak), 2+ (moderate), 3+ (strong), 4+ (intense). The intensity of staining was determined by evaluation of the entire specimen. Semi-quantitative evaluation of target antigen expression was conducted using a grading system based upon the percentage of cells throughout the entire specimen expressing each target antigen as follows: 0-5% with positive target antigen expression = <5%; 6-25% = 1st quartile (1Q); 26-50% = 2nd quartile (2Q); 51-75% = 3rd quartile (3Q); 76-100% = 4th quartile (4Q).

Fig. 2. Immunohistochemical Staining for phospho-ERK of Human Melanoma Biopsies pre- and post-treament.

## Description of the Invention

The present invention provides a method of assessing the efficacy of specific compounds in treating a disease in a mammalian subject, or a cell derived therefrom which is associated with signal transduction pathways comprising, VEGFR, PDGFR, and/or FLT-3. Preferred methods of the present invention provide for the modulation of diseases and conditions associated with VEGFR2, VEGPR3, and/or PDGFR-beta. The method can comprise, e.g., administering of an aryl urea compound as described below, pharmaceutically-acceptable salts thereof, derivatives thereof, etc.

The present invention also provides methods for identifying conditions and diseases which can be modulated with compounds of the present invention. These methods facilitate the selection of subjects who can be efficiently treated with specific compounds. Additionally, the invention provides methods for monitoring subjects who have been administered a compound . This includes, e.g., determining the efficacy of compounds in the treatment of various diseases and conditions, and for determining treatment regimens.

The aryl urea compounds whose efficacy is to be assessed a comprise compounds of Formula I, pharmaceutically acceptable salts thereof, esters thereof, stereoisomers thereof (both isolated and in mixtures), prodrugs thereof, and any active derivatives thereof, which are collectively referred to herein as the "compounds of the invention" and the like.

Formula I is as follows:

$$B\text{-}NH\text{-}C(O)\text{-}NH\text{-}L\text{-}M\text{-}L^1\text{-}(Q)_{1\text{-}3} \qquad (I)$$

wherein B is

(i) phenyl, optionally substituted with 1-3 substituents independently selected from the group consisting of $R^1$, $OR^1$, $NR^1R^2$, $S(O)qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, halogen, cyano; and nitro;

(ii) naphthyl, optionally substituted with 1-3 substituents independently selected from the group consisting of $R^1$, $OR^1$, $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, halo-

gen, cyano, and nitro;

(iii) a 5 or 6 membered monocyclic heteroaryl group, having 1-3 heteroatoms independently selected from the group consisting of O, N and S, optionally substituted with 1-3 substituents independently selected from the group consisting of $R^1$, $OR^1$, $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, halogen, cyano, oxo, and nitro; or

(iv) an 8 to 10 membered bicyclic heteroaryl group in which the first ring is bonded to the NH of Figure 1 and contains 1-3 heteroatoms independently selected from the group consisting of O, N, and S; and the second ring is fused to the first ring using 3 to 4 carbon atoms. The bicyclic heteroaryl group is optionally substituted with 1-3 substituents independently selected from the group consisting of $R^1$, $OR^1$, $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, halogen, cyano, oxo, and nitro.

L is

(i) phenyl, optionally substituted with 1-3 substituents independently selected from the group consisting of $C_1$-$C_5$ linear or branched alkyl, $C_1$-$C_5$ linear or branched haloalkyl, $C_1$-$C_3$ alkoxy, hydroxy, amino, $C_1$-$C_3$ alkylamino, $C_1$-$C_6$ dialkylamino, halogen, cyano, and nitro;

(ii) naphthyl, optionally substituted with 1-3 substituents independently selected from the group consisting of $C_1$-$C_5$ linear or branched alkyl, $C_1$-$C_5$linear or branched haloalkyl, $C_1$-$C_3$ alkoxy, hydroxy, amino, $C_1$-$C_3$ alkylamino, $C_1$-$C_6$ dialkylamino, halogen, cyano, and nitro;

(iii) a 5 or 6 membered monocyclic heteroaryl group, having 1-3 heteroatoms independently selected from the group consisting of O, N and S, optionally substituted with 1-3 substituents independently selected from the group consisting of $C_1$-$C_5$ linear or branched alkyl, $C_1$-$C_5$ linear or branched haloalkyl, $C_1$-$C_3$ alkoxy, hydroxy, amino, $C_1$-$C_3$ alkylamino, $C_1$-$C_6$ dialkylamino, halogen, cyano, and nitro; or

(iv) an 8 to 10 membered bicyclic heteroaryl group having 1-6 heteroatoms independently selected from the group consisting of O, N and S, optionally substituted with 1-3 substituents independently selected from the group consisting of $C_1$-$C_5$ linear or branched alkyl, $C_1$-$C_5$ linear or branched haloalkyl, $C_1$-$C_3$ alkoxy, hydroxy, amino, $C_1$-$C_3$ alkylamino, $C_1$-$C_6$ dialkylamino, halogen, cyano, and nitro.

M is

(a) $-(CH_2)_m-O-(CH_2)_1-$,
(b) $-(CH_2)_m-(CH_2)_1-$,
(c) $-(CH_2)_m-C(O)-(CH_2)_1-$,
(d) $-(CH_2)_m-NR^3-(CH_2)_1$,
(e) $-(CH_2)_m-$ $NR^3C(O)-(CH_2)_1-$,
(f) $-(CH_2)_m-S-(CH_2)_1-$,
(g) $-(CH_2)_m-C(O)NR^3$ $-(CH_2)_1-$,
(h) $-(CH_2)_m-CF_2-(CH_2)_1-$,
(i) $-(CH_2)_m-CCl_2-(CH_2)_1-$,
G) $-(CH_2)_m-CHF-(CH_2)_1-$,
(k) $-(CH_2)_m-CH(OH)-(CH_2)_1-$;
(l) $-(CH_2)_m-C{\equiv}C-(CH_2)_1-$;
(m) $-(CH_2)_m-C=C-(CH_2)_1-$;
(n) $-(CH_2)_m-CR^4R^5-(CH_2)_1-$;
or
(o) a single bond, where m and 1 are 0;

wherein the variables m and l are integers independently selected from 0-4,
L' is

(i) phenyl, optionally substituted with 1-2 additional substituents other than Q, independently selected from the group consisting of $R^1$, $OR^1$, $NR^1R^2$, $S(O)_qR^1$, $SO^2NR^1R^2$, $NR^1SO_2R^2$, $NR^1C(O)R^2$, $NR^1C(O)OR^2$, halogen, cyano and nitro;

(ii) naphthyl, optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogen, cyano and nitro;

(iii) a 5 and 6 membered monocyclic heteroaryl group, having 1-3 heteroatoms independently selected from the group consisting of O, N and S, optionally substituted with 1-2 additional substituents other than Q, independently

selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogen, cyano and nitro and also oxides (e.g. =O, -O⁻ or -OH);

(iv) an 8 to 10 membered bicyclic heteroaryl group; having 1-6 heteroatoms independently selected from the group consisting of O, N and S, optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1(CO)OR^2$, halogen, cyano and nitro and also oxides (e.g. =O, -O- or-OH).

(v) a saturated and partially saturated $C_3$-$C_6$ monocyclic carbocyclic moiety optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogen, cyano and, nitro;

(vi) a saturated and partially saturated $C_8$-$C_{10}$ bicyclic carbocyclic moiety, optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogen, cyano and nitro;

(vii) a saturated and partially saturated 5 and 6 membered monocyclic heterocyclic moiety, having 1-3 heteroatoms independently selected from the group consisting of O, N and S, optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2N$,$R^1C(O)R^2$, $NR^1C(O)OR^2$, halogen, cyano and nitro, and also oxides (e.g. =O, -O⁻ or-OH); or

(viii) a saturated and partially saturated 8 to 10 membered bicyclic heterocyclic moiety, having 1-6 heteroatoms independently selected from the group consisting of O, N and S, optionally substituted with 1-2 additional substituents other than Q, independently selected from the group consisting of $R^1$, $OR^1$, $NR^1R^2$, S(O)q$R^1$, $SO_2NR^1R^2$, $NR^1SO_2R^2$, $NR^1C(O)R^2$, $NR^1C(O)OR^2$, halogen, cyano and nitro, and also oxides (e.g. =O, -O⁻ or -OH);

each Q is independently $C(O)R^4$, $C(O)OR^4$ and $C(O)NR^4R^5$;
wherein each $R^1$ - $R^5$ is independently selected from the group consisting of:

(a) hydrogen,
(b) $C_1$-$C_5$ linear, branched, or cyclic alkyl,
(c) phenyl,
(d) $C_1$-$C_3$ alkyl-phenyl, wherein the alkyl moiety is optionally substituted with halogen up to per-halo;
(e) up to per-halo substituted $C_1$-$C_5$ linear or branched alkyl.
(f) $-(CH_2)_q$-X, where X is a 5 or 6 membered monocyclic heterocyclic ring, containing 1-4 atoms selected from oxygen, nitrogen and sulfur, which is saturated, partially saturated, or aromatic, or a 8-10 membered bicyclic heteroaryl having 1-4 heteroatoms selected from the group consisting of O, N and S; and wherein said alkyl moiety is optionally substituted with halogen up to per-halo,

wherein each $R^1$ - $R^5$, other than per-halo substituted $C_1$-$C_5$ linear or branched alkyl, is optionally substituted with 1-3 substituents independently selected from the group consisting of $C_1$-$C_5$ linear or branched alkyl, up to perhalo substituted $C_1$-$C_5$ linear or branched alkyl, $C_1$-$C_3$ alkoxy, hydroxy, carboxy, amino, $C_1$-$C_3$ alkylamino, $C_1$-$C_6$ dialkylamino, halogen, cyano, and nitro;

wherein the variable p is an integer selected from 0, 1, or 2 and the variable q is an integer selected from 0, 1, 2, 3, or 4.

In formula I, suitable hetaryl groups include, but are not limited to, 5-10 membered ring systems containing monocyclic and bicyclic rings, at least one of which is aromatic, in which one or more, e.g., 1-4 carbon atoms in one or more of the rings can be replaced by oxygen, nitrogen or sulfur atoms. In bicyclic ring systems, each ring can have from 3-7 atoms.

"Monocyclic heteroaryl" means an aromatic monocyclic ring having 5 to 6 ring atoms, at least one of which is a hetero atom selected from N, O and S, the remaining atoms being carbon. When more than one hetero atom is present in the moiety, they are selected independently from the other(s) so that they may be the same or different. Monocyclic heteroaryl moieties include, but are not limited to pyrrole, furan, thiophene, imidazole, pyrazole, thiazole, oxazole, isoxazole, isothiazole, triazole, tetrazole, thiadiazole, oxadiazole, pyridine, pyrimidine, pyridazine, pyrazine, and triazine.

Bicyclic heteroaryl means fused bicyclic moieties where one of the rings is chosen from the monocyclic heteroaryl rings described above and the second ring is either benzene or another monocyclic heteroaryl ring described above. When both rings in the bicyclic moiety are heteroaryl rings, they may be the same or different, as long as they are chemically accessible by means known in the art. Bicyclic heteroaryl rings include synthetically accessible 5-5, 5-6, or 6-6 fused bicyclic aromatic structures including, for example but not by way of limitation, benzoxazole (fused phenyl and oxazole), quinoline (fused phenyl and pyridine), imidazopyrimidine (fused imidazole and pyrimidine), and the like.

The phrase "5 or 6 membered heterocyclic ring, containing at least one atom selected from oxygen, nitrogen and sulfur, which is saturated, partially saturated, or aromatic" includes, by no way of limitation, tetrahydropyrane, tetrahydrofurane, 1,3-dioxolane, 1,4-dioxane, morpholine, thiomorpholine, piperazine, piperidine, piperidinone, tetrahydropyrimidone, pentamethylene sulfide, tetramethylene sulfide, dihydropyrane, dihydrofuran, dihydrothiophene, pyrrole, furan, thiophene,

imidazole, pyrazole, thiazole, oxazole, isoxazole, isothiazole, triazole, pyridine, pyrimidine, pyridazine, pyrazine, triazine, and the like.

The term "$C_1$-$C_3$ alkyl-phenyl" includes, by no way of limitation, 3-phenylpropyl, 2-phenyl-1-methyl-ethyl. Substituted examples include 2-[2-chlorophenyl]ethyl, 3,4-dimethylphenyl-methyl, and the like.

Suitable substituted and unsubstituted heteroaryl groups for the compounds of this invention, such as those for B, L and L' of formula I, include, but are not limited to the following monocyclic heteroaryl groups:

2- or 3-furyl,
2- or 3-thienyl,
2- or 4-triazinyl,
1-, 2- or 3-pyrrolyl,
1-, 2-, 4- or 5-imidazolyl,
1-, 3-, 4- or 5-pyrazolyl,
2-, 4- or 5-oxazolyl,
3-, 4- or 5-isoxazolyl,
2-, 4- or 5-thiazolyl,
3-, 4- or 5-isothiazolyl,
2-, 3- or 4-pyridyl,
2-, 4-, 5- or 6-pyrimidinyl,
1,2,3-triazol-1-, -4- or-5-yl,
1,2,4-triazol-1-, -3- or -5-yl,
1- or 5-tetrazolyl,
1,2,3-oxadiazol-4- or-5-yl,
1,2,4-oxadiazol-3- or-5-yl,
1,3,4-thiadiazol-2- or-5-yl,
1,2,4-oxadiazol-3- or-5-yl,
1,3,4-thiadiazol-2- or -5-yl,
1,3,4-thiadiazol-3- or-5-yl,
1,2,3-thiadiazol-4- or -5-yl,
2-, 3-, 4-, 5- or 6-2H-thiopyranyl,
2-, 3- or 4-4H-thiopyranyl,
3- or 4-pyridazinyl, pyrazinyl, and

the following bicyclic heterocyclic groups:

benzofuryl, benzothienyl, indolyl, benzimidazolyl, benzopyrazolyl, benzoxazolyl, benzisoxazolyl, benzothiazolyl, benzisothiazolyl, benz-1,3-oxadiazolyl, quinolinyl, isoquinolinyl, quinazolinyl, tetrahydroquinolinyl, tetrahydroisoquinolinyl, dihydrobenzofuryl, pyrazolo[3,4-b]pyrimidinyl, purinyl, benzodiazine, pterindinyl, pyrrolo[2,3-b]pyridinyl, pyrazolo[3,4-b]pyridinyl, oxazo[4,5-b]pyridinyl, imidazo[4,5-b]pyridinyl, cyclopentenopyridine, cyclohexanopyridine, cyclopentanopyrimidine, cyclohexanopyrimidine, cyclcopentanopyrazine, cyclohexanopyrazine, cyclopentanopyridiazine, cyclohexanopyridazine, cyclopentanoimidazole, cyclohexanoimidazole, cyclopentanothiophene and cyclohexanothiophene.

Suitable aryl groups which do not contain heteroatoms include, for example, phenyl and 1- and 2-naphthyl, tetrahydronaphthyl, indanyl, indenyl, benzocyclobutanyl, benzocycloheptanyl and benzocycloheptenyl.

Suitable linear alkyl groups and alkyl portions of groups, e.g., alkoxy, alkylphenyl and alkylheteroaryl etc. throughout include methyl, ethyl, propyl, butyl, pentyl, etc. Suitable branched alkyl groups include all branched isomers such as isopropyl, isobutyl, sec-butyl, tert-butyl, etc.

The term "alkoxy" means a straight or branched chain alkoxy group having saturated carbon atoms which may be linear or branched with single or multiple branching, and includes such groups as methoxy, ethoxy, n-propoxy, isopropoxy, and the like. It also includes halogenated groups such as 2,2-dichloroethoxy, trifluoromethoxy, and the like.

$C_1$-$C_3$alkylamino means methylamino, ethylamino, propylamino or isopropylamino. Examples of $C_1$-$C_6$ dialkylamino group include but are not limited to diethylamino, ethyl-isopropylamino, means methylamino, methyl-isobutylamino, dihexylamino.

Suitable halogens include F, Cl, Br, and/or I, from one to per-substitution (i.e. all H atoms on a group replaced by a halogen atom) being possible where an alkyl group is substituted by halogen, mixed substitution of halogen atom types also being possible on a given moiety. Preferred halogens are Cl, Br and F.

The term "up to perhalo substituted linear and branched alkyl," includes alkyl groups having one alkyl hydrogen

replaced with halogen, alkyl groups wherein all hydrogens are replaced with halogen, alkyl groups wherein more than one but less than all hydrogens are replaced by halogen and alkyl groups having alkyl hydrogens replaced by halogen and other substituents. Examples include chloromethyl, dichloromethyl, trichloromethyl, fluoromethyl, difluoromethyl, trifluoromethyl, and the like.

The term "cycloalkyl", as used herein, refers to cyclic structures having 3-8 members in the ring such as cyclopropyl, cyclobutyl and cyclopentyl and cyclic structures having 3-8 members with alkyl substituents such that, for example, "$C_3$ cycloalkyl" includes methyl substituted cyclopropyl groups.

The term "saturated carbocyclic moieties" defines only the cyclic structure, i.e. cyclopentyl, cyclohexyl, etc. Any alkyl substitution on these cyclic structures is specifically identified.

Saturated monocyclic and bicyclic carbocyclic moieties include cyclopropyl, cyclobutyl, cyclopentyl, cyclohexyl, and decahydronaphthalene.

Partially saturated monocyclic and bicyclic carbocyclic moieties include cyclopentenyl, cyclohexenyl, cyclohexadienyl and tetrahydronaphthalene.

Saturated monocyclic and bicyclic heterocyclic moieties include tetrahydropyranyl, tetrahydrofuranyl, 1,3-dioxolane, 1,4-dioxanyl, morpholinyl, thiomorpholinyl, piperazinyl, piperidinyl, piperidinonyl, tetrahydropyrimidonyl, pentamethylene sulfide and tetramethylene sulfide.

Partially saturated monocyclic and bicyclic heterocyclic moieties include dihydropyranyl, dihydrofuranyl, dihydrothienyl, dihydropiperidinyl, and dihydropyrimidonyl.

When any moiety is "substituted", it can have up to the highest number of indicated substituents, and each substituent can be located at any available position on the moiety and can be attached through any available atom on the substituent. "Any available position" means any position on the moiety that is chemically accessible through means known in the art or taught herein and that does not create an unduly unstable molecule. When there are two or more substituents on any moiety, each substituent is defined independently of any other substituent and can, accordingly, be the same or different.

The term "optionally substituted" means that the moiety so modified may be either unsubstituted, or substituted with the identified substituent(s).

It is understood that where L' is pyridine, the term "hydroxy" as a pyridine substituent includes 2-, 3-, and 4-hydroxypyridine, but also includes those structures referred to in the art as 1-oxo-pyridine and 1-hydroxy-pyridine.

Where the plural form of the word compounds, salts, and the like, is used herein, this is taken to mean also a single compound, salt, or the like.

The substituted structures of B and L' are preferably each, independently, selected from the group consisting of methyl, trifluoromethyl, ethyl, n-propyl, n-butyl, n-pentyl, isopropyl, *tert*-butyl, sec-butyl, isobutyl, cyclopropyl, cyclobutyl, cyclopentyl, methoxy, ethoxy, propoxy, Cl, Br and F, cyano, nitro, hydroxy, amino, methylamino, dimethylamino, ethylamino and diethylamino.

Other substituents for B and L' particularly include:

phenyl, pyridinyl, pyrimidinyl, chlorophenyl, dichlorophenyl, bromophenyl, dibromophenyl, chloropyridinyl, bromopyridinyl, dichloropyridinyl, dibromopyridinyl methylphenyl, methylpyridinyl quinolinyl, isoquinolinyl, isoindolinyl, pyrazinyl, pyridazinyl, pyrrolinyl, imidazolinyl, thienyl, furyl, isoxazolinyl, isothiazolinyl, benzopyridinyl, benzothiazolyl,
$C_1$-$C_5$ acyl;
NH($C_1$-$C_5$ alkyl, phenyl or pyridinyl), such as aminophenyl; N($C_1$-$C_5$ alkyl)($C_1$-$C_5$ alkyl, phenyl or pyridinyl), such as diethylamino and dimethyl amino;
S(O)q ($C_1$-$C_5$ alkyl); such as methanesulfonyl;
S(O)q H;
$SO_2NH_2$ ;
$SO_2NH$($C_1$-$C_5$ alkyl);
$SO_2N$($C_1$-$C_5$ alkyl)($C_1$-$C_5$ alkyl);
$NHSO_2$($C_1$-$C_5$ alkyl); N($C_1$-$C_3$ alkyl) $SO_2$($C_1$-$C_5$ alkyl);
CO($C_1$-$C_6$ alkyl or phenyl);
C(O)H;
C(O)O($C_1$-$C_6$ alkyl or phenyl), such as $C(O)OCH_3$, $-C(O)OCH_2CH_3$,$-C(O)OCH_2CH_2CH_3$;
C(O)OH;
$C(O)NH_2$ (carbamoyl);
C(O)NH($C_1$-$C_6$ alkyl or phenyl), such as N-methylethyl carbamoyl, N-methyl carbamoyl, N-ethylcarbamoyl, or N-dimethylamino ethyl carbamoyl;
C(O)N($C_1$-$C_6$ alkyl or phenyl)($C_1$-$C_6$ alkyl, phenyl or pyridinyl), such as N-dimethylcarbamoyl;
C(N($C_1$-$C_5$ alkyl)) ($C_1$-$C_5$ alkyl);
NHC(O)($C_1$-$C_6$ alkyl or phenyl) and

$N(C_1-C_5$ alkyl,)C(O)($C_1-C_5$ alkyl).

Each of the above substituents is optionally partially or fully halogenated, such as difluoromethyl sulfonyl.

An embodiment of this invention includes the administration of compounds of this invention wherein in formula I, L, B and L' follow one of the following of combinations:

B= phenyl, L=phenyl and L' is phenyl, pyridinyl, quinolinyl, isoquinolinyl or not present,
B= phenyl, L=pyridinyl and L' is phenyl, pyridinyl, quinolinyl, isoquinolinyl or not present,
B=phenyl, L = naphthyl and L' is phenyl, pyridinyl, quinolinyl, isoquinolinyl or not present,
B=pyridinyl, L= phenyl and L' is phenyl, pyridinyl, quinolinyl, isoquinolinyl or not present,
B=pyridinyl, L= pyridinyl and L' is phenyl, pyridinyl, quinolinyl, isoquinolinyl or not present,
B=pyridinyl, L= naphthyl and L' is phenyl, pyridinyl, quinolinyl, isoquinolinyl or not present,
B=isoquinolinyl, L= phenyl and L' is phenyl, pyridinyl, quinolinyl, isoquinolinyl or not present,
B= isoquinolinyl, L= pyridinyl and L' is phenyl, pyridinyl, quinolinyl, isoquinolinyl or not present,
B= isoquinolinyl, L= naphthyl and L' is phenyl, pyridinyl, quinolinyl, isoquinolinyl or not present,
B= quinolinyl, L= phenyl and L' is phenyl, pyridinyl, quinolinyl, isoquinolinyl or not present,
B= quinolinyl, L= pyridinyl and L' is phenyl, pyridinyl, quinolinyl, isoquinolinyl or not present,
B= quinolinyl, L= naphthyl and L' is phenyl, pyridinyl, quinolinyl, isoquinolinyl or not present.

The structure M of formula I is preferably -O-, a single bond, -S-, -NH-, - $N(CH_3)$-, -$NHCH_2$-, - $NC_2H_4$-, -$CH_2$-, -C(O)-, -CH(OH)-, -NHC(O)$N(CH_3)CH_2$-,-$N(CH_3)C(O)N(CH_3)CH_2$-, -$CH_2C(O)N(CH_3)$-, -C(O)$N(CH_3)CH_2$-, -NHC(O)-, -$N(CH_3)C(O)$-, -C(O)$N(CH_3)$-, -C(O)NH-, -$CH_2O$- -$CH_2S$-, -$CH_2N(CH_3)$-, -$OCH_2$-,-CHF-, -$CF_2$-, -$CCl_2$-, -S-$CH_2$-, and -N$(CH_3)CH_2$- .
Compounds of the invention of particular interest include those of formula I wherein L', L, M and Q are as defined above and B is phenyl, optionally substituted with 1-4 halogen.
Compounds of the invention of particular interest also include those of formula I wherein L, L' and Q are as defined above, M is -O- and B is phenyl, optionally substituted with 1-4 halogen.
Compounds of the invention of particular interest also include those of formula I wherein B is phenyl or pyridyl, optionally substituted with 1-6 substituents independently selected from the group consisting of $R^1$ and halogen, L' and Q are as defined above, M is -0- and L is phenyl, optionally substituted with 1-4 halogen.
Compounds of the invention of particular interest also include those of formula I wherein B is phenyl, optionally substituted with 1-6 substituents independently selected from the group consisting of $R^1$ and halogen, L' and Q are as defined above, M is -0- and L is phenyl, optionally substituted with 1-4 halogen.
Compounds of the invention of particular interest also include those of formula I wherein B is 4-chloro(2-trifluoromethyl) phenyl, optionally substituted by the group consisting of $R^1$ and halogen, L' and Q are as defined above, M is -O- and L is phenyl, optionally substituted with 1-4 halogen.
One of ordinary skill in the art will recognize that some of the compounds of Formula (I) can exist in different geometrical isomeric forms. It is intended that all such configurations (including enantiomers and diastereomers), are included within the scope of the present invention. A number of the compounds of Formula I possess asymmetric centers, depending on the location a nature of various substituents. and can therefore exist in racemic and optically active forms as well as in the form of racemic or non-racemic mixtures thereof, and in the form of diastereomers and diastereomeric mixtures. Asymmetric carbon atoms may be present in the (*R*) or (*S*) configuration or (*R,S*) configuration. In certain instances, asymmetry may also be present due to restricted rotation about a given bond, for example, the central bond adjoining two substituted aromatic rings of the specified compounds. All of these compounds, including *cis* isomers, *trans* isomers, diastereomic mixtures, racemates, non-racemic mixtures of enantiomers, substantially pure, and pure enantiomers, are considered to be within the scope of the compounds of this invention and are collectively referred to when reference is made to compounds of this invention. Therefore, the methods of the present invention encompass the use of any isolated racemic or optically active form of compounds described in Formula I which possess c-raf, b-raf , p38, VEGFR, PDGFR, and/or FLT-3 activity.

Methods of separation of enantiomeric and diastereomeric mixtures are well known to one skilled in the art. The optical isomers can be obtained by resolution of the racemic mixtures according to conventional processes, for example, by the formation of diastereoisomeric salts using an optically active acid or base. Examples of appropriate acids are tartaric, diacetyltartaric, dibenzoyltartaric, ditoluoyltartaric and camphorsulfonic acid. Mixtures of diastereoisomers can be separated into their individual diastereomers on the basis of their physical chemical differences by methods known to those skilled in the art, for example, by chromatography or fractional crystallization. The optically active bases or acids are liberated from the separated diastereomeric salts.

Another process for separation of optical isomers involves the use of a chiral chromatography column (e.g., chiral HPLC columns) optimally chosen to maximize the separation of the enantiomers. Suitable chiral HPLC columns

are manufactured by Diacel, e.g., Chiracel OD and Chiracel OJ. The optically active compounds of Formula (I) can likewise be obtained by utilizing optically active starting materials.

The present invention envisages any separated, isolated, pure or partially purified isomers or racemic mixtures of the compounds of formula I which possess VEGFR, PDGFR, and/or FLT-3 activity, and/or an efficacy in modulating any of the diseases and/or conditions mentioned herein. The term stereoisomer is understood to encompass diastereoisomers, enantiomers, geometric isomers, etc.

Preferred compounds are those with the absolute configuration of the compound of Formula I which produce the more desirable biological activity are also included within the scope of the present invention. The purification of said isomers and the separation of said isomeric mixtures can be accomplished by standard techniques known in the art. Herein, substantially pure enantiomers is intended to mean that no more than 5% w/w of the corresponding opposite enantiomer is present.

Pharmaceutically-acceptable salts of these compounds, as well as commonly used prodrugs of these compounds, are also within the scope of the invention. The term "pharmaceutically acceptable salt" refers to a relatively non-toxic, inorganic, or organic acid addition salt of a compound of the present invention. For example, see S. M. Berge, et al. "Pharmaceutical Salts," J. Pharm. Sci. 1977, 66; 1-19.

Suitable salts are especially the pharmaceutically acceptable salts of compounds of formula (I) or such as, for example, organic or inorganic acid addition salts of compounds of formula (I). Suitable acid addition salts include acetate, adipate, alginate, ascorbate, aspartate, benzoate, benzenesulfonate, bisulfate, butyrate, citrate, camphorate, camphorsulfonate, cinnamate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, itaconate, lactate, maleate, mandelate, methanesulfonate, 2-naphthalenesulfonate, nicotinate, nitrate, oxalate, pamoate, pectinate, persulfate, 3-phenylpropionate, picrate, pivalate, propionate, succinate, sulfonate, tartrate, thiocyanate, tosylate, and undecanoate. Suitable inorganic acids include but are not limited to halogen acids (such as hydrochloric acid and hydrobromic acid), sulfuric acid, or phosphoric acid. Suitable organic acids include but are not limited to carboxylic, phosphonic, sulfonic, or sulfamic acids, with examples including acetic acid, propionic acid, octanoic acid, decanoic acid, trifluoroacetic acid, dodecanoic acid, glycolic acid, lactic acid, 2- or 3-hydroxybutyric acid, $\gamma$-aminobutyric acid (GABA), gluconic acid, glucosemonocarboxylic acid, benzoic acid, salicylic acid, phenylacetic acid and mandelic acid, fumaric acid, succinic acid, adipic acid, pimelic acid, suberic acid, azeiaic acid, maleic acid, tartaric acid, citric acid, glucaric acid, galactaric acid, amino acids (such as glutamic acid, aspartic acid, N-methylglycine, acetytaminoacetic acid, N-acetylasparagine or N-acetylcysteine), pyruvic acid, acetoacetic acid, methanesulfonic acid, tri-fluoromethane sulfonic acid, 4-toluene sulfonic acid, benzenesulfonic acid, 1-naphthalenesulfonic acid, 2-naphthalenesulfonic acid, phosphoserine, and 2- or 3-glycerophosphoric acid.

In addition, pharmaceutically acceptable salts include acid salts of inorganic bases, such as salts containing alkaline cations (e.g., $Li^+$ $Na^+$ or $K^+$), alkaline earth cations (e.g., $Mg^{+2}$, $Ca^{+2}$ or $Ba^{+2}$), the ammonium cation, as well as acid salts of organic bases, including aliphatic and aromatic substituted ammonium, and quaternary ammonium cations, such as those arising from protonation or peralkylation of triethylamine, *N,N*-diethylamine, *N,N*-dicyclohexylamine, lysine, pyridine, *N,N*-dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).

Base salts include alkali metal salts such as potassium and sodium salts, alkaline earth metal salts such as calcium and magnesium salts, and ammonium salts with organic bases such as dicyclohexylamine and N-methyl-D-glucamine. Additionally, basic nitrogen containing groups may be quaternized with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and strearyl chlorides, bromides and iodides, aralkyl halides like benzyl and phenethyl bromides and others.

The esters of appropriate compounds of this invention are well-tolerated, pharmaceutically acceptable esters such as alkyl esters including methyl, ethyl, propyl, isopropyl, butyl, isobutyl or pentyl esters. Additional esters such as phenyl-$C_1$-$C_5$ alkyl may be used, although methyl ester is preferred.

The formation of prodrugs is well known in the art in order to enhance the properties of the parent compound; such properties include solubility, absorption, biostability and release time (see "Pharmaceutical Dosage Form and Drug Delivery Systems" (Sixth Edition), edited by Ansel et al., published by Williams & Wilkins, pages 27-29, (1995) which is hereby incorporated by reference). Commonly used prodrugs of the disclosed oxazolyl-phenyl-2,4-diamino-pyrimidine compounds are designed to take advantage of the major drug biotransformation reactions and are also to be considered within the scope of the invention. Major drug biotransformation reactions include N-dealkylation, O-dealkylation, aliphatic hydroxylation, aromatic hydroxylation, N-oxidation, S-oxidation, deamination, hydrolysis reactions, glucuronidation, sulfation and acetylation (see Goodman and Gilman's The Pharmacological Basis of Therapeutics (Ninth Edition), editor Molinoff et al., pub. by McGraw-Hill, pages 11-13, (1996), which is hereby incorporated by reference).

The present invention provides methods for assessing the efficacy of compounds which are capable of modulating one or more signal transduction pathways comprising, VEGFR-2, VEGFR-3, PDGFR-beta, and/or Flt-3. Raf is

an important signaling molecule involved in the regulation of a number of key cellular processes, including cell growth, cell survival and invasion. It is a member of the Ras/Raf/MEK/ERK pathway. This pathway is present in most tumor cells. VEGFR-2, VEGFR-3, PDGFR-beta and Flt-3 are transmembrane receptor molecules which, when stimulated by an appropriate ligand, trigger the Ras/Raf/MEK/ERK cell signaling pathway, leading to a cascade of cellular events. Each of these receptor molecules have tyrosine kinase activity.

The VEGFR receptors are stimulated by vascular endothelial growth factors (VEGF), and are important control points in the regulation of endothelial cell development and function. The PDGF-beta receptor regulates cell proliferation and survival in a number of cell types, including mesenchymal cells. Flt-3 is a receptor for the FL ligand. It is structurally similar to c-kit, and modulates the growth of pluripotent haemopoietic cells, influencing the development of T-cells, B-cells, and dendritic cells.

Any gene or isoform of VEGFR-2, VEGFR-3, PDGFR-beta, and/or Flt-3 can be modulated in accordance with present invention, including both wild-type and mutant forms.

By the term "modulate," it is meant that the functional activity of the pathway (or a component of it) is changed in comparison to its normal activity in the absence of the compound. This effect includes any quality or degree of modulation, including, increasing, agonizing, augmenting, enhancing, facilitating, stimulating, decreasing, blocking, inhibiting, reducing, diminishing antagonizing, etc.

Tables 8 and 9 show the activity of a compound N-[4-chloro-3-(trifluoromethyl)phenyl]-N'-{4-[2-N-methylcarbamoyl-4-pyridyloxy]phenyl} urea of the present invention in vitro (biochemical) and in vivo (cellular) assays, respectively. As indicated by its biochemical activity profile (Table 8), the compound is a potent inhibitor of a number of different cellular kinases. Moreover, it has the ability to affect Kinase activity, cell growth, and other cellular processes in whole cells. Table 9 shows the concentration of compound which inhibited target phosphorylation in whole cells by 50% (IC50). While the activity profile is representative of an active compound of the present invention, not all active compounds are required to have the same rank order of potency for them to be useful in the present invention. Assays for biochemical and cellular activity are conventional and can be carried out using any suitable format, including as described in the examples below. See, also, VEGPR-3 kinase assays (e.g., Manley et al., J. Med. Chem., 45(26):5687-93, 2002; Stahl et al., Angew Chem. Int. Ed. Engl., 41(7):1174-8, 2002); FLT3 kinase assays (Yee et al., Blood, 100(8):2941-9, 2002; Kelly et al., Cancer Cell., 1(5):421-32. 2002).

The compounds to be assessed in the present invention can also modulate one or more of the following processes, including, but not limited to, e.g., cell growth (including e.g., differentiation, cell survival, and/or proliferation), tumor cell growth (including, e.g., differentiation, cell survival, and/or proliferation), tumor regression, endothelial cell growth (including e.g., differentiation, cell survival, and/or proliferation), angiogenesis (blood vessel growth), lymphangiogenesis (lymphatic vessel growth), and/or hematopoiesis (e.g., T- and B-cell development, dendritic cell development, etc.).

While not wishing to be bound by any theory or mechanism of action, it has been found that compounds to be assessed in possess the ability to modulate kinase activity. The Methods of the present invention, however, are not limited to any particular mechanism or how the compounds achieve their therapeutic effect. By the phrase "kinase activity," it is meant a catalytic activity in which a gamma-phosphate from adenosine triphosphate (ATP) is transferred to an amino acid residue (e.g., serine, threonine, or tyrosine) in a protein substrate. A compound can modulate kinase activity, e.g., inhibiting it by directly competing with ATP for the ATP-binding pocket of the kinase, by producing a conformational change in the enzyme's structure that effects its activity (e.g., by disrupting the biologically-active three-dimensional structure), etc.

Kinase activity can be determined routinely using conventional assay methods. Kinase assays topically comprise the kinase enzyme, substrates, buffers, and components of a detection system. A typical kinase assay involves the reaction of a protein kinase with a peptide substrate and an ATP, such as 32P-ATP, to produce a phosphorylated end-product (for instance, a phosphoprotein when a peptide substrate is used. The resulting end-product can be detected using any suitable method. When radioactive ATP is utilized, a radioactively labeled phosphoprotein can be separated from the unreacted gamma-32P-ATP using an affinity membrane or gel electrophoresis, and then visualized on the gel using autoradiography or detected with a scintillation counter. Non-radioactive methods can also be used. Methods can utilize an antibody which recognizes the phosphorylated substrate, e.g., an anti-phosphotyrosine antibody. For instance, kinase enzyme can incubated with a substrate in the presence of ATP and Kinase buffer under conditions which are effective for the enzyme to phosphorylate the substrate. The reaction mixture can be separated, e.g., electrophoretically, and then phosphorylation of the substrate can be measured, e.g., by Western blotting using an anti-phosphotyrosine antibody. The antibody can be labeled with a detectable label, e.g., an enzyme, such as HRP, avidin or biotin, chemiluminescent reagents, etc. Other methods can utilize ELISA formats, affinity membrane separation, fluorescence polarization assays, luminescent assays, etc.

An alternative to a radioactive format is time-resolved fluorescence resonance energy transfer (TR-PRET). This methods follows the standard kinase reaction, where a substrate, e.g., biotinylated poly(GluTyr), is phosphorylated by a protein kinase in the presence of ATP. The end-product can then detected with a europium chelate phosphospecific

antibody (anti-phosphotyrosine orphosphoserine/theonine), and streptavidin-APC, which binds the biotinylated substrate, These two components are brought together spatially upon binding, and energy transfer from the phosphospecific antibody to the acceptor (SA-APC) produces fluorescent readout in the homogeneous format.

The compounds to be assessed in the present invention can be used to treat and/or prevent any disease or condition involving one or more cellular signal transduction pathways comprising VEGFR-2, VEGFR-3, PDGPR-beta, and/or Hlt-3. The term "treating" is used conventionally, e.g., the management or care of a subject for the purpose of combating, alleviating, reducing, relieving, improving the condition of, etc., of a disease or disorder. Diseases and conditions that can be treated include any of those mentioned above and below, as well as:

VEGFR-2 associated diseases include, e.g., cancer, tumor growth, inflammatory disease, rheumatoid arthritis, retinopathy, psoriasis, glomerulonephritis, asthma, chronic bronchitis, atherosclerosis, transplant rejection, conditions involving angiogenesis, etc.;

VEGFR-3 associated diseases include, e.g., cancer, corneal disease, inflamed cornea (e.g., Hamrah, Am. J. Path., 163:57-68,2003), corneal transplantation (Cursiefen et al., Cornea, 22:273-81, 2003), lymphatic hyperplasia, conditions involving lymphangiogenesis; etc.;

PDGFR-beta associated diseases include, e.g., diseases or conditions characterized by cell proliferation, cell matrix production, cell movement, and/or extracellular matrix production. Specific examples, include, e.g., tumors, malignancies, cancer, metastasis, chronic myeloid leukemia, inflammation, renal disease, diabetic nephropathy, mesangial proliferative glomerulonephritis, fibrotic conditions, atherosclerosis, restenosis, hypertension-related arterosclerosis, venous bypass graft arterosclerosis, scleroderma, interstitial pulmonary diseases, synovial disorders, arthritis, leukemias, lymphomas, etc;

Flt-3 associated diseases include, e.g., immune-related disorders, blood cell disorders, conditions involving hematopoietic cell development (e.g., T-cells, B-cells, dendritic cells, cancer, anemia, HIV, acquired immune deficiency syndrome, etc.

In addition, the specific compounds can be used to treat conditions and disorders disclosed in U.S. Pat. No. 6,316,479, e.g, glomerular sclerosis, interstitial nephritis, interstitial pulmonary fibrosis, atherosclerosis, wound scarring and sclerodenna.

The specific compounds also have a broad therapeutic activity to treat or prevent the progression of a broad array of diseases, such as inflammatory conditions, coronary restenosis, tumor-associated angiogenesis, atherosclerosis, autoimmune diseases, inflammation, certain kidney diseases associated with proliferation of glomerular or mesangial cells, and ocular diseases associated with retinal vessel proliferation, psoriasis, hepatic cirrhosis, diabetes, atherosclerosis, restenosis, vascular graft restenosis, in-stent stenosis, angiogenesis, ocurlar diseases, pulmonary fibrosis, obliterative bronchiolitis, glomerular nephritis, rheumatoid arthritis.

The present invention also provides the assessment of specific compounds for treating, preventing, modulating, etc., one or more of the following conditions in humans and/or other mammals: retinopathy, including diabetic retimopathy, ischemic retinal-vein occlusion, retinopathy of prematurity and age related macular degeneration; rheumatoid arthritis, psoriasis, or bullous disorder associated with subepidermal blister formation, including bullous pemphigoid, erythema multiforme, or dermatitis herpetiformis, rheumatic fever, bone resorption, postmenopausal osteoperosis, sepsis, gram negative sepsis, septic shock, endotoxic shock, toxic shock syndrome, systemic inflammatory response syndrome, inflammatory bowel disease (Crohn's disease and ulcerative colitis), Jarisch-Herxheimer reaction, asthma, adult respiratory distress syndrome, acute pulmomary fibrotic disease, pulmonary sarcoidosis, allergic respiratory disease, silicosis coal worker's pneumoconiosis, alveolar injury, hepatic failure, liver disease during acute inflammation, severe alcoholic hepatitis, malaria (Plasmodium falciparum malaria and cerebral malaria), non-insulin-dependent diabetes mellitus (NIDDM), congestive heart failure, damage following heart disease, atherosclerosis, Alzheimer's disease, acute encephalitis, brain injury, multiple sclerosis (demyelation and oligiodendrocyte loss in multiple sclerosis), advanced cancer, lymphoid malignancy, pancreatitis, impaired wound healing in infection, inflammation and cancer, myelodysplastic syndromes, systemic lupus erythematosus, biliary cirrhosis, bowel necrosis, radiation injury/ toxicity following administration of monoclonal antibodies, host-versus-graft reaction (ischemia reperfusion injury and allograft rejections of kidney, liver, heart, and skin), lung allograft rejection (obliterative bronchitis, or complications due to total hip replacement, ad an infectious disease selected from tuberculosis, Helicobacter pylori infection during peptic ulcer disease, Chaga's disease resulting from Trypanosoma cruzi infection, effects of Shiga-like toxin resulting from E. coli infection, effects of enterotoxin A resulting from Staphylococcus infection, meningococcal infection, and infections from Borrelia burgdorferi Treponema pallidum, cytomegalovirus, influenza virus, Theiler's encephalomyelitis virus, and the human immunodeficiency virus (HIV), papilloma, blastoglioma, Kaposi's sarcoma, melanoma, lung cancer, ovarian cancer, prostate cancer, squamous cell carcinoma, astrocytoma, head cancer, neck cancer, bladder cancer, breast cancer, colorectal cancer, thyroid cancer, pancreatic cancer, gastric cancer, hepatocellular carcinoma, leukemia, lymphoma, Hodgkin's disease, Burkitt's disease, arthritis, rheumatoid arthritis, diabetic retinopathy, angiogenesis, restenosis, in-stent restenosis, vascular graft restenosis, pulmonary fibrosis, hepatic cirrhosis, atherosclerosis, glomerulonophritis, diabetic nephropathy, thrombic micoangiopathy syndromes, transplant rejection, psoriasis, diabetes, wound healing, inflammation, and neurodegenerative

diseases, hyperimmune disorders, hemangioma, myocardial angiogenesis, coronary and cerebral collateral vascularization, ischemia, corneal disease, rubeosis, neovascular glaucoma, macular degeneration retinopathy of prematurity, wound healing, ulcer Helicobacter related diseases, fractures, endometriosis, a diabetic condition, cat scratch fever, thyroid hyperplasia, asthma or edema following burns, trauma, chronic lung disease, stroke, polyps, cysts, synovitis, chronic and allergic inflammation, ovarian hyperstimulation syndrome, pulmonary and cerebral edema, keloid, fibrosis, cirrhosis, carpal tunnel syndrome, adult respiratory distress syndrome, ascites, an ocular conditions, a cardiovascular condition; Crow-Fukase (POEMS) disease, Crohn's disease, glomerulonophritis, osteoarthritis, multiple sclerosis, graft rejection, Lyme disease, sepsis, von Hippel Lindau disease, pemphigoid, Paget's disease, polycystic kidney disease, sarcoidosis, throiditis, hyperviscosity syndrome, Osler-Weber-Rendu disease, chronic occlusive pulmonary disease, radiation, hypoxia, preeclampsia, menometrorrhagia, endometriosis, infection by Herpes simplex, ischemic retinopathy, corneal angiogensis, Herpes Zoster, human immumodeficiency virus, parapoxvirus, protozoa, toxoplasmosis, and tumor-associated effusion and edema.

Compounds can possess more than one of the mentioned activities, and therefore can target a plurality of signal transduction pathways. Thus, these compound can achieve therapeutic and prophylactic effects which normally are only obtained when using a combination of dlifferent compounds. For instance, the ability to inhibit both new vessel formation (e.g., associated with VEGFR-2 and -3 function) (e.g., blood and/or lymph) and cell-proliferation (e.g., associated with raf and PDGFR-beta function) using a single compound is especially beneficial in the treatment of cancer, and other cell-proliferation disorders that are facilitated by neo-vascularization. Thus, the present invention relates specifically to the assessment of compounds which possess at least anti-cell proliferation and and-angiogenic (i.e., inhibits angiogenesis) activity. Any disorder or condition that would benefit from inhibiting vessel growth and cell proliferation can be treated in accordance with the present invention. Using a single compound is also advantageous because its range of activities can be more precisely defined.

As indicated above, the present invention relates to the assessement of specific compounds for treating and/or preventing diseases and conditions; and/or modulating one or more of the pathways, polypeptides, genes, diseases, conditions, etc., associated with VEGFR-2, VEGFR,-3, PDGFR-beta, and/or Flt-3. The methods generally involve administering effective amounts of compounds of the present invention, where an effective amount is the quantity of the compound which is useful to achieve the desired result. Compounds can be administered in any effective form by any effective route, as discussed in more detail below.

The use includes modulating tumor cell proliferation, including inhibiting cell proliferation. The latter indicates that the growth and/or differentiation of tumor cells is reduced, decreased, diminished, slowed, etc. The term "proliferation" includes any process which relates to cell growth and division, and includes differentiation and apoptosis. raf kinases play a key role in the activation of the cytoplasmic signaling cascade involved in cell proliferation, differentiation, and apoptosis. For example, studies have found that inhibiting c-raf-1 by anti-sense oligonucleotides can block cell proliferation (see above). Any amount of inhibition is considered therapeutic.

Examples of breast cancer include, but are not limited to, invasive ductal carcinoma, invasive lobular carcinoma, ductal carcinoma in situ, and lobular carcinoma in situ.

Examples of cancers of the respiratory tract include, but are not limited to, small-cell and non-small-cell lung carcinoma, as well as bronchial adenoma and pleuropulmonary blastoma.

Examples of brain cancers include, but are not limited to, brain stem and hypophtalmic glioma, cerebellar and cerebral astrocytoma, medulloblastoma, ependymoma, as well as neuroectodermal and pineal tumor.

Tumors of the male reproductive organs include, but are not limited to, prostate and testicular cancer. Tumors of the female reproductive organs include, but are not limited to, endometrial, cervical, ovarian, vaginal, and vulvar cancer, as well as sarcoma of the uterus.

Tumors of the digestive tract include, but are not limited to, anal, colon, colorectal, esophageal, gallbladder, gastric, pancreatic, rectal, small-intestine, and salivary gland cancers.

Tumors of the urinary tract include, but are not limited to, bladder, penile, kidney, renal pelvis, ureter, and urethral cancers.

Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, squamous cell carcinoma, Kaposi's sarcoma, malignant melanoma, Merkel cell skin cancer, and non-melanoma skin cancer.

Head-and-neck cancers include, but are not limited to, laryngeal, hypopharyngeal, nasopharyngeal, and/or oropharyngeal cancers, and lip and oral cavity cancer.

Lymphomas include, but are not limited to, AIDS-related lymphoma, non-Hodgkin's lymphoma, cutaneous T-cell lymphoma, Hodgkin's disease, and lymphoma of the central nervous system.

Sarcomas include, but are not limited to, sarcoma of the soft tissue, osteosarcoma, malignant fibrous histiocytoma, lymphosarcoma, and rhabdomyosarcoma.

Leukemias include, but are not limited to, acute myeloid leukemia, acute lymphoblastic leukemia, chronic lymphocytic leukemia, chronic myelogenous leukemia, and hairy cell leukemia.

In addition to inhibiting the proliferation of tumor cells, the specific compounds can also cause tumor regression, e.g., a decrease in the size of a tumor, or in the extent of cancer in the body.

The present invention also relates to the assessement of specific compounds for modulating angiogenesis and/or lymphangiogenesis in a system comprising cells, comprising administering to the system an effective amount of a compound described herein. A system comprising cells can be an in vivo system, such as a tumor in a patient, isolated organs, tissues, or cells, in vitro assays system (CAM, BCE, etc), animal models (e.g., in vivo, subcutaneous, cancer models), hosts in need of treatment (e.g., hosts suffering from diseases having angiogenic and/or lymphangiogenic component, such as cancer), etc. Preferred compounds of the present invention inhibit angiogenesis and/or lymphangiogenesis, e.g., the formation of new blood vessels.

Inappropriate and ectopic expression of angiogenesis can be deleterious to an organism. A number of pathological conditions are associated with the grower of extraneous blood vessels. These include, e.g., diabetic retinopathy, neovascular glaucoma, psoriasis, retrolental fibroplasias, angiofibroma, inflammation, etc. In addition, the increased blood supply associated with cancerous and neoplastic tissue, encomages growth, leading to rapid tumor enlargement and metastasis. Moreover, the growth of new blood and lymph vessels in a tumor provides an escape route for renegade cells, encouraging metastasis and the consequence spread of die cancer.

Useful systems for modulating angiogenesis, include, e.g., neovascularization of tumor explants (e.g., U.S. Pat Nos. 5,192,744; 6.024,688), chicken chorioallantoic membrane (CAM) assay (e.g., Taylor and Folkman, Nature, 297:307-312, 1982; - Eliceiri et al., J. Cell Biol., 140,1255-1263, 1998), bovine capillary endothelial (BCE) cell assay (e.g., U.S. Pat. No. 6,024,688; Polverini, P. J. et al., Methods Enzymol., 198: 440-450, 1991), migration assays, and HUVEC (human umbilical cord vascular endothelial cell) growth inhibition assay (e.g., U.S. Pat. No. 6,060,449). In addition, useful system for modulating lymphangiogenesis, include, e.g., rabbit ear model (e.g., Szuba et al., FASEB J., 16(14):1985-7, 2002).

Modulation of angiogenesis can be determined by any suitable methods. For example, the degree of tissue vascularity is typically determined by assessing the number and density of vesssels present in a given sample. For example, microvessel density (MVD) can be estimated by counting the number of endothelial clusters in a high-power microscopic :field, or detecting a marker specific for microvascular endothelium or other markers of growing or established blood vessels, such as CD31 (also known as platelet-endothelial cell adhesion molecule or PECAM). A CD31 antibody can be employed in conventional immunohistological methods to immunostain tissue sections as described by, e.g., Penfold et al, Br. J. Oral and Maxill. Surg., 34: 37-41; U.S. Pat. No. 6,017,949; Dellas et al., Gyn. Oncol, 67:27-33, 1997; and others. Other markers for angiogenesis, include, e.g., Vezfl (e.g., Xiang et al., Dev. Bio., 206:123-141, 1999), angiopoietin, Tie-1, and Tie-2 (e.g., Sato et al., Nature, 376:70-74, 1995). Additionally, levels of circulating VEGF, such as VEGF-165, VEGF-C, VEGF-D, can be measured in an RELISA to determine whether it is above a threshold value that indicates angiongenic activity in the body.

Additionally, the present invention relates to methods of screening patients to determine their susceptibility to compounds of the present invention. For example, the presenting invention relates to methods of selecting subjects having a disease for treatment with a compound of formula I, comprising, one or more of the following steps in any effective order, e.g., Measuring the expression or activity of VEGFR-2, VEGFR-3, PDGFR-buta, and/or Flt-3, in a sample obtained from a subject having a disease.

The term "susceptibility" is used broadly to indicate, e.g., ability to respond, toxicity or other adverse effects, etc. For example, the invention relates to methods of determining whether a condition can be modulated by a compound disclosed herein, comprising measuring the expression or activity of VEGFR-2, VEGFR-3, PDGFR-beta, and/or Flt-3 in cells having said condition. The results can be used to determine or predict whether a subject will respond to a compound of the present invention. For example, where the condition is a tumor, the methods can be used to predict whether the tumor is susceptible to compounds of the present invention. By the term "susceptible," it is meant that tumor can be treated with it, e.g., causing tumor regression or cell death, inhibiting cell proliferation, inhibiting tumor growth, inhibiting tumor metastasis, etc.

Whether a condition, such as a tumor, is susceptible to a compound of the present invention can be determined routinely. For instance, cells or tissues (e.g., tumor cells, a biopsy sample, etc.) that exhibit the condition can be assayed for the presence and/or activity of VEGFR-2, VEGFR-3, PDGFR-beta, and/or Flt-3. When high levels of expression and/or activity are identified, this can indicate that the subject will respond to, and benefit from, a compound of the present invention. Levels of gene expression (e.g., mRNA levels), gene amplification, or gene product activity (e.g., tyrosine kinase activity) can be utilized to characterize the state of the cell with respect to the corresponding gene and signaling pathway. For example, the target genes of the present invention possess tyrosine Kinase activity, and therefore kinase activity can be used to assess the cell or tissue state. In the example below, activity was measured by looking at the levels of substrate phosphorylated by it. This can be done quantitatively (e.g., using isotopes, spectroscopy, etc.)

or semi-quantitatively as in the example where the levels were assessed visually and assigned a level of intensity from +1 to +4. A cell or tissue which has a high level of phosphorylated substrate (and a high number of cells exihibiting the heightened activity) can be considered to have a high level of kinase activity, and therefore be a candidate for therapy with a compound of the present invention. More than one activity can be assessed, and the results from several targets can be utilized in deciding whether a subject's condition (e.g., a tumor) will be responsive to a compound of the present invention.

High levels of target activity can be relative to a control or other standard. For instance, in the example below, high levels of activity were with reference to a cell type (stromal) in the tissue section which normally does not express substantial levels of the target gene. High levels can therefore be where cells express a statistically higher amount of measured activity or phosphoryated substrate than the standard or control used as a comparison. High levels can also be where 25% or more cells express the target activity (e.g., phospho-ERK).

The method can further comprise a step of comparing the expression in a sample with a normal control, or expression in a sample obtained from normal or unaffected tissue. Comparing can be done manually, against a standard, in an electronic form (e.g., against a database), etc. The normal control can be a standard sample that is provided with the assay; it can be obtained from adjacent, but unaffected, tissue from the same patient; or, it can be pre-determined values, etc. Gene expression, protein expression (e.g., abundance in a cell), protein activity (e.g., kinase activity), etc., can be determined.

For instance, a biopsy from a cancer patient can be assayed for the presence, quantity, and/or activity of VEGFR-2, VEGFR-3, PDGFR-beta, and/or Flt-3. Increased expression or activity of one or more of these can indicate that the cancer can be targeted for treatment by a compound of the present invention. For example, as described in the examples below, raf activity can be monitored by its ability to initiate the cascade leading to ERK phosphorylation (i.e., raf/MEK/ERK), resulting in phospho-ERK. Increased phospho-ERK levels in a cancer shows that its raf activity is elevated, suggesting the use of compounds of the present invention to treat it. In addition to biopsy samples, phospho-ERK (other markers) can also be measured in other body fluids, such as serum, blood, cerebral spinal fluid, urine, etc., such as in peripheral blood lymphocytes (PBLs). For the latter, inhibition of ERK phosphorylation can be measured following activation with phorbol myristate acetate using antibodies as described in the examples below.

In addition, patients having cancer can be selected and monitored on the basis of whether the tissue is experiencing neovacularization, and how much. This can be assessed as discussed above, e.g., using immunohistochemistry for vessel markers (e.g., CD31), circulating levels of a VGFR ligand, etc.

Patient selection and monitoring can also be made on the basis of the appearance in a body fluid (such as blood) above normal levels of the shedded ectodomains derived from the various receptors, including the extracellular portions of VEGFR-2, VEGFR-3, PDGFR-beta, and Flt-3. Detection methods can be carried out routinely, e.g., using antibodies which specifically bind to the extracellular domain.

Measuring expression includes determining or detecting the amount of the polypeptide present in a cell or shed by it, as well as measuring the underlying mRNA, where the quantity of mRNA present is considered to reflect the quantity of polypeptide manufactured by the cell. Furthermore, the genes for VEGFR-2, VEGFR-3, PDGFR-beta, and/or Flt-3 can be analyzed to determine whether there is a gene defect responsible for aberrant expression or polypeptide activity. Genes sequences are publically available; e.g., NM_004333 Homo sapiens v-raf murine sarcoma viral oncogene homolog B1 (BRAF); NM_004119 Homo sapiens fins-related tyrosine kinase 3 (FLT3); NM_002609 Homo sapiens platelet-derived growth factor receptor, beta polypeptide (PDGFRB); NM_002253 Homo sapiens VEGFR2; NM_182925 Homo sapiens fins-related tyrosine kinase 4 (FLT4); L35253 Homo sapiens p38 mitogen activated protein (MAP) Kinase.

Polypeptide detection can be carried out by any available method, e.g., by Western blots, ELISA, dot blot, immunoprecipitation, RIA, immunohistochemistry, etc. For instance, a tissue section can be prepared and labeled with a specific antibody (indirect or direct and visualized with a microscope. Amount of a polypeptide can be quantitated without visualization, e.g., by preparing a lysate of a sample of interest, and then determining by ELISA or Western the amount of polypeptide per quantity of tissue. Antibodies and other specific binding agents can be used. There is no limitation on how detection is performed.

Assays can be utilized which permit quantification and/or presence/absence detection of a target nucleic acid (e.g., genes, mRNA, etc., for raf, VEGFR, PDGFR, Flt-3, etc) in a sample. Assays can be performed at the single-cell level, or in a sample comprising many cells, where the assay is "averaging" expression over the entire collection of cells and tissue present in the sample. Any suitable assay format can be used, including, but not limited to, e.g., Southern blot analysis, Northern blot analysis, polymerase chain reaction ("PCR") (e.g., Saiki et al., Science, 241:53, 1988; U.S. Pat. Nos. 4,683,195, 4,683,202, and 6,040,166; PCR Protocols: A Guide to Methods and Applications, Innis et al., eds., Academic Press, New York, 1990), reverse transcriptase polymerase chain reaction ("RT-PCR"), anchored PCR, rapid amplification of cDNA ends ("RACE") (e.g., Schaefer in Gene Cloning and Analysis: Current Innovations, Pages 99-115, 1997), ligase chain reaction ("LCR") (EP 320 308), one-sided PCR (Ohara et al., Proc. Natl. Acad. Sci., 86:5673-5677, 1989), indexing methods (e.g., U.S. Pat. No. 5,508,169), in situ hybridization, differential display (e.g., Liang et al., Nucl. Acid. Res., 21:3269-3275, 1993; U.S. Pat. Nos. 5,262,311, 5,599,672 and 5,965,409; WO97/18454; Prashar and Weissman, Proc. Natl. Acad. Sci., 93:659-663, and U.S. Pat. Nos. 6,010,850 and 5,712,126; Welsh et al., Nucleic Acid Res., 20:4965-4970, 1992, and U.S. Pat. No. 5,487,985) and other RNA fingerprinting techniques, nucleic acid sequence based amplification ("NASBA") and other transcription based amplification systems (e.g., U.S. Pat. Nos. 5,409,818 and 5,554,527; WO 88/10315), polynucleotide arrays (e.g., U.S. Pat. Nos. 5,143,854, 5,424,186; 5,700,637, 5,874,219, and 6,054,270; PCT WO 92/10092; PCT WO 90/15070), Qbeta Replicase (PCT/US87/00880), Strand Displacement Amplification ("SDA"), Repair Chain Reaction ("RCR"), nuclease protection assays, subtraction-based methods, Rapid-Scan, etc. Additional useful methods include, but are not limited to, e.g., template-based amplification methods, competitive PCR (e.g., U.S. Pat. No. 5,747,251), redox-based assays (e.g., U.S. Pat. No. 5,871,918), Taqman-based assays (e.g., Holland et al., Proc. Natl. Acad, Sci., 88:7276-7280, 1991; U.S. Pat. Nos. 5,210,015 and 5,994,063), real-time fluorescence-based monitoring (e.g., U.S. Pat. 5,928,907), molecular energy transfer labels (e.g., U.S. Pat. Nos. 5,348,853, 5,532,129, 5,565,322, 6,030,787, and 6,117,635; Tyagi and Kramer, Nature Biotech., 14:303-309, 1996). Any method suitable for single cell analysis of gene or protein expression can be used, including in situ hybridization, immunocytochemistry, MACS, FACS, flow cytometry, etc. For single cell assays, expression products can be measured using antibodies, PCR, or other types of nucleic acid amplification (e.g., Brady et al., Methods Mol. & Cell. Biol. 2, 17-25, 1990; Eberwine et al., 1992, Proc. Natl. Acad. Sci., 89, 3010-3014, 1992; U.S. Pat. No. 5,723,290). These and other methods can be carried out conventionally, e.g., as described in the mentioned publications.

Activity of VEGFR-2, VEGFR-3, PDGFR-beta, and Flt-3 can be assessed routinely, e.g., as described in the examples below, or using standard assays for kinase activity (see, above).

Measuring expression includes evaluating the all aspects of the transcriptional and translational machinery of the gene. For instance, if a promoter defect causes, or is suspected of causing, the disorder, then a sample can be evaluated (i.e., "assessed") by looking (e.g., sequencing or restriction mapping) at the promoter sequence in the gene, by detecting transcription products (e.g., RNA), by detecting translation product (e.g., polypeptide). Any measure of whether the gene is functional can be used, including, polypeptide, polynucleotide, and functional assays for the gene's biological activity.

In making the assessment, it can be useful to compare the results to a gene which is not associated with the disorder, or to the same gene but in a unaffected tissue or region of the same tissue. The nature of the comparison can be determined routinely, depending upon how the assessing is accomplished. If, for example, the mRNA levels of a sample is detected, then the mRNA levels of a normal can serve as a comparison, or a gene which is known not to be affected by the disorder. Methods of detecting mRNA are well known, and discussed above, e.g., but not limited to, Northern blot analysis, polymerase chain reaction (PCR), reverse transcriptase PCR, RACE PCR, etc. Similarly, if polypeptide production is used to evaluate the gene, then the polypeptide in a normal tissue sample can be used as a comparison, or, polypeptide from a different gene whose expression is known not to be affected by the disorder. These are only examples of how such a method could be carried out.

Patients can also be selected for treatment if they have a particular genotype which is known to be associated with a cancer, especially genotypes which affect the Raf/Mek/Erk pathway, such as mutations in the BRAF, KRAS, or MEK genes. Along these lines, the the present invention relates to methods for selecting patients for treatment involving determining the the presence of a VEGFR-2, VEGFR-3, PDGFR-beta, and/or Flt-3 gene mutation in a sample obtained from a subject, wherein said mutation is associated with a disease, and administering said compound of formula I to subjects who are identified as having said mutation.

The presence of the mutation can be determined conventionally, e.g., obtaining cells or a tissue sample from a subject, extracting nucleic acid from it, determining the gene sequence or structure of a target gene (using, e.g., mRNA, cDNA, genomic DNA, etc), comparing the sequence or structure of the target gene to the structure of the normal gene, whereby a difference in sequence or structure indicates a mutation in the gene in the subject. Mutations can be determined using any effective method, e.g., comparing restriction maps, nucleotide sequences, amino acid sequences, RFLPs, DNAse sites, DNA methylation fingerprints (e.g., U.S. Pat. No. 6,214,556), protein cleavage sites, molecular weights, electrophoretic mobilities, charges, ion mobility, etc., between a standard gene and the subject's gene. Proteins can also be compared. To carry out such methods, all or part of the gene or polypeptide can be compared. For example, if nucleotide sequencing is utilized, the entire gene can be sequenced, including promoter, introns, and exons, or only parts of it can be sequenced and compared, e.g., exon 1, exon 2, etc.

The present invention also provides methods of assessing the efficacy of a compound of the present invention in treating a disease, comprising one or more of the following steps in any effective order, e.g., measuring the expression or activity of VEGFR-2, VEGFR-3, PDGFR-beta, and/or Flt-3 in a sample obtained from said subject who has been treated with a compound of the present invention, and determining the effects of said compound on said expression or activity. The measuring step can be carried out as described already.

For instance, biopsy samples can be removed from patients who have been treated with a compound of the present invention, and then assayed for the presence and/or activity of the mentioned signaling molecules. As discussed above, decreased levels of phospho-ERK in the cancer tissue (e.g., compared to a normal tissue or before treatment) indicate that the compound is exerting *in vivo* efficacy and a therapeutic effect.

Determining the effects of the compound on expression or activity includes performing a comparison step between a tissue sample and a control, or other type of standard. Examples of standards that can be used, include, but are not limited to, a tissue sample prior to treatment, a tissue sample from an unaffected tissue or from an unaffected region of the affected tissue (e.g., from a region of the tissue which is not transformed, cancerous, etc.), etc. A standard can also be a value, or range of values, that is representative of normal levels of expression that have been established for that marker. The comparison can also be made between samples collected from at least two different timepoints during the treatment regimen with a compound of the present invention. For example, samples can be collected from various times after initiation of the drug treatment, and analysis of expression and/or activity levels can be used to monitor the progress/prognosis of the subject, e.g., how the subject is responding to the drug regimen. Any timepoint can be used, e.g., daily, twice a week, weekly, every two weeks, every month, yearly, a plurality of timepoints (at least 2, 3, 4, 8, 12, etc.).

The phrase "determining the effect" indicates that the result produced by the compound is analyzed and/or identified. For instance, in Example 3, data is shown that compound reduced the levels of phospho-ERK (i.e., the effect of the compound on raf activity was determined by measuring phospho-ERK). Any type of effect can be identified, e.g., where the expression and/or activity is reduced, decreased, downregulated, inhibited, blocked, increased, up-regulated, unchanged, etc.

The method can be used to determine appropriate dosages and dosing regimens, e.g., how much compound to administer and at what frequency to administer it. By monitoring its effect on the signaling molecules in the tissue, the clinician can determine the appropriate treatment protocol and whether it is achieving the desired effect, e.g., on modulating or inhibiting the signal transduction pathway. For instance, if the compound is not effective in knocking down the amounts of a marker, such as phospho-ERK, the dosage can be increased in the patient or given more frequently. Similarly, dosages and/or frequency can be reduced when it is shown that the compound is effective in knocking down the levels of phospho-ERK or other marker for the disease state. Since the compounds can be administered in combination with others treatments, e.g., radiation, chemotherapy, and other agents, the monitoring of the subject can be used to assess the combined effects of the treatment regimen on the progress of the disease.

Examples of mutations, include mutations in K-RAS; mutations in the BRAF gene, such as mutations at position 599, such as V599E, and positions 461, 462, 463, 465, 468, 593, 596, 60, etc., which are associated with cancers, such as melanoma.

The specific compounds also can be used as markers to determine the presence and quantity of VEGFR-2, VEGFR-3? PDGFR-bta, and/or Flt-3. Methods can involve the presence of VEGFR-2, VEGFR-3, PDGFR-bets, and/or Flt-3 in a sample comprising a biological material, comprising one or more of the following steps in any effective order, e.g., contacting said sample comprising a biological material with a compound of the present invention, and determining whether said compound binds to said material. The compound can be labeled, or it can be used as a competitor to a labeled compound, such as labeled-ATP.

The invention also provides the assessment of specific compounds for treating, preventing, modulating,etc., diseases and conditions in mammals comprising the assessment of a specific compound with another modulator of the signal transduction pathway comprising, but not limited to VEGFR, PDGPR, and/or FLT-3. These can be present an the same composition or in separate formulations or dosage units. Administration can be the same or different routes, and can be simultaneous, sequential, etc.

The invention also relates to the assessment of specific compounds for treating, preventing, modulating, etc., diseases and conditions, comprising the assessment of a specific compound with another active agent, e.g., once or more per day for up to 28 consecutive days with the concurrent or intermittent use of another active agent over the same total time period.

Optional anti-hyper-proliferative agents which can be added to the composition include but are not limited to compounds listed on the cancer chemotherapy drug regimens in the 11$^{th}$ Edition of the *Merck Index,* (1996), which is hereby incorporated by reference, such as asparaginase, bleomycin, carboplatin, carmustine chlorambucil, cisplatin, colaspase, cyclophosphamide, cytarabine, dacarbazine, dactinomycin, daunorubicin, doxorubicin (adriamycine), epirubicin, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other anti-hyper-proliferative agents suitable for use with the composition of the invention include, but are not limited to, those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996), which is hereby incorporated by reference, such as aminoglutethimide, L-asparaginase, azathioprine, 5-azacytidine cladribine, busulfan, diethylstilbestrol, 2',2'-difluorodeoxycytidine, docetaxel, erythrohydroxynonyladenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

The specific compounds can be administered in any form by any effective route, including, e.g., oral, parenteral, enteral, intravenous, intraperitoneal, topical, transdermal (e.g., using any standard patch), ophthalmic, nasally, local, non-oral, such as aerosal, inhalation, subcutaneous, intramuscular, buccal, sublingual, rectal, vaginal, intra-arterial, and intrathecal, etc. They can be administered alone, or in combination with any ingredient(s), active or inactive. They can be administered in any effective dosage, e.g., from about 0.1 to about 200 mg/kg of total body weight.

The present invention relates to the assessement of specific compounds described above (Compounds of Formula I), including salts and esters thereof and compositions thereof, to treat mammalian hyper-proliferative disorders. The use of these specific compounds comprise administering to a mammal in need thereof, including a human, an amount of a specific compound, or a pharmaceutically acceptable salt or ester thereof, which is effective to treat the disorder. Hyper-proliferative disorders include but are not limited to solid tumors, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukemias.

Synthetic transformations that may be employed in the synthesis of compounds of Formula I and in the synthesis of intermediates involved in the synthesis of compounds of Formula I are known by or accessible to one skilled in the art. Collections of synthetic transformations may be found in compilations, such as:

- J. March. Advanced Organic Chemistry, 4th ed.; John Wiley: New York (1992)
- R.C. Larock. Comprehensive Organic Transformations, 2nd ed.; Wiley-VCH: New York (1999)
- F.A. Carey; R.J. Sundberg. Advanced Organic Chemistry, 2nd ed.; Plenum Press: New York (1984)
- T.W. Greene; P.G.M. Wuts. Protective Groups in Organic Synthesis, 3rd ed.; John Wiley: New York (1999)
- L.S. Hegedus. Transition Metals in the Synthesis of Complex Organic Molecules, 2nd ed.; University Science Books: Mill Valley, CA (1994)
- L.A. Paquette, Ed. The Encyclopedia of Reagents for Organic Synthesis; John Wiley: New York (1994)
- A.R. Katritzky; O. Meth-Cohn; C.W. Rees, Eds. Comprehensive Organic Functional Group Transformations; Pergamon Press: Oxford, UK (1995)
- G. Wilkinson; F.G A. Stone; E.W. Abel, Eds. Comprehensive Organometallic Chemistry; Pergamon Press: Oxford, UK (1982)
- B.M. Trost; I. Fleming. Comprehensive Organic Synthesis; Pergamon Press: Oxford, UK (1991)
- A.R. Katritzky; C.W. Rees Eds. Comprehensive Heterocylic Chemistry; Pergamon Press: Oxford, UK (1984)
- A.R. Katritzky; C.W. Rees; E.F.V. Scriven, Eds. Comprehensive Heterocylic Chemistry II; Pergamon Press: Oxford, UK (1996)
- C. Hansch; P.G. Sammes; J.B. Taylor, Eds. Comprehensive Medicinal Chemistry: Pergamon Press: Oxford, UK (1990).

In addition, recurring reviews of synthetic methodology and related topics include Organic Reactions; John Wiley: New York; Organic Syntheses; John Wiley: New York; Reagents for Organic Synthesis: John Wiley: New York; The Total Synthesis of Natural Products; John Wiley: New York; The Organic Chemistry of Drug Synthesis; John Wiley: New York; Annual Reports in Organic Synthesis; Academic Press: San Diego CA; and Methoden der Organischen Chemie (Houben-Weyl); Thieme: Stuttgart, Germany. Furthermore, databases of synthetic transformations include *Chemical Abstracts,* which may be searched using either CAS OnLine or SciFinder, *Handbuch der Organischen Chemie* (Beilstein), which may be searched using SpotFire, and REACCS.

### *General Preparative Methods*

The diaryl ureas of Formula I may be prepared by the use of known chemical reactions and procedures, some from starting materials which are commercially available. Nevertheless, general preparative methods are provided below to aid one skilled in the art in synthesizing these compounds, with more detailed examples being provided in the Experimental section which follows.

Substituted anilines may be generated using standard methods (March. Advanced Organic Chemistry, 3rd Ed.; John Wiley: New York (1985). Larock. Comprehensive Organic Transformations; VCH Publishers: New York (1989)). As shown in Scheme I, aryl amines are commonly synthesized by reduction of nitroaryls using a metal catalyst, such as Ni, Pd, or Pt, and $H_2$ or a hydride transfer agent, such as formate, cyclohexadiene, or a borohydride (Rylander. Hydrogenation Methods; Academic Press: London, UK (1985)). Nitroaryls may also be directly reduced using a strong hydride source, such as $LiAlH_4$ (Seyden-Penne. Reductions by the Alumino- and Borohydrides in Organic Synthesis; VCH Publishers: New York (1991)), or using a zero valent metal, such as Fe, Sn or Ca, often in acidic media. Many methods exist for the synthesis of nitroaryls (March. Advanced Organic Chemistry, 3rd Ed.; John Wiley: New York (1985). Larock. Comprehensive Organic Transformations; VCH Publishers: New York (1989)).

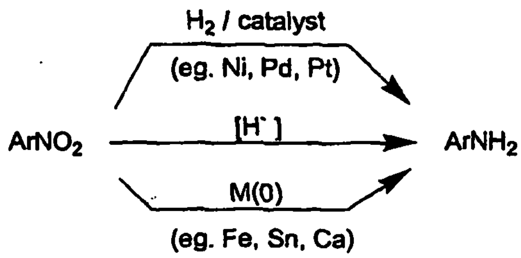


**Scheme I Reduction of Nitroaryls to Aryl Amines**

Nitroaryls are commonly formed by electrophilic aromatic nitration using $HNO_3$, or an alternative $NO_2^+$ source. Nitroaryls may be further elaborated prior to reduction. Thus, nitroaryls substituted with

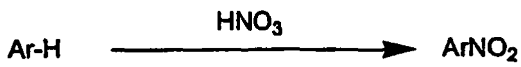


potential leaving groups (e.g. F, Cl, Br, etc.) may undergo substitution reactions on treatment with nucleophiles, such as thiolate (exemplified in Scheme II) or phenoxide. Nitroaryls may also undergo Ullman-type coupling reactions (Scheme II).

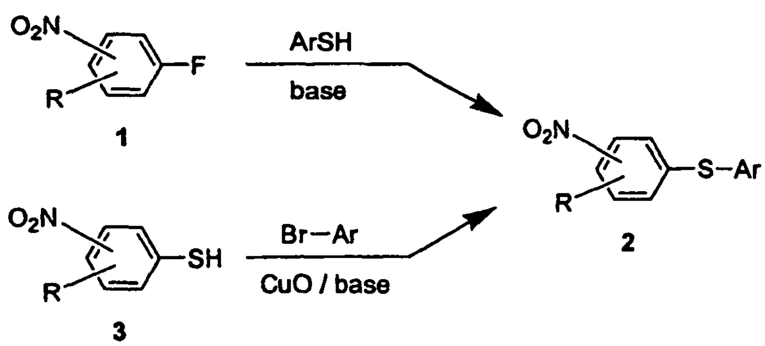


**Scheme II Selected Nucleophilic Aromatic Substitution using Nitroaryls**

Nitroaryls may also undergo transition metal mediated cross coupling reactions. For example, nitroaryl electrophiles, such as nitroaryl bromides, iodides or triflates, undergo palladium mediated cross coupling reactions with aryl nucleophiles, such as arylboronic acids (Suzuki reactions, exemplified below), aryltins (Stille reactions) or arylzincs (Negishi reaction) to afford the biaryl (5).

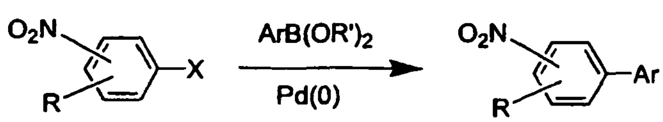


4 5

As shown in Scheme III, non-symmetrical urea formation may involve reaction of an aryl isocyanate (14) with an aryl amine (13). The heteroaryl isocyanate may be synthesized from a heteroaryl amine by treatment with phosgene or a phosgene equivalent, such as trichloromethyl chloroformate (diphosgene), bis(trichloromethyl) carbonate (triphosgene), or *N*,*N*'-carbonyldiimidazole (CDI). The isocyanate may also be derived from a heterocyclic carboxylic acid derivative,

such as an ester, an acid halide or an anhydride by a Curtius-type rearrangement. Thus, reaction of acid derivative 16 with an azide source, followed by rearrangement affords the isocyanate. The corresponding carboxylic acid (17) may also be subjected to Curtius-type rearrangements using diphenylphosphoryl azide (DPPA) or a similar reagent.

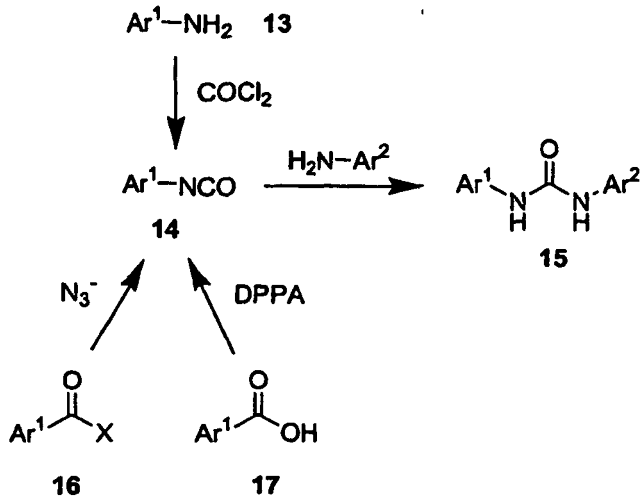


**Scheme III Selected Methods of Non-Symmetrical Urea Formation**

Finally, ureas may be further manipulated using methods familiar to those skilled in the art.

The compounds may be administered orally, topically, parenterally, by inhalation or spray or rectally in dosage unit formulations. The term 'administration by injection' includes intravenous, intramuscular, subcutaneous and parenteral injections, as well as use of infusion techniques. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropyl methylcellulose, sodium alginate, polyvinylpyrrolidone, gum tragacanth and gum acacia; dispersing or wetting agents may be a naturally occurring phosphatide, for example, lecithin, or condensation products or an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned

above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter and polyethylene glycols.

The specific compounds may also be administered parenterally, that is, subcutaneously, intravenously, intraocularly, intrasynovially, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulation ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethylcellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution,

isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

The specific compounds may also be administrated transdermally using methods ("patches") known to those skilled in the art (see, for example: Chien; "Transdermal Controlled Systemic Medications"; Marcel Dekker, Inc.; 1987. Lipp et al. WO94/04157 3Mar94). Such transdermal patches may be used to provide continuous or discontinuous infusion of the compounds of the present invention in controlled amounts. The construction and use of transdermal patches for the delivery of pharmaceutical agents is well known in the art (see, e.g., US Patent No. 5,023,252, issued June 11, 1991). Such patches may be constructed for continuous, pulsatile, or on demand delivery of pharmaceutical agents. For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated $C_8$-$C_{18}$ fatty alcohols such as lauryl alcohol or cetyl alcohol, saturated or unsaturated $C_8$-$C_{18}$ fatty acids such as stearic acid, saturated or unsaturated fatty esters with up to 24 carbons such as methyl, ethyl, propyl, isopropyl, n-butyl, sec-butyl isobutyl tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated $C_8$-$C_{18}$ fatty alcohols, saturated or unsaturated $C_8$-$C_{18}$ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates may also be used as matrix components. Additional additives, such as viscous resins or oils may be added to increase the viscosity of the matrix.

Controlled release formulations for parenteral administration include liposomal, polymeric microsphere and polymeric gel formulations which are known in the art.

It may be desirable or necessary to introduce the pharmaceutical composition to the patient via a mechanical delivery device. The construction and use of mechanical delivery devices for the delivery of pharmaceutical agents is well known in the art. Direct techniques for, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US Patent No. 5,011,472, issued April 30, 1991.

The compositions can also contain other conventional pharmaceutically acceptable compounding ingredients, generally referred to as carriers or diluents, as necessary or desired. Conventional procedures for preparing such compositions in appropriate dosage forms can be utilized. Such ingredients and procedures include those described in the following references, each of which is incorporated herein by reference: Powell, M.F. et al, "Compendium of Excipients for Parenteral Formulations" PDA Journal of Pharmaceutical Science & Technology 1998, 52(5), 238-311; Strickley, R.G "Parenteral Formulations of Small Molecule Therapeutics Marketed in the United States (1999)-Part-1" PDA Journal of Pharmaceutical Science & Technology 1999, 53(6), 324-349; and Nema, S. et al, "Excipients and Their Use in Injectable Products" PDA Journal of Pharmaceutical Science & Technology 1997, 51(4), 166-171.

These compositions can be utilized to achieve the desired pharmacological effect by administration to a patient in need thereof. A patient, for the purpose of this invention, is a mammal, including a human, in need of treatment for the particular condition or disease. Therefore, the present invention includes pharmaceutical compositions which are comprised of a pharmaceutically acceptable carrier and a pharmaceutically effective amount of a compound, or salt

thereof, of the present invention. A pharmaceutically acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is that amount which produces a result or exerts an influence on the particular condition being treated. The compounds of the present invention can be administered with pharmaceutically acceptable carriers well known in the art using any effective conventional dosage unit forms, including immediate, slow and timed release preparations, orally, parenterally, topically, nasally, ophthalmically, otically, sublingually, rectally, vaginally, and the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule which can be of the ordinary hard- or soft-shelled gelatin type containing, for example, surfactants, lubricants, and inert fillers such as lactose, sucrose, calcium phosphate, and corn starch.

In another embodiment, the specific compounds may be tableted with conventional tablet bases such as lactose, sucrose and cornstarch in combination with binders such as acacia, corn starch or gelatin, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, coloring agents, and flavoring agents such as peppermint, oil of wintergreen, or cherry flavoring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance tablets, pills or capsules may be coated with shellac, sugar or both.

The compositions can also contain other conventional pharmaceutically acceptable compounding ingredients, generally referred to as carriers or diluents, as necessary or desired. Conventional procedures for preparing such compositions in appropriate dosage forms can be utilized. Such ingredients and procedures include those described in the following references, each of which is incorporated herein by reference: Powell, M.F. et al, "Compendium of Excipients for Parenteral Formulations" PDA Journal of Pharmaceutical Science & Technology 1998, 52(5), 238-311; Strickley, R.G "Parenteral Formulations of Small Molecule Therapeutics Marketed in the United States (1999)-Part-1" PDA Journal of Pharmaceutical Science & Technology 1999, 53(6), 324-349; and Nema, S. et al, "Excipients and Their Use in Injectable Products" PDA Journal of Pharmaceutical Science & Technology 1997, 51(4), 166-171.

Commonly used pharmaceutical ingredients which can be used as appropriate to formulate the composition for its intended route of administration include:

**acidifying agents** (examples include but are not limited to acetic acid, citric acid, fumaric acid, hydrochloric acid, nitric acid);

**alkalinizing agents** (examples include but are not limited to ammonia solution, ammonium carbonate, diethanolamine, monoethanolamine, potassium hydroxide, sodium borate, sodium carbonate, sodium hydroxide, triethanolamine, trolamine);

**adsorbents** (examples include but are not limited to powdered cellulose and activated charcoal);

**aerosol propellants** (examples include but are not limited to carbon dioxide, $CCl_2F_2$, $F_2ClC$-$CClF_2$ and $CClF_3$)

**air displacement agents** (examples include but are not limited to nitrogen and argon);

**antifungal preservatives** (examples include but are not limited to benzoic acid, butylparaben, ethylparaben, methylparaben, propylparaben, sodium benzoate);

**antimicrobial preservatives** (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);

**antioxidants** (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);

**binding materials** (examples include but are not limited to block polymers, natural and synthetic rubber, polyacrylates, polyurethanes, silicones, polysiloxanes and styrene-butadiene copolymers);

**buffering agents** (examples include but are not limited to potassium metaphosphate, dipotassium phosphate, sodium acetate, sodium citrate anhydrous and sodium citrate dihydrate)

**carrying agents** (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection)

**chelating agents** (examples include but are not limited to edetate disodium and edetic acid)
**colorants** (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);
**clarifying agents** (examples include but are not limited to bentonite);
**emulsifying agents** (examples include but are not limited to acacia, cetomacrogol, cetyl alcohol, glyceryl monostearate, lecithin, sorbitan monooleate, polyoxyethylene 50 monostearate);
**encapsulating agents** (examples include but are not limited to gelatin and cellulose acetate phthalate)
**flavorants** (examples include but are not limited to anise oil, cinnamon oil, cocoa, menthol, orange oil, peppermint oil and vanillin);
**humectants** (examples include but are not limited to glycerol, propylene glycol and sorbitol);
**levigating agents** (examples include but are not limited to mineral oil and glycerin);
**oils** (examples include but are not limited to arachis oil, mineral oil, olive oil, peanut oil, sesame oil and vegetable oil);
**ointment bases** (examples include but are not limited to lanolin, hydrophilic ointment, polyethylene glycol ointment, petrolatum, hydrophilic petrolatum, white ointment, yellow ointment, and rose water ointment);
**penetration enhancers (transdermal delivery)** (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas)
**plasticizers** (examples include but are not limited to diethyl phthalate and glycerol);
**solvents** (examples include but are not limited to ethanol, corn oil, cottonseed oil, glycerol, isopropanol, mineral oil, oleic acid, peanut oil, purified water, water for injection, sterile water for injection and sterile water for irrigation);
**stiffening agents** (examples include but are not limited to cetyl alcohol, cetyl esters wax, microcrystalline wax, paraffin, stearyl alcohol, white wax and yellow wax);
**suppository bases** (examples include but are not limited to cocoa butter and polyethylene glycols (mixtures));
**surfactants** (examples include but are not limited to benzalkonium chloride, nonoxynol 10, oxtoxynol 9, polysorbate 80, sodium lauryl sulfate and sorbitan monopalmitate);
**suspending agents** (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
**sweetening agents** (examples include but are not limited to aspartame, dextrose, glycerol, mannitol, propylene glycol, saccharin sodium, sorbitol and sucrose);
**tablet anti-adherents** (examples include but are not limited to magnesium stearate and talc);
**tablet binders** (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
**tablet and capsule diluents** (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
**tablet coating agents** (examples include but are not limited to liquid glucose, hydroxyethyl cellulose, hydroxypropyl cellulose, hydroxypropyl methylcellulose, methylcellulose, ethylcellulose, cellulose acetate phthalate and shellac);
**tablet direct compression excipients** (examples include but are not limited to dibasic calcium phosphate);
**tablet disintegrants** (examples include but are not limited to alginic acid, carboxymethylcellulose calcium, microcrystalline cellulose, polacrillin potassium, cross-linked polyvinylpyrrolidone, sodium alginate, sodium starch glycollate and starch);
**tablet glidants** (examples include but are not limited to colloidal silica, corn starch and talc);
**tablet lubricants** (examples include but are not limited to calcium stearate, magnesium stearate, mineral oil, stearic acid and zinc stearate);
**tablet/capsule opaquants** (examples include but are not limited to titanium dioxide);
**tablet polishing agents** (examples include but are not limited to carnauba wax and white wax);
**thickening agents** (examples include but are not limited to beeswax, cetyl alcohol and paraffin);
**tonicity agents** (examples include but are not limited to dextrose and sodium chloride);
**viscosity increasing agents** (examples include but are not limited to alginic acid, bentonite, carbomers, carboxymethylcellulose sodium, methylcellulose, polyvinyl pyrrolidone, sodium alginate and tragacanth); and
**wetting agents** (examples include but are not limited to heptadecaethylene oxycetanol, lecithins, sorbitol monooleate, polyoxyethylene sorbitol monooleate, and polyoxyethylene stearate).

The total amount of the active ingredient to be administered will generally range from about 0.001 mg/kg to about 200 mg/kg, and preferably from about 0.01 mg/kg to about 20 mg/kg body weight per day. A unit dosage may contain from

about 0.5 mg to about 1500 mg of active ingredient, and can be administered one or more times per day. For all regimens of use disclosed herein for compounds of Formula I, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily rectal dosage regime will preferably be from 0.01 to 200 mg/Kg of total body weight. The daily vaginal dosage regime will preferably be from 0.01 to 200 mg/Kg of total body weight. The daily topical dosage regime will preferably be from 0.1 to 200 mg administered between one to four times daily. The transdermal concentration will preferably be that required to maintain a daily dose of from 0.01 to 200 mg/Kg. The daily inhalation dosage regime will preferably be from 0.01 to 100 mg/Kg of total body weight. These dosages regimes can be achieved with multiple dosages within a single day or extended dosages, such as those given on a weekly or monthly basis.
Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and gender of the patient treated, and the nature and extent of the condition treated.

It will be appreciated by those skilled in the art that the particular method of administration will depend on a variety of factors, all of which are considered routinely when administering therapeutics. It will also be appreciated by one skilled in the art that the specific dose level for a given patient depends on a variety of factors, including specific activity of the compound administered, age, body weight, health, sex, diet, time and route of administration, rate of excretion, etc. It will be further appreciated by one skilled in the art that the optimal course of treatment, i.e., the mode of treatment and the daily number of doses of a compound of Formula I or a pharmaceutically acceptable salt thereof given for a defined number of days, can be ascertained by those skilled in the art using conventional treatment tests.
It will be understood, however, that the specific dose level for any particular patient will depend upon a variety of factors, including the activity of the specific compound employed, the age, body weight, general health, sex, diet, time of administration, route of administration, and rate of excretion, drug combination and the severity of the condition undergoing therapy.

It will be further appreciated by one skilled in the art that the optimal course of treatment, i.e., the mode of treatment and the daily number of doses of a compound of this invention given for a defined number of days, can be ascertained by those skilled in the art using conventional treatment tests.

Specific preparations of the specific compounds are already described in the patent literature, and can be adapted to the compounds of the present invention. For example, Riedl, B., et al., "O-Carboxy Aryl Substituted Diphenyl Ureas as raf Kinase Inhibitors" *PCT Int. Appl.,* WO 00 42012, Riedl, B., et al, "O-Carboxy Aryl Substituted Diphenyl Ureas as p38 Kinase Inhibitors" *PCT Int. Appl.,* WO 00 41698.

Pharmaceutical compositions can be illustrated as follows

**Sterile IV Solution:** A 5 mg/ml solution of the desired compound of this invention is made using sterile, injectable water, and the pH is adjusted if necessary. The solution is diluted for administration to 1- 2 mg/ml with sterile 5% dextrose and is administered as an IV infusion over 60 minutes.

**Lyophilized powder for IV administration:** A sterile preparation can be prepared with (i) 100 - 1000 mg of the desired compound of this invention as a lyophilized powder, (ii) 32- 327 mg/ml sodium citrate, and (iii) 300 - 3000 mg Dextrin 40. The formulation is reconstituted with sterile, injectable saline or dextrose 5% to a concentration of 10 to 20 mg/ml, which is further diluted with saline or dextrose 5% to 0.2 - 0.4 mg/ml, and is administered either IV bolus or by IV infusion over 15 - 60 minutes.

**Intramuscular suspension:** The following solution or suspension can be prepared, for intramuscular injection:

50 mg/ml of the desired, water-insoluble compound of this invention
5 mg/ml sodium carboxymethylcellulose
4 mg/ml TWEEN 80
9 mg/ml sodium chloride
9 mg/ml benzyl alcohol

**Hard Shell Capsules:** A large number of unit capsules are prepared by filling standard two-piece hard galantine capsules each with 100 mg of powdered active ingredient, 150 mg of lactose, 50 mg of cellulose and 6 mg of magnesium

stearate.

**Soft Gelatin Capsules:** A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.

**Tablets:** A large number of tablets are prepared by conventional procedures so that the dosage unit was 100 mg of active ingredient, 0.2 mg. of colloidal silicon dioxide, 5 mg of magnesium stearate, 275 mg of microcrystalline cellulose, 11 mg. of starch, and 98.8 mg of lactose. Appropriate aqueous and non-aqueous coatings may be applied to increase palatability, improve elegance and stability or delay absorption.

**Immediate Release Tablets/Capsules:** These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.
Methods for preparing the compounds of this invention are also described in the following U.S. applications:.

09/425,228, filed October 22, 1999;
09/722,418 filed November 28, 2000
09/758,547, filed January 12, 2001;
09/838,285, filed April 20, 2001;
09/838,286, filed April 20, 2001; and

The compounds can be produced from know compounds (or from starting materials which, in turn, can be produced from known compounds), e.g., through the general preparative methods shown below. The activity of a given compound to inhibit raf kinase can be routinely assayed, e.g., according to procedures disclosed below. The following examples are for illustrative purposes only and are not intended, nor should they be construed to limit the invention in any way.

## EXAMPLES

All reactions were performed in flame-dried or oven-dried glassware under a positive pressure of dry argon or dry nitrogen, and were stirred magnetically unless otherwise indicated. Sensitive liquids and solutions were transferred via syringe or cannula, and introduced into reaction vessels through rubber septa. Unless otherwise stated, the term 'concentration under reduced pressure' refers to use of a Buchi rotary evaporator at approximately 15 mmHg. Unless otherwise stated, the term 'under high vacuum' refers to a vacuum of 0.4 -1.0 mmHg.

All temperatures are reported uncorrected in degrees Celsius (˚C). Unless otherwise indicated, all parts and percentages are by weight.

Commercial grade reagents and solvents were used without further purification. N-cyclohexyl-*N*'-(methylpolystyrene)carbodiimide was purchased from Calbiochem-Novabiochem Corp. 3-*tert*-Butylaniline, 5-*tert*-butyl-2-methoxyaniline, 4-bromo-3-(trifluoromethyl)aniline, 4-chloro-3-(trifluoromethyl)aniline 2-methoxy-5-(trifluoromethyl)aniline, 4-*tert*-butyl-2-nitroaniline, 3-amino-2-naphthol, ethyl 4-isocyanatobenzoate, *N*-acetyl-4-chloro-2-methoxy-5-(bifluoromethyl)aniline and 4-chloro-3-(trifluoromethyl)phenyl isocyanate were purchased and used without further purification. Syntheses of 3-amino-2-methoxyquinoline (E. Cho et al. WO 98/00402; A. Cordi et al. EP 542,609; IBID Bioorg. Med Chem.. 3, 1995, 129), 4-(3-carbamoylphenoxy)-1-nitrobenzene (K. Ikawa Yakugaku Zasshi 79,1959, 760; Chem. Abstr. 53, 1959, 12761b), 3-tert-butylphenyl isocyanate (O. Rohr et al. DE 2,436,108) and 2-methoxy-5-(trifluoromethyl)phenyl isocyanate (K. Inukai et al. JP 42,025,067; IBID Kogyo Kagaku Zasshi 70,1967, 491) have previously been described.

Thin-layer chromatography (TLC) was performed using Whatman® pre-coated glass-backed silica gel 60A F-254 250 μm plates. Visualization of plates was effected by one or more of the following techniques: (a) ultraviolet illumination, (b) exposure to iodine vapor, (c) immersion of the plate in a 10% solution of phosphomolybdic acid in ethanol followed by heating, (d) immersion of the plate in a cerium sulfate solution followed by heating, and/or (e) immersion of the plate in an acidic ethanol solution of 2,4-dinitrophenylhydrazine followed by heating. Column chromatography (flash chromatography) was performed using 230-400 mesh EM Science® silica gel.

Melting points (mp) were determined using a Thomas-Hoover melting point apparatus or a Mettler FP66 automated melting point apparatus and are uncorrected. Fourier transform infrared spectra were obtained using a Mattson 4020 Galaxy Series spectrophotometer. Proton ($^1H$) nuclear magnetic resonance (NMR) spectra were measured with a General Electric GN-Omega 300 (300 MHz) spectrometer with either $Me_4Si$ (δ 0.00) or residual protonated solvent ($CHCl_3$ δ 7.26; MeOH δ 3.30; DMSO δ 2.49) as standard. Carbon ($^{13}C$) NMR spectra were measured with a General

Electric GN-Omega 300 (75 MHz) spectrometer with solvent ($CDCl_3$ δ 77.0; MeOD-$d_3$; δ 49.0; DMSO-$d_6$ δ 39.5) as standard. Low-resolution mass spectra (MS) and high resolution mass spectra (HRMS) were either obtained as electron impact (EI) mass spectra or as fast atom bombardment (FAB) mass spectra. Electron impact mass spectra (EI-MS) were obtained with a Hewlett Packard 5989A mass spectrometer equipped with a Vacumetrics Desorption Chemical Ionization Probe for sample introduction. The ion source was maintained at 250 ˚C. Electron impact ionization was performed with electron energy of 70 eV and a trap current of 300 μA. Liquid-cesium secondary ion mass spectra (FAB-MS), an updated version of fast atom bombardment were obtained using a Kratos Concept 1-H spectrometer. Chemical ionization mass spectra (CI-MS) were obtained using a Hewlett Packard MS-Engine (5989A) with methane or ammonia as the reagent gas ($1x10^{-4}$ torr to $2.5x10^{-4}$ torr). The direct insertion desorption chemical ionization (DCI) probe (Vaccumetrics, Inc.) was ramped from 0-1.5 amps in 10 sec and held at 10 amps until all traces of the sample disappeared (~1-2 min). Spectra were scanned from 50-800 amu at 2 sec per scan. HPLC - electrospray mass spectra (HPLC ES-MS) were obtained using a Hewlett-Packard 1100 HPLC equipped with a quaternary pump, a variable wavelength detector, a C-18 column, and a Finnigan LCQ ion trap mass spectrometer with electrospray ionization. Spectra were scanned from 120-800 amu using a variable ion time according to the number of ions in the source. Gas chromatography - ion selective mass spectra (GC-MS) were obtained with a Hewlett Packard 5890 gas chromatograph equipped with an HP-1 methyl silicone column (0.33 mM coating; 25 m x 0.2 mm) and a Hewlett Packard 5971 Mass Selective Detector (ionization energy 70 eV). Elemental analyses are conducted by Robertson Microlit Labs, Madison NJ.

All compounds displayed NMR spectra, LRMS and either elemental analysis or HRMS consistent with assigned structures.

**List of Abbreviations and Acronyms:**

| | |
|---|---|
| AcOH | acetic acid |
| anh | anhydrous |
| atm | atmosphere(s) |
| BOC | *tert*-butoxycarbonyl |
| CDI | 1,1'-carbonyl diimidazole |
| conc | concentrated |
| d | day(s) |
| dec | decomposition |
| DMAC | *N,N*-dimethylacetamide |
| DMPU | 1,3-dimethyl-3,4,5,6-tetrahydro-2(1H)-pyrimidinone |
| DMF | *N,N*-dimethylformamide |
| DMSO | dimethylsulfoxide |
| DPPA | diphenylphosphoryl azide |
| EDCI | 1-(3-dimethylaminopropyl)-3-ethylcarbodiimide |
| EtOAc | ethyl acetate |
| EtOH | ethanol (100%) |
| $Et_2O$ | diethyl ether |
| $Et_3N$ | triethylamine |
| h | hour(s) |
| HOBT | 1-hydroxybenzotriazole |
| *m*-CPBA | 3-chloroperoxybenzoic acid |
| MeOH | methanol |
| pet. ether | petroleum ether (boiling range 30-60 ˚C) |
| temp. | temperature |
| THF | tetrahydrofuran |
| TFA | trifluoroAcOH |
| Tf | trifluoromethanesulfonyl |

The following general methods are described in copending application serial number 09/948,915, filed September 10, 2001, and are hereby incorporated by reference.

A. General Methods for Synthesis of Substituted Anilines, pages 18-43
B. Synthesis of Urea Precursors, page 43,
C. Methods of Urea Formation, pages 44-51 and

D. Interconversion of Ureas, pages 52-56.

**EXAMPLE A**

N-[4-chloro-3-(trifluoromethyl)phenyl]-N'-{4-[2-carbamoyl-(4-yridyloxy)]phenyl} urea

Step 1: Preparation of 4-chloro-2-pyridinecarboxamide

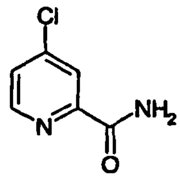

To a stirred mixture of methyl 4-chloro-2-pyridinecarboxylate hydrochloride (1.0 g, 4.81 mmol) dissolved in conc. aqueous ammonia (32 mL) was added ammonium chloride (96.2 mg, 1.8 mmol, 0.37 equiv.), and the heterogeneous reaction mixture was stirred at ambient temperature for 16h. The reaction mixture was poured into EtOAc (500 mL) and water (300 mL). The organic layer was washed with water (2 x 300 mL) and a saturated NaCl solution (1 x 300 mL), dried ($MgSO_4$), concentrated in *vacuo* to give 4-chloro-2-pyridinecarboxamide as a beige solid (604.3 mg, 80.3%): TLC (50% EtOAc / hexane) $R_f$ 0.20; $^1$H-NMR (DMSO-$d_6$) δ 8.61 (d, J = 5.4 Hz, 1H), 8.20 (broad s, 1H), 8.02 (d, J = 1.8 Hz, 1H), 7.81 (broad s, 1H), 7.76 to 7.73 (m, 1H).

Step 2: Preparation of 4-(4-aminophenoxy)-2-pyridinecarboxamide

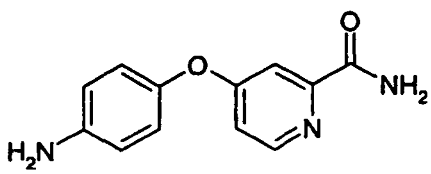

To 4-aminophenol (418 mg, 3.83 mmol) in anh DMF(7.7 mL) was added potassium *tert*-butoxide (447 mg, 3.98 mmol, 1.04 equiv.) in one portion. The reaction mixture was stirred at room temperature for 2 h, and a solution of 4-chloro-2-pyridinecarboxamide (600 mg, 3.83 mmol, 1.0 equiv.) in anh DMF (4 mL) was then added. The reaction mixture was stirred at 80 ˚C for 3 days and poured into a mixture of EtOAc and a saturated NaCl solution. The organic layer was sequentially washed with a saturated $NH_4Cl$ solution then a saturated NaCl solution, dried ($MgSO_4$), and concentrated under reduced pressure. The crude product was purified using MPLC chromatography (Biotage®; gradient from 100% EtOAc to followed by 10% MeOH / 50% EtOAc / 40% hexane) to give the 4-chloro-5-trifluoromethylaniline as a brown solid (510 mg, 58%). $^1$H-NMR (DMSO-$d_6$) δ 8.43 (d, J = 5.7 Hz, 1H), 8.07 (br s, 1H), 7.66 (br s, 1H), 7.31 (d, J = 2.7 Hz, 1H), 7.07 (dd, J = 5.7 Hz, 2.7 Hz, 1H), 6.85 (d, J = 9.0 Hz, 2 H), 6.62 (d, J = 8.7 Hz, 2H), 5.17 (broad s, 2H); HPLC EI-MS *m/z* 230 ($(M+H)^+$.

Step 3: Preparation of N-[4-chloro-3-(trifluoromethyl)phenyl]-N'-{4-[2-carbamoyl-(4-pyridyloxy)]phenyl} urea

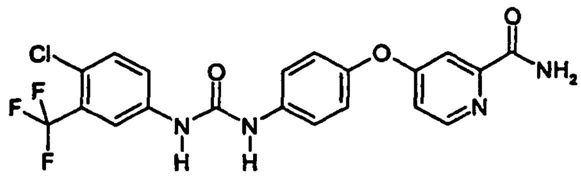

A mixture of 4-chloro-5-trifluoromethylaniline (451 mg, 2.31 mmol, 1.1 equiv.) and 1,1'-carbonyl diimidazole (419 mg, 2.54 mmol, 1.2 equiv.) in anh dichloroethane (5.5 mL) was stirred under argon at 65 °C for 16 h. Once cooled to room temperature, a solution of 4-(4-aminophenoxy)-2-pyridinecarboxamide (480 mg, 2.09 mmol) in anh THF (4.0 mL) was added, and the reaction mixture was stirred at 60°C for 4 h. The reaction mixture was poured into EtOAc, and the organic layer was washed with water (2x) and a saturated NaCl solution (1x), dried ($MgSO_4$), filtered, and evaporated in *vacuo.* Purification using MPLC chromatography (Biotage®; gradient from 100% EtOAc to 2% MeOH / EtOAc) gave N-[4-chloro-3-(trifluoromethyl)phenyl]-N'-{4-[2-carbamoyl-(4-pyridyloxy)]phenyl} urea as a white solid (770 mg, 82%): TLC (EtOAc) $R_f$ 0.11, 100% ethyl acetate $^1$H-NMR (DMSO-$d_6$) δ 9.21 (s, 1H), 8.99 (s, 1H), 8.50 (d, J = 5.6 Hz, 1H), 8.11 (s, 1H), 8.10 (s, 1H), 7.69 (broad s, 1H), 7.64 (dd, J = 8.2 Hz, 2.1 Hz, 1H), 7.61 (s, 1H), 7.59 (d, J = 8.8 Hz, 2H), 7.39 (d, J = 2.5 Hz, 1H), 7.15 (d, J = 8.9 Hz, 2H), 7.14 (m, 1H); MS LC-MS ($NIH^+$ = 451). Anal. calcd for $C_{20}H_{14}ClF_3N_4O_3$: C 53.29% H 3.13% N 12.43%. Found: C 53.33% H 3.21% N 12.60%.

**Example B**

N-[4-chloro-3-(trifluoromethyl)phenyl]-N'-{4-[2-N-methylcarbamoyl-4-pyridyloxy]phenyl} urea

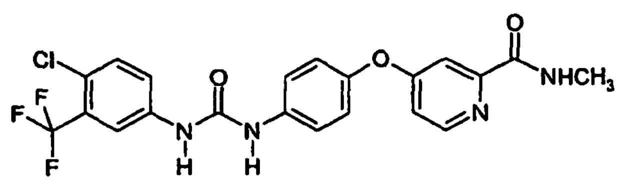

Step 1: 4-Chloro-*N*-methyl-2-pyridinecarboxamide is first synthesized from 4-chloropyridine-2-carbonyl chloride by adding 4-chloropyridine-2-carbonyl chloride HCl salt (7.0 g, 32.95 mmol) in portions to a mixture of a 2.0 M methylamine solution in THF (100 mL) and MeOH (20 mL) at 0 °C. The resulting mixture is stored at 3 °C for 4 h, then concentrated under reduced pressure. The resulting nearly dry solids are suspended in EtOAc (100 mL) and filtered. The filtrate is washed with a saturated NaCl solution (2 x 100 mL), dried ($Na_2SO_4$) and concentrated under reduced pressure to provide 4-chloro-*N*-methyl-2-pyridinecarboxamide as a yellow, crystalline solid.

Step 2: A solution of 4-aminophenol (9.60 g, 88.0 mmol) in anh. DMF (150 mL) is treated with potassium *tert*-butoxide (10.29 g, 91.7 mmol), and the reddish-brown mixture is stirred at room temp. for 2 h. The contents are treated with 4-chloro-*N*-methyl-2-pyridinecarboxamide (15.0 g, 87.9 mmol) from Step 1 and $K_2CO_3$ (6.50 g, 47.0 mmol) and then heated at 80 °C for 8 h. The mixture is cooled to room temp. and separated between EtOAc (500 mL) and a saturated NaCl solution (500 mL). The aqueous phase is back-extracted with EtOAc (300 mL). The combined organic layers are washed with a saturated NaCl solution (4 x 1000 mL), dried ($Na_2SO_4$) and concentrated under reduced pressure. The resulting solids are dried under reduced pressure at 35 °C for 3 h to afford 4-(2-(*N*-methylcarbamoyl)-4-pyridyloxy)aniline as a light-brown solid. $^1$H-NMR (DMSO-$d_6$) δ 2.77 (d, *J*=4.8 Hz, 3H), 5.17 (br s, 2H), 6.64, 6.86 (AA'BB' quartet, *J*=8.4 Hz, 4H), 7.06 (dd, *J*=*5.5,* 2.5 Hz, 1H), 7.33 (d, *J*=2.5 Hz, 1H), 8.44 (d, *J*=5.5 Hz, 1H), 8.73 (br d, 1H); HPLC ES-MS *m*/*z* 244 ($(M+H)^+$).

Step 3: A solution of 4-chloro-3-(trifluoromethyl)phenyl isocyanate (14.60 g, 65.90 mmol) in $CH_2Cl_2$ (35 mL) is added dropwise to a suspension of 4-(2-(*N*-methylcarbamoyl)-4-pyridyloxy)aniline from Step 2; (16.0 g, 65.77 mmol) in $CH_2Cl_2$ (35 mL) at 0 °C. The resulting mixture is stirred at room temp. for 22 h. The resulting yellow solids are removed by filtration, then washed with $CH_2Cl_2$ (2 x 30 mL) and dried under reduced pressure (approximately 1 mmHg) to afford *N*-(4-chloro-3-(trifluoromethyl)phenyl)-*N*'-(4-(2-(*N*-methylcarbamoyl)-4-pyridyloxy)phenyl) urea as an off-white solid: mp

207-209 °C; $^1H$-NMR (DMSO-$d_6$) δ 2.77 (d, *J*=4.8 Hz, 3H), 7.16 (m, 3H), 7.37 (d, *J*=2.5 Hz, 1H), 7.62 (m, 4H), 8.11 (d, *J*=2.5 Hz, 1H), 8.49 (d, *J*=5.5 Hz, 1H), 8.77 (br d, 1H), 8.99 (s, 1H), 9.21 (s, 1H); HPLC ES-MS *m/z* 465 ($(M+H)^+$).

**Example C**

N-[2-methoxy-5-(trifluoromethyl)phenyl]-N'-{4-[2-N-methylcarbamoyl-4-pyridyloxy]phenyl} urea

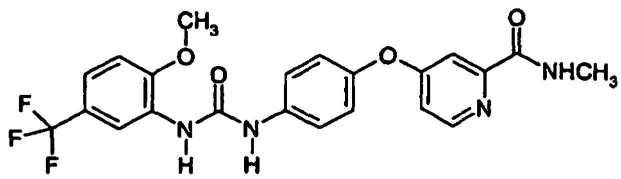

Step 1: 4-Chloro-*N*-methyl-2-pyridinecarboxamide is first synthesized from 4-chloropyridine-2-carbonyl chloride by adding 4-chloropyridine-2-carbonyl chloride HCl salt (7.0 g, 32.95 mmol) in portions to a mixture of a 2.0 M methylamine solution in THF (100 mL) and MeOH (20 mL) at 0 °C. The resulting mixture is stored at 3 °C for 4 h, then concentrated under reduced pressure. The resulting nearly dry solids are suspended in EtOAc (100 mL) and filtered. The filtrate is washed with a saturated NaCl solution (2 x 100 mL), dried ($Na_2SO_4$) and concentrated under reduced pressure to provide 4-chloro-*N*-methyl-2-pyridinecarboxamide as a yellow, crystalline solid.

Step 2: A solution of 4-aminophenol (9.60 g, 88.0 mmol) in anh. DMF (150 mL) is treated with potassium *tert*-butoxide (10.29 g, 91.7 mmol), and the reddish-brown mixture is stirred at room temp. for 2 h. The contents are treated with 4-chloro-*N*-methyl-2-pyridinecarboxamide (15.0 g, 87.9 mmol) from Step 1 and $K_2CO_3$ (6.50 g, 47.0 mmol) and then heated at 80 °C for 8 h. The mixture is cooled to room temp. and separated between EtOAc (500 mL) and a saturated NaCl solution (500 mL). The aqueous phase is back-extracted with EtOAc (300 mL). The combined organic layers are washed with a saturated NaCl solution (4 x 1000 mL), dried ($Na_2SO_4$) and concentrated under reduced pressure. The resulting solids are dried under reduced pressure at 35 °C for 3 h to afford 4-(2-(*N*-methylcarbamoyl)-4-pyridyloxy)aniline as a light-brown solid. $^1H$-NMR (DMSO-$d_6$) δ 2.77 (d, *J*=4.8 Hz, 3H), 5.17 (br s, 2H), 6.64, 6.86 (AA'BB' quartet, *J*=8.4 Hz, 4H), 7.06 (dd, *J*=5.5, 2.5 Hz, 1H), 7.33 (d, *J*=2.5 Hz, 1H), 8.44 (d, *J*=5.5 Hz, 1H), 8.73 (br d, 1H); HPLC ES-MS *m/z* 244 ($(M+H)^+$).

Step 3: To a solution of 2-methoxy-5-(trifluoromethyl)aniline (0.15 g) in anh $CH_2Cl_2$ (15 mL) at 0 °C is added CDI (0.13 g). The resulting solution is allowed to warm to room temp. over 1 h, is stirred at room temp. for 16 h, then is treated with 4-(2-(*N*-methylcarbamoyl)-4-pyridyloxy)aniline (0.18 g) from Step 2. The resulting yellow solution is stirred at room temp. for 72 h, then is treated with $H_2O$ (125 mL). The resulting aqueous mixture is extracted with EtOAc (2 x 150 mL). The combined organics are washed with a saturated NaCl solution (100 mL), dried ($MgSO_4$) and concentrated under reduced pressure. The residue is triturated (90% EtOAc/10% hexane). The resulting white solids are collected by filtration and washed with EtOAc. The filtrate is concentrated under reduced pressure and the residual oil purified by column chromatography (gradient from 33% EtOAc/67% hexane to 50% EtOAc/50% hexane to 100% EtOAc) to give *N*-(2-methoxy-5-(trifluoromethyl)phenyl)-*N'*-(4-(2-(*N*-methylcarbamoyl)-4-pyridyloxy)phenyl) urea as a light tan solid: TLC (100% EtOAc) $R_f$ 0.62; $^1H$ NMR (DMSO-$d_6$) δ 2.76 (d, *J*=4.8 Hz, 3H), 3.96 (s, 3H), 7.1-7.6 and 8.4-8.6 (m, 11H), 8.75 (d, *J*=4.8 Hz, 1H), 9.55 (s, 1 H); FAB-MS *m/z* 461 ($(M+H)^+$).

Listed below are compounds listed in the Tables below which have been synthesized according to the Detailed Experimental Procedures given above:

**Syntheses of Exemplified Compounds**

The synthesis of the exemplified compounds is more particularly described in U.S. Patent Application No. 20020042517, published April 11, 2002.

**Tables**

The compounds listed in Tables 1-6 below were synthesized according to the general methods shown above, and the more detailed exemplary procedures described in U.S. Patent Application No. 20020042517, published April 11, 2002.

**Table 1. 3-*tert*-Butylphenyl Ureas**

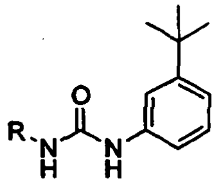

| Entry | mp R (˚C) | HPLC (min.) | TLC $R_f$ | TLC Solvent System | Mass Spec. [Source] | |
|---|---|---|---|---|---|---|
| 1 | 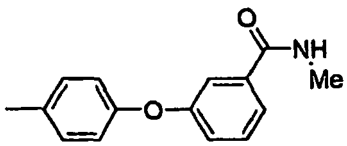 | | 0.22 | 50% EtOAc /50% hexane | 418 (M+H)+ (HPLC ES-MS) | |
| 2 | 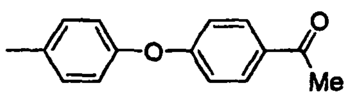 | | 0.58 | 50% EtOAc / 50% hexane | 403 (M+H)+ (HPLC ES-MS) | |
| 3 | 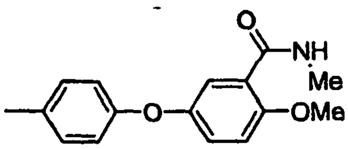 | 133-135 | 0.68 | 100% EtOAc | 448 (M+H)+ (FAB) | |

**2. 5-*tert*-Butyl-2-methoxyphenyl Ureas**

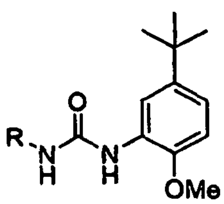

| Entry | R | mp (˚C) | HPL C (min. ) | TL C $R_f$ | TLC Solvent System | Mass Spec. [Source] | |
|---|---|---|---|---|---|---|---|
| 4 | 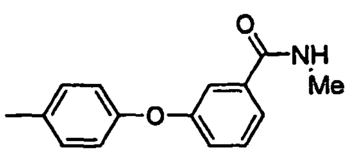 | | 5.93 | | | 448 (M+H)+ (HPLC ES-MS) | |
| 5 | 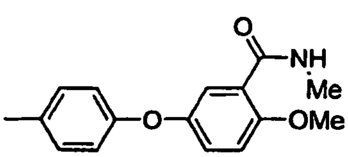 | 120-122 | | 0.67 | 100% EtOAc | 478 (M+H)+ (FAB) | |

(continued)

| Entry | R | mp (˚C) | HPL C (min. ) | TL C $R_f$ | TLC Solvent System | Mass Spec. [Source] | |
|---|---|---|---|---|---|---|---|
| 6 | 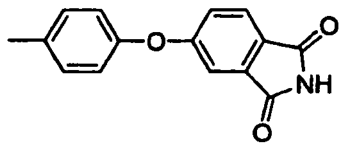 | | | 0.40 | 50% EtOAc /50% hexane | 460 (M+H)+ (HPLC ES-MS) | |
| 7 | 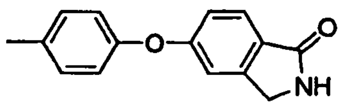 | | | 0.79 | 50% EtOAc /50% hexane | 446 (M+H)+ (HPLC ES-MS) | |

Table 3. 5-(Trifluoromethyl)-2-methoxyphenyl Ureas

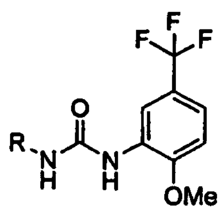

| Entry | R | mp (˚C) | HPL C (min. ) | TLC $R_f$ | TLC Solvent System | Mass Spec. [Source ] | |
|---|---|---|---|---|---|---|---|
| 8 | 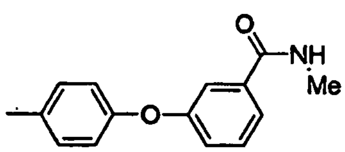 | 250 (dec) | | | | 460 (M+H) + (FAB) | |
| 9 | 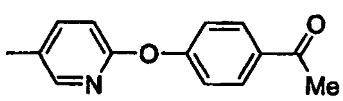 | 206- 208 | | 0.54 | 10% MeOH/ 90% CH2C12 | 446 (M+H) + (HPLC ES-MS) | |
| 10 | 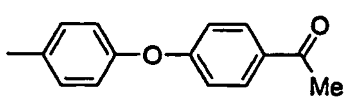 | | | 0.33 | 50% EtOAc/ 50% pet ether | 445 (M+H) + (HPLC ES-MS) | |
| 11 | 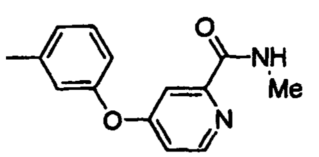 | | | 0.20 | 2% Et3N/ 98% EtOAc | 461 (M+H) + (HPLC ES-MS) | |
| 12 | 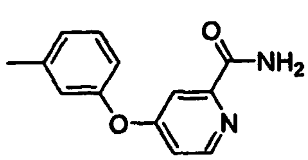 | | | 0.27 | 1% Et3N/ 99% EtOAc | 447 (M+H) + (HPLC ES-MS) | |

(continued)

| Entry | R | mp (˚C) | HPL C (min. ) | TLC $R_f$ | TLC Solvent System | Mass Spec. [Source ] | |
|---|---|---|---|---|---|---|---|
| 13 | 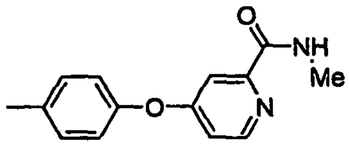 | | | 0.62 | 100% EtOAc | 461 (M+H) + (FAB) | |
| 14 | 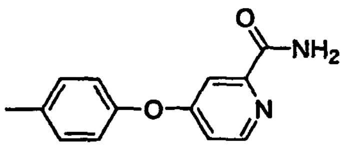 | 114- 117 | | 0.40 | 1% Et3N/ 99% EtOAc | 447 (M+H) + (FAB) | |
| 15 | 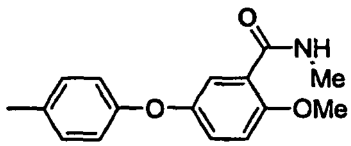 | 232- 235 | | 0.54 | 100% EtOAc | 490 (M+H) + (FAB) | |
| 16 | 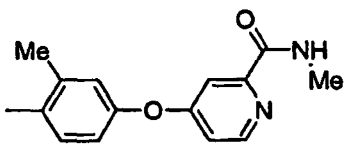 | 210- 213 | | 0.29 | 5% MeOH/ 45% EtOAc/ 50% pet ether | 475 (M+H) + (HPLC ES-MS) | |
| 17 | 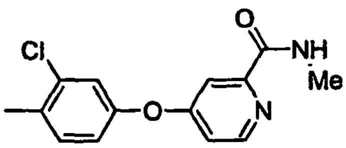 | 187- 188 | | 0.17 | 50% EtOAc/ 50% pet ether | 495 (M+H) + (HPLC ES-MS) | |
| 18 | 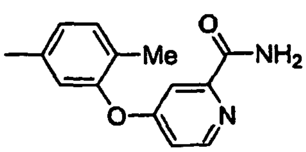 | | | 0.48 | 100% EtOAc | 475 (M+H) + (HPLC ES-MS) | |
| 19 | 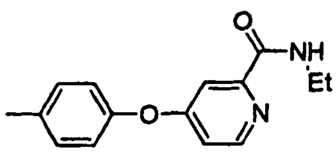 | 194- 196 | | 0.31 | 5% MeOH/ 45% EtOAc/ 50% pet ether | 475 (M+H) + (HPLC ES-MS) | |
| 20 | 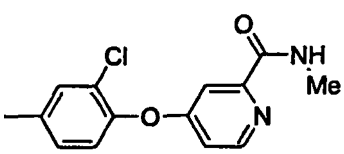 | 214- 216 | | 0.25 | 5% MeOH/ 45% EtOAc/ 50% pet ether | 495 (M+H) + (HPLC ES-MS) | |

(continued)

| Entry | R | mp (˚C) | HPL C (min. ) | TLC $R_f$ | TLC Solvent System | Mass Spec. [Source ] | |
|---|---|---|---|---|---|---|---|
| 21 | 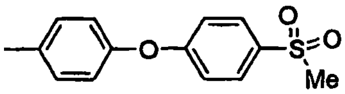 | 208- 210 | | 0.30 | 50% EtOAc/ 50% hexane | 481 (M+H) + (HPLC ES-MS) | |
| 22 | 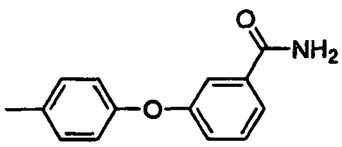 | 188- 190 | | 0.30 | 70% EtOAc/ 50% hexane | 447 (M+H) + (HPLC ES-MS) | |
| 23 | 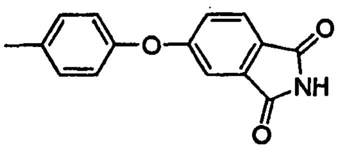 | | | 0.50 | 70% EtOAc/ 30% hexane | 472 (M+H) + (FAB) | |
| 24 | 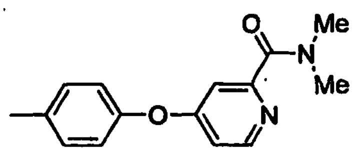 | 203- 205 | | 0.13 | 100% EtOAc | 479 (M+H) + (HPLC ES-MS) | |
| 25 | 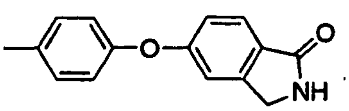 | | | 0.09 | 75% EtOAc/ 25% hexane | 458 (M+H) + (HPLC ES-MS) | |
| 26 | 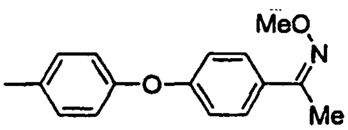 | 169- 171 | | 0.67 | 50% EtOAc/ 50% pet ether | 474 (M+H) + (HPLC ES-MS) | |
| 27 | 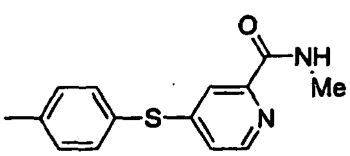 | 218- 219 | | 0.40 | 50% EtOAc/ 50% pet ether | 477 (M+H) + (HPLC ES-MS) | |
| 28 | 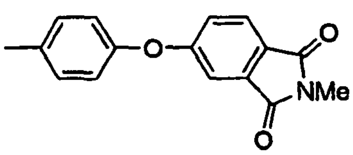 | 212- 214 | | 0.30 | 40% EtOAc/ 60% hexane | | |

(continued)

| Entry | R | mp (˚C) | HPL C (min. ) | TLC $R_f$ | TLC Solvent System | Mass Spec. [Source ] | |
|---|---|---|---|---|---|---|---|
| 29 | 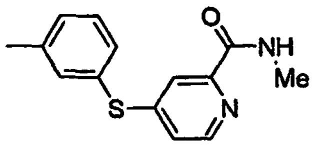 | | | 0.33 | 50% EtOAc/ 50% pet ether | 474 (M+H) + (HPLC ES-MS) | |
| 30 | 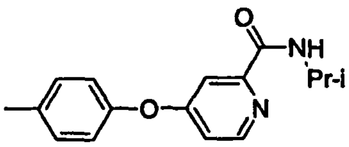 | 210-211 | | | | | |
| 31 | 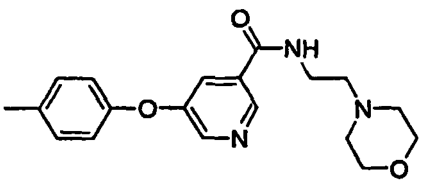 | 210- 204 | | 0.43 | 10% MeOH/ CH2Cl2 | | |
| 32 | 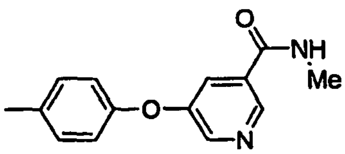 | 247- 249 | | 0.57 | 10% MeOH/ CH2Cl2 | | |
| 33 | 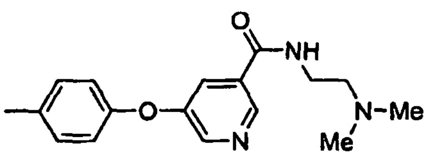 | 217- 219 | | 0.07 | 10% MeOH/ CH2Cl2 | | |
| 34 | 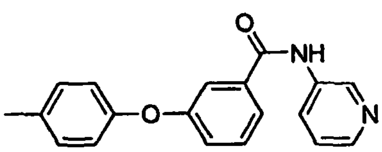 | | | 0.11 | 70% EtOAc/ 30% hexane | | |
| 35 | 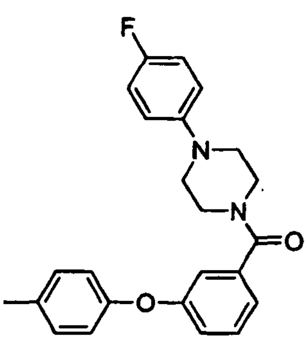 | | | 0.38 | 70% EtOAc/ 30% hexane | | |

(continued)

| Entry | R | mp (˚C) | HPL C (min. ) | TLC $R_f$ | TLC Solvent System | Mass Spec. [Source ] | |
|---|---|---|---|---|---|---|---|
| 36 | 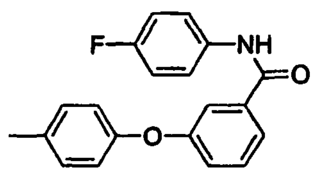 | | | 0.77 | 70% EtOAc/ 30% hexane | | |
| 37 | 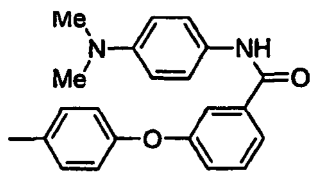 | | | 0.58 | 70% EtOAc/ 30% hexane | | |
| 38 | 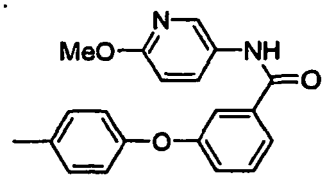 | | | 0.58 | 70% EtOAc/ 30% hexane | | |
| 39 | 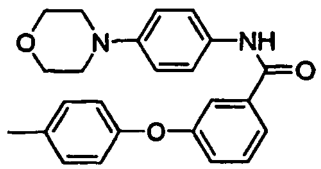 | | | 0.17 | 70% EtOAc/ 30% hexane | | |
| 40 | 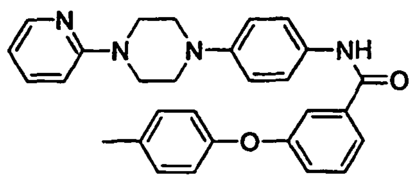 | | | 0.21 | 70% EtOAc/ 30% hexane | | |

**Table 4. 3-(Trifluoromethyl)-4-chlorophenyl Ureas**

| |
|---|
| 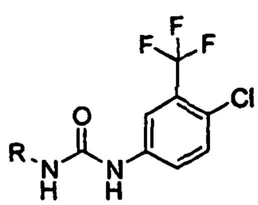 |

(continued)

| Entry | R | mp (˚C) | HPL C (min. ) | TLC $R_f$ | TLC Solvent System | Mass Spec. [Source] | |
|---|---|---|---|---|---|---|---|
| 41 | 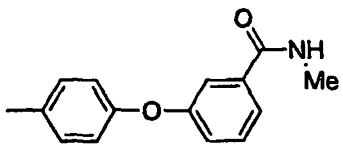 | 163- 165 | | 0.08 | 50% EtOAc/ 50% pet ether | 464 (M+H)+ (HPLC ES-MS) | |
| 42 | 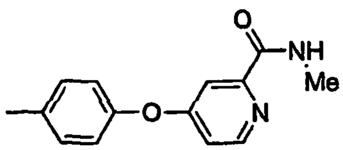 | 215 | | 0.06 | 50% EtOAc/ 50% pet ether | 465 (M+H)+ (HPLC ES-MS) | |
| 43 | 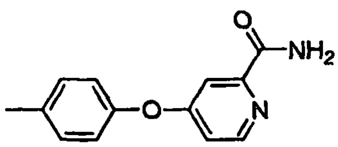 | | | 0.10 | 50% EtOAc/ 50% pet ether | 451 (M+H)+ (HPLC ES-MS) | |
| 44 | 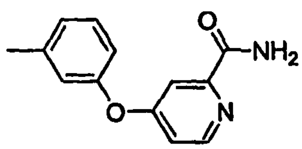 | | | 0.25 | 30% EtOAc/ 70% pet ether | 451 (M+H)+ (HPLC ES-MS) | |
| 45 | 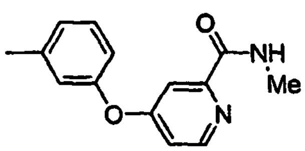 | | | 0.31 | 30% EtOAc/ 70% pet ether | 465 (M+H)+ (HPLC ES-MS) | |
| 46 | 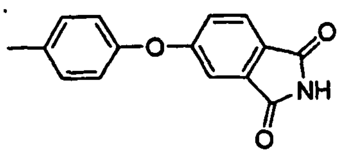 | 176- 179 | | 0.23 | 40% EtOAc/ 60% hexane | 476 (M+H)+ (FAB) | |
| 47 | 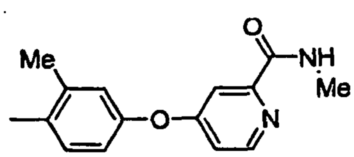 | | | 0.29 | 5% MeOH/ 45% EtOAc/ 50% pet ether | 478 (M+H)+ (HPLC ES-MS) | |
| 48 | 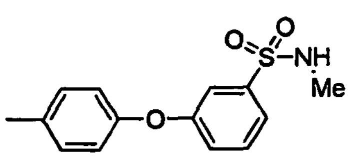 | 206-209 | | | | | |

(continued)

| Entry | R | mp (˚C) | HPL C (min. ) | TLC $R_f$ | TLC Solvent System | Mass Spec. [Source] | |
|---|---|---|---|---|---|---|---|
| 49 | 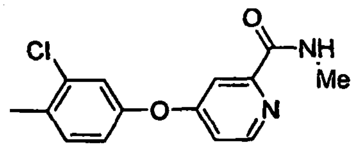 | 147- 151 | | 0.22 | 50% EtOAc/ 50% pet ether | 499 (M+H)+ (HPLC ES-MS) | |
| 50 | 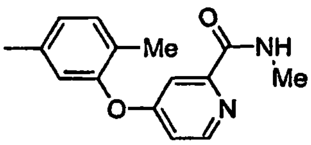 | | | 0.54 | 100% EtOAc | 479 (M+H)+ (HPLC ES-MS) | |
| 51 | 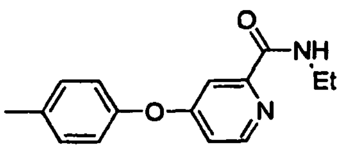 | 187- 189 | | 0.33 | 5% MeOH/ 45% EtOAc/ 50% pet ether | 479 (M+H)+ (HPLC ES-MS) | |
| 52 | 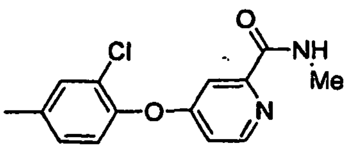 | 219 | | 0.18 | 5% MeOH/ 45% EtOAc/ 50% pet ether | 499 (M+H)+ (HPLC ES-MS) | |
| 53 | 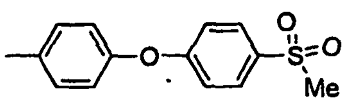 | 246- 248 | | 0.30 | 50% EtOAc/ 50% hexane | 485 (M+H)+ (HPLC ES-MS) | |
| 54 | 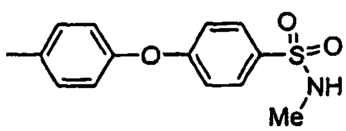 | 196- 200 | | 0.30 | 70% EtOAc/ 30% hexane) | 502 (M+H)+ (HPLC ES-MS) | |
| 55 | 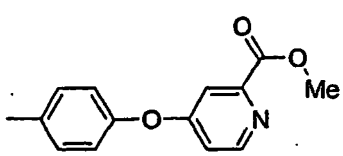 | 228- 230 | | 0.30 | 30% EtOAc/ 70% CH2Cl2 | 466 (M+H)+ (HPLC ES-MS) | |
| 56 | 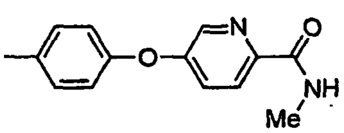 | 238-245 | | | | | |

(continued)

| Entry | R | mp (˚C) | HPL C (min. ) | TLC $R_f$ | TLC Solvent System | Mass Spec. [Source] | |
|---|---|---|---|---|---|---|---|
| 57 | 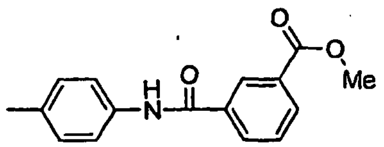 | 221- 222 | | 0.75 | 80% EtOAc/ 20% hexane | 492 (M+H)+ (FAB) | |
| 58 | 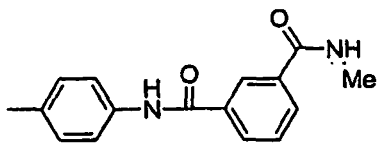 | 247 | | 0.35 | 100% EtOAc | | |
| 59 | 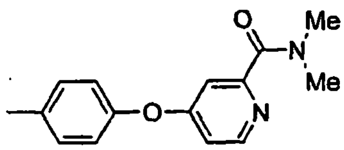 | 198- 200 | | 0.09 | 100% EtOAc | 479 (M+H)+ (HPLC ES-MS) | |
| 60 | 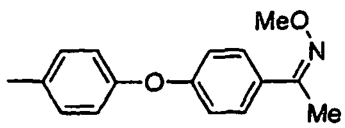 | 158- 160 | | 0.64 | 50% EtOAc/ 50% pet ether | | |
| 61 | 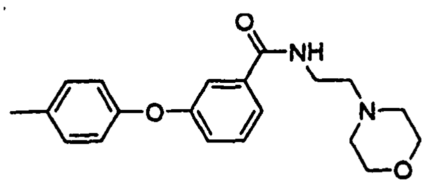 | 195- 197 | | 0.39 | 10% MeOH/ CH2C1 2 | | |
| 62 | 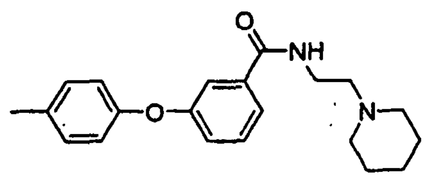 | 170- 172 | | 0.52 | 10% MeOH/ CH2C1 2 | | |
| 63 | 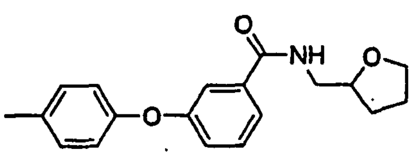 | 168- 171 | | 0.39 | 10% MeOH/ CH2C1 2 | | |
| 64 | 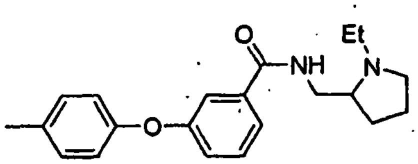 | 176- 177 | | 0.35 | 10% MeOH/ CH2C1 2 | | |

(continued)

| Entry | R | mp (˚C) | HPL C (min. ) | TLC $R_f$ | TLC Solvent System | Mass Spec. [Source] | |
|---|---|---|---|---|---|---|---|
| 65 | 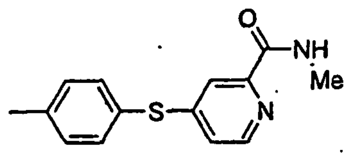 | 130- 133 | | | | 487 (M+H)+ (HPLC ES-MS) | |
| 66 | 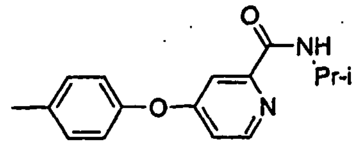 | 155 | | | | | |
| 67 | 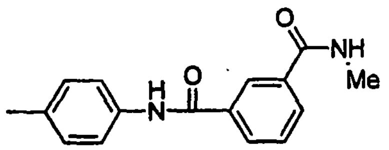 | 225- 229 | | 0.23 | 100% EtOAc | | |
| 68 | 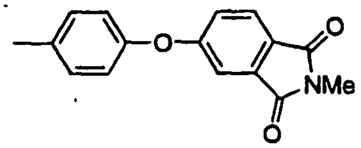 | 234- 236 | | 0.29 | 40% EtOAc/ 60% hexane | | |
| 69 | 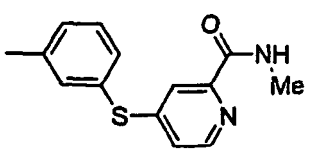 | | | 0.48 | 50% EtOAc/ 50% pet ether | 481 (M+H)+ (HPLC ES-MS) | |
| 70 | 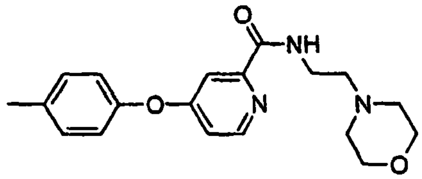 | | | 0.46 | 5% MeOH/ 95% CH2Cl2 | 564 (M+H)+ (HPLC ES-MS) | |
| 71 | 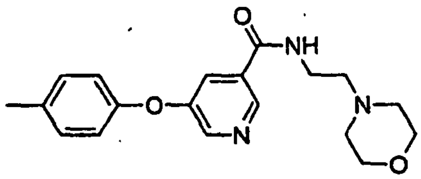 | 199- 201 | | 0.50 | 10% MeOH/ CH2Cl 2 | | |
| 72 | 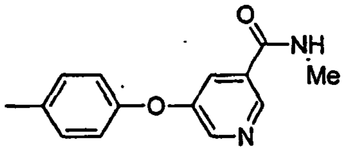 | 235- 237 | | 0.55 | 10% MeOH/ CH2C1 2 | | |

(continued)

| Entry | R | mp (˚C) | HPL C (min. ) | TLC $R_f$ | TLC Solvent System | Mass Spec. [Source] | |
|---|---|---|---|---|---|---|---|
| 73 | 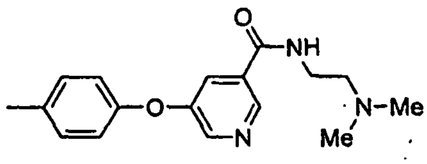 | 200- 201 | | 0.21 | 50% MeOH/ CH2C1 2 | | |
| 74 | 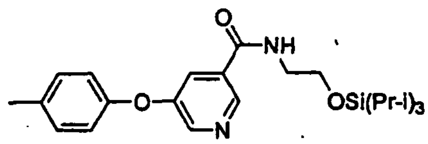 | 145-148 | | | | | |
| 75 | 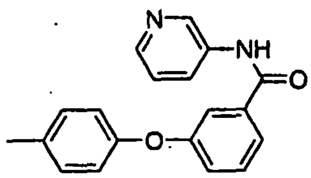 | | | 0.12 | 70% EtOAc/ 30% hexane | 527 (M+H)+ (HPLC ES-MS) | |
| 76 | 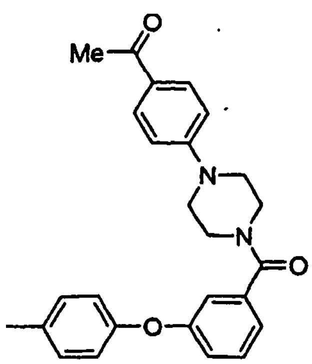 | | | 0.18 | 70% EtOAc/ 30% hexane | | |
| 77 | 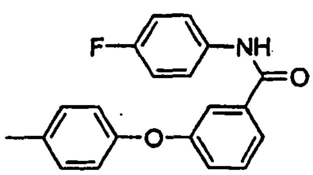 | | | 0.74 | 70% EtOAc/ 30% hexane | | |
| 78 | 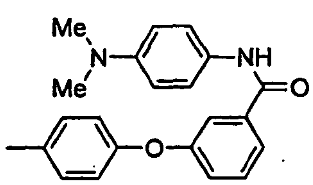 | | | 0.58 | 70% EtOAc/ 30% hexane | | |

(continued)

| Entry | R | mp (˚C) | HPL C (min. ) | TLC $R_f$ | TLC Solvent System | Mass Spec. [Source] | |
|---|---|---|---|---|---|---|---|
| 79 | 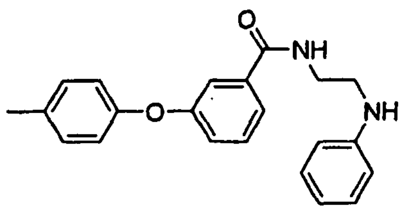 | | | 0.47 | 70% EtOAc/ 30% hexane | 569 (M+H)+ (HPLC ES-MS) | |
| 80 | 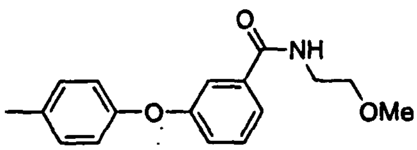 | | | 0.18 | 70% EtOAc/ 30% hexane | 508 (M+H)+ (HPLC ES-MS) | |
| 81 | 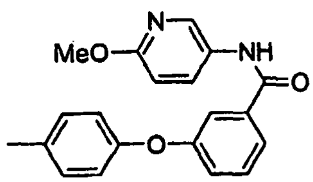 | | | 0.58 | 70% EtOAc/ 30% hexane | 557 (M+H)+ (HPLC ES-MS) | |
| 82 | 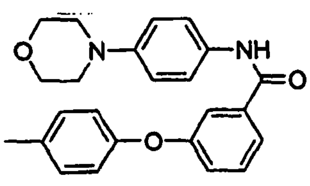 | | | 0.37 | 70% EtOAc/ 30% hexane | 611 (M+H)+ (HPLC ES-MS) | |
| 83 | 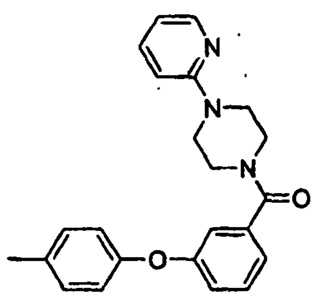 | | | 0.19 | 70% EtOAc/ 30% hexane | | |
| 84 | 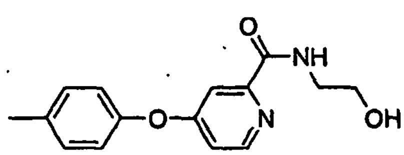 | 179-183 | | | | | |

Table 5. 3-(Trifluoromethyl)-4-bromophenyl Ureas

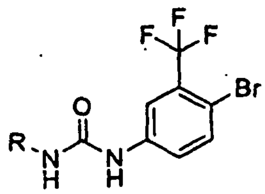

| Entry | R | mp (˚C) | HPL C (min. ) | TLC $R_f$ | TLC Solvent System | Mass Spec. [Source] | |
|---|---|---|---|---|---|---|---|
| 85 | 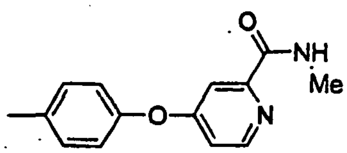 | 186-187 | | 0.13 | 50% EtOAc/ 50% pet ether | 509 (M+H)+ (HPLC ES-MS) | |
| 86. | 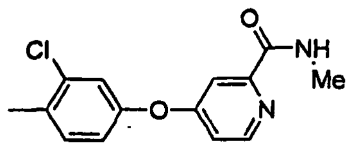 | 150-152 | | 0.31 | 50% EtOAc/ 50% pet ether | 545 (M+H)+ (HPLC ES-MS) | |
| 87 | 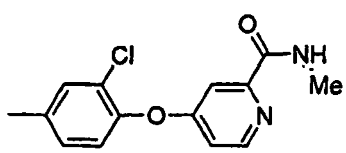 | 217-219 | | 0.16 | 50% EtOAc/ 50% pet ether | 545 (M+H)+ (HPLC ES-MS) | |
| 88 | 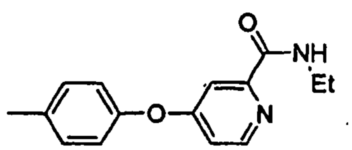 | 183-184 | | 0.31 | 50% EtOAc/ 50% pet ether | 525 (M+H)+ (HPLC ES-MS) | |
| 89 | 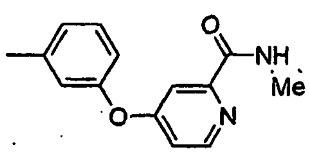 | | | 0.21 | 50% EtOAc/ 50% pet ether | 511 (M+H)+ (HPLC ES-MS) | |
| 90 | 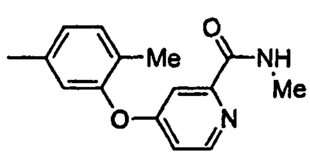 | | | 0.28 | 50% EtOAc/ 50% pet ether | 525 (M+H)+ (HPLC ES-MS), | |
| 91 | 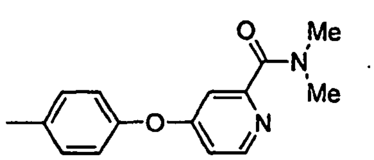 | 214-216 | | 0.28 | 50% EtOAc/ 50% pet ether | 522 (M+H)+ (HPLC ES-MS) | |

(continued)

| Entry | R | mp (˚C) | HPL C (min. ) | TLC $R_f$ | TLC Solvent System | Mass Spec. [Source] | |
|---|---|---|---|---|---|---|---|
| 92 | 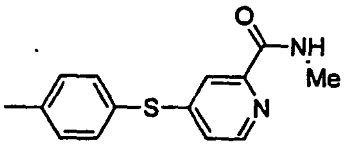 | | | 0.47 | 50% EtOAc/ 50% pet ether | 527 (M+H)+ (HPLC ES-MS) | |
| 93 | 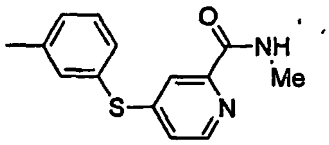 | | | 0.46 | 50% EtOAc/ 50% pet ether | 527 (M+H)+ (HPLC ES-MS) | |
| 94 | 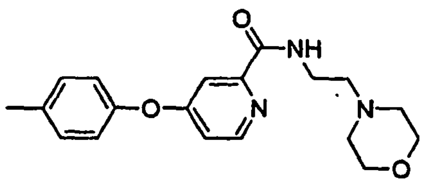 | 145-150 | | 0.41 | 5% MeOH/ 95% CH2Cl2 | | |

**Table 6. 5-(Trifluoromethyl)-4-chloro-2-methoxyphenyl Ureas**

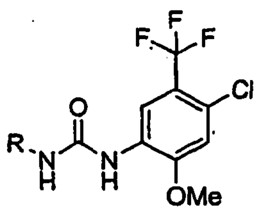

| Entry | R | mp (˚C) | HPL C (min. ) | TLC $R_f$ | TLC Solvent System | Mass Spec. [Source] | |
|---|---|---|---|---|---|---|---|
| 95 | 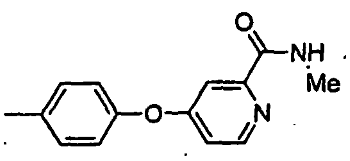 | 140- 144 | | 0.29 | 5% MeOH/ 45% EtOAc/ 50% pet ether | 495 (M+H)+ (HPLC ES-MS) | |
| 96 | 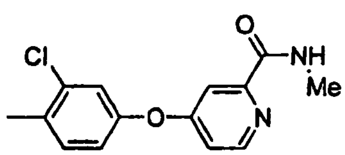 | 244- 245 | | 0.39 | 5% MeOH/ 45% EtOAc/ 50% pet ether | 529 (M+H)+ (HPLC ES-MS) | |

(continued)

| Entry | R | mp (˚C) | HPL C (min. ) | TLC $R_f$ | TLC Solvent System | Mass Spec. [Source] | |
|---|---|---|---|---|---|---|---|
| 97 | 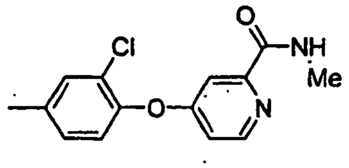 | 220- 221 | | 0.25 | 5% MeOH/ 45% EtOAc/ 50% pet ether | 529 (M+H)+ (HPLC ES-MS) | |
| 98 | 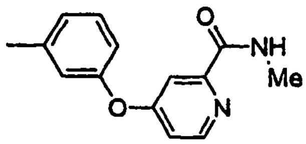 | | | 0.27 | 5% MeOH/ 45% EtOAc/ 50% pet ether | 495 (M+H)+ (HPLC ES-MS) | |
| 99 | 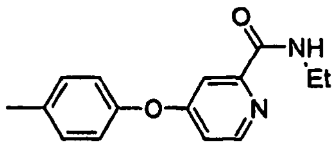 | 180- 181 | | 0.52 | 5% MeOH/ 45% EtOAc/ 50% pet ether | 509 (M+H)+ (HPLC ES-MS) | |
| 100 | 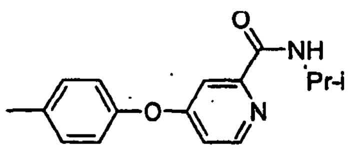 | 162-165 | | | | | |

**Table 7. Additional Ureas**

| Entry | R | mp (˚C) | HPL C (min. ) | TLC $R_f$ | TLC Solvent System | Mass Spec. [Source] | |
|---|---|---|---|---|---|---|---|
| 101 | 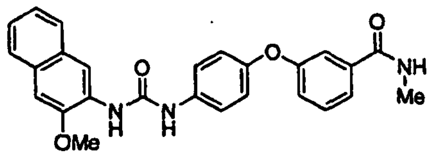 | 162-165 | | | | | |
| 102 | 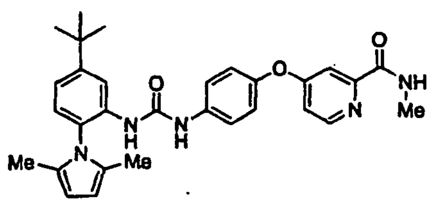 | | | 0.10 | 50% EtOAc/ 50% hexane | 442 (M+H)+ (HPLC ES-MS) | |

(continued)

| Entry | R | mp (˚C) | HPL C (min. ) | TLC $R_f$ | TLC Solvent System | Mass Spec. [Source] | |
|---|---|---|---|---|---|---|---|
| 103 | 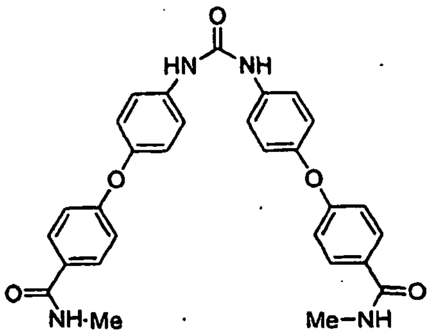 | 125- 130 | | 0.24 | 40% EtOAc/ 60% hexane | 512 (M+H)+ (FAB) | |

Selected compounds are named below

From WO 2000/41698

| Entry No | Name |
|---|---|
| 1 | {3-[4-({[3-(tert- |
| | butyl)phenyl]amino}carbonylamino)phenoxy]phenyl}-N-methylcarboxamide |
| 11 | N-[2-methoxy-5-(trifluoromethyl)phenyl]({3-[2-(N-methylcarbamoyl)(4-pyridyloxy)]phenyl}amino) caxboxamide |
| 12 | 4-[3-({N-[2-methoxy-5-(trifluoromethyl)phenyl]carbamoyl}amino)phenoxy]pyridine-2-carboxamide |
| 13 | N-[2-methoxy-5-(trifluoromethyl)phenyl]({4-[2-(N-methylcarbamoyl)(4-pyridyloxy)]phenyl}amino) carboxamide |
| 14 | 4-[4-({N-[2-methoxy-5-(trifluoromethyl)phenyl]carbamoyl}amino)phenoxy]pyridine-2-carboxamide |
| 16 | {4-[4-({N-[2-methoxy-5-(trifluoromethyl)phenyl]carbamoyl}amino)-3-methylphenoxy](2-pyridyl)}-N-methylcarboxamide |
| 17 | ({2-chloro-4-[2-(N-methylcarbamoyl)(4-pyridyloxy)]phenyl}amino)-N-[2-methoxy-5-(trifluoromethyl) phenyl]carboxamide |
| 19 | ({4-[2-(N-ethylcarbamoyl)(4-pyridyloxy)]phenyl}amino)-N-[2-methoxy-5-(trifluoromethyl)phenyl] carboxamide |
| 20 | ({3-chloro-4-[2-(N-methylcarbamoyl)(4-pyridyloxy)]phenyl}amino)-N-[2-methoxy-5-(trifluoromethyl) phenyl]carboxamide |
| 22 | 3-[4-({N-[2-methoxy-5-(trifluoromethyl)phenyl]carbamoyl}amino)phenoxy]benzamide |
| 24 | ({4-[2-(N,N-dimethylcarbamoyl)(4-pyridyloxy)]phenyl}amino)-N-[2-methoxy-5-(trifluoromethyl)phenyl] carboxamide |
| 27 | N-[2-methoxy-5-(trifluoromethyl)phenyl]({4-[2-(N-methylcarbamoyl)(4-pyridylthio)]phenyl}amino) carboxamide |
| 29 | N-[2-methoxy-5-(trifluoromethyl)phenyl]({3-[2-(N-methylcarbamoyl)(4-pyridylthio)]phenyl}amino) carboxamide |
| 31 | N-[2-methoxy-5-(trifluoromethyl)phenyl][(4-{5-[N-(2-morpholin-4-ylethyl)carbamoyl](3-pyridyloxy)} phenyl)amino]carboxamide |
| 32 | N-[2-methoxy-5-(trifluoromethyl)phenyl]({4-[5-(N-methylcarbamoyl)(3-pyridyloxy)]phenyl} amino) carboxamide |

(continued)

| Entry No | Name |
|---|---|
| 34 | N-[2-methoxy-5-(trifluoromethyl)phenyl]({4-[3-(N-(3-pyridyl)carbamoyl)phenoxy]phenyl} amino) carboxamide |
| 42 | {4-[4-({[4-chloro-3-(trifluoromethyl)phenyl]amino}carbonylamino)phenoxy](2-pyridyl)}-N-methylcarboxamide |
| 43 | 4-[4-({[4-chloro-3-(trifluoromethyl)phenyl]amino} carbonylamino)phenoxy]pyridi ne-2-carboxamide |
| 44 | 4-[3-({[4-chloro-3-(trifluoromethyl)phenyl]amino}carbonylamino)phenoxy]pyridi ne-2-carboxamide |
| 45 | {[4-chloro-3-(trifluoromethyl)phenyl]amino}-N-{3-[2-(N-methylcarbamoyl)(4-pyridyloxy)]phenyl} carboxamide |
| 47 | {[4-chloro-3-(trifluoromethyl)phenyl]amino} -N-{2-methyl-4-[2-(N-methylcarbamoyl)(4-pyridyloxy)] phenyl} carboxamide |
| 49 | {4-[3-chloro-4-({[4-chloro-3-(trifluoromethyl)phenyl]amino}carbonylamino)phenoxy](2-pyridyl) -N-methylcarboxamide |
| 51 | N-[4-chloro-3-(trifluoromethyl)phenyl]({4-[2-(N-ethylcarbamoyl)(4-pyridyloxy)]phenyl}amino) carboxamide |
| 61 | {3-[4-({[4-chloro-3-(trifluoromethyl)phenyl]amino}carbonylamino)phenoxy]phenyl }-N-(2-morpholin-4-ylethyl)carboxamide |
| 62 | {3-[4-({[4-chloro-3-(trifluoromethyl)phenyl]amino}carbonylamino)phenoxy]phenyl }-N-(2-piperidylethyl) carboxamide |
| 65 | {4-[4-({[4-chloro-3-(trifluoromethyl)phenyl]amino}carbonylamino)phenylthio](2-pyridyl)}-N-methylcarboxamide |
| 69 | {[4-chloro-3-(trifluoromethyl)phenyl]amino}-N-{3-[2-(N-methylcarbamoyl)(4-pyridylthio)]phenyl} carboxamide |
| 70 | {4-[4-({[4-chloro-3-(trifluoromethyl)phenyl]amino}carbonylamino)phenoxy](2-pyridyl)}-N-(2-morpholin-4-ylethyl)carboxamide |
| 72 | {5-[4-({[4-chloro-3-(trifluoromethyl)phenyl]amino}carbonylamino)phenoxy](3-pyridyl)}-N-methylcarboxamide |
| 75 | N-[4-chloro-3-(trifluoromethyl)phenyl]({4-[3-(N-(3-pyridyl)carbamoyl)phenoxy]phenyl}amino) carboxamide. |
| 84 | {4-[4-({[4-chloro-3-(trifluoromethyl)pheuyl]amino}carbonylanimo)phenoxy](2-pyridyl)}-N-(2-hydroxyethyl)carboxamide |
| 87 | {4-[4-({[4-bromo-3-(trifluoromethyl)phenyl]amino}carbonylamino)-2-chlorophenoxy](2-pyridyl)}-N-methylcarboxamide |
| 88 | N-[4-bromo-3-(trifluoromethyl)phenyl]({4-[2-(N-ethylcarbamoyl)(4-pyridyloxy)]phenyl}amino) carboxamide |
| 89 | {[4-bromo-3-(trifluoromethyl)phenyl]amino}-N-{3-[2-(N-methylcarbamoyl)(4-pyridyloxy)]phenyl} carboxamide |
| 90 | {[4-bromo-3-(trifluoromethyl)phenyl]amino}-N-{4-methyl-3-[2-(N-methylcarbamoyl)(4-pyridyloxy)] phenyl}carboxamide |
| 93 | {[4-bromo-3-(trifluoromethyl)phenyl]amino}-N-{3-[2-(N-methylcarbamoyl)(4-pyridylthio)]phenyl} carboxamide |
| 94 | {4-[4-({[4-bromo-3-(trifluoromethyl)phenyl]amino}carbonylamino)phenoxy](2-pyridyl)}-N-(2-morpholin-4-ylethyl)carboxamide |
| 95 | N-[4-chloro-2-methoxy-5-(trifluoromethyl)phenyl]({4-[2-(N-methylcarbamoyl)(4-pyridyloxy)]phenyl} amino)carboxamide |

(continued)

| Entry No | Name |
|---|---|
| 96 | N-[4-chloro-2-methoxy-5-(trifluoromethyl)phenyl]({2-chloro-4-[2-(N-methylcarbamoyl)(4-pyridyloxy)] phenyl}amino)carboxamide |
| 97 | N-[4-chloro-2-methoxy-5-(trifluoromethyl)phenyl]({3-chloro-4-[2-(N-methylcarbamoyl)(4-pyridyloxy)] phenyl}amino)carboxamide |
| 98 | N-[4-chloro-2-methoxy-5-(trifluoromethyl)phenyl]({3-[2-(N-methylcarbamoyl)(4-pyridyloxy)]phenyl} amino)carboxamide |
| 99 | N-[4-chloro-2-methoxy-5-(trifluoromethyl)phenyl]({4-[2-(N-ethylcarbamoyl)(4-pyridyloxy)]phenyl} amino)carboxamide |
| | |

The compounds listed below are suitable for use in this invention and their synthesis is described with greater particularity in WO 2002/85859

| Entry No | Name |
|---|---|
| 16 | [(4-fluorophenyl)amino]-N-(3-isoquinolyl)carboxamide |
| 25 | N-(2-methoxy(3-quinolyl))[(4-(4-pyridyloxy)phenyl)amino] carboxamide |
| 27 | N-(2-methoxy(3-quinolyl))[(3-(4-pyridylthio)phenyl)amino]carboxamide |
| 28 | N-[1-(4-methylpiperazinyl)(3-isoquinolyl)][(4-(4-pyridyloxy)phenyl)amino]carboxamide |

and WO 2002/85857

| Entry No | Name |
|---|---|
| 25 | N-(2-methoxy(3-quinolyl))[(4-(4-pyridyloxy)phenyl)amino] carboxamide |
| 27 | N-(2-methoxy(3-quinolyl))[(3-(4-pyridylthio)phenyl)amino]carboxamide |
| 28 | N-[1-(4-methylpiperazinyl)(3-isoquinolyl)][(4-(4-pyridyloxy)phenyl)amino]carboxamide |

The following publications relate to VEGFR-3 inhibition and their description of disease states mediated by VEGFR-3 and assays to determine such activity.

| | |
|---|---|
| WO95/33772 | Alitalo, et. al. |
| WO95/33050 | Charnock-Jones, et. al.. |
| WO96/39421 | Hu, et. al. |
| WO98/33917 | Alitalo, et. al. |
| WO02/057299 | Alitalo, et. al. |
| WO02/060950 | Alitalo, et. al. |
| WO02/081520 | Boesen, et. al. |

The following publications relate to VEGFR-2 inhibition and their description of disease states mediated by VEGFR-2 and assays to determine such activity.

| | |
|---|---|
| EP0882799 | Hanai, et. al. |
| EP1167384 | Ferraram, et, al. |
| EP1086705 | Sato, et. al. |
| EP11300032 | Tesar, et. al. |
| EP1166798 | Haberey, et. al. |
| EP1166799 | Haberey, et. al. |

(continued)

| | |
|---|---|
| EP1170017 | Maini, et. al. |
| EP1203827 | Smith |
| WO02/083850 | Rosen, et. al. |

The following publications relate to FLT-3 inhibition and for their description of disease states mediated by FLT-3 and assays to determine such activity.

| | |
|---|---|
| 2002/0034517 | Brasel, et. al. |
| 2002/0107365 | Lyman, et. al. |
| 2002/0111475 | Graddis, et. al. |
| EP0627487 | Beckermann, et. al. |
| W09846750 | Bauer, et. al. |
| W09818923 | McWherter, et. al. |
| W09428391 | Beckermann, et al. |
| W09426891 | Bimbaum, et al. |

The following patents and publication relate to PDGF/PDGFR inhibition and their description of the disease states mediated by PDGFR-beta and assays to determine such activity.

| | |
|---|---|
| 5,094,941 | Hart, et. al. |
| 5,371,205 | Kelly, et. al. |
| 5,418,135 | Pang |
| 5,444,151 | Vassbotn, et. al. |
| 5,468,468 | LaRochelle, et. al. |
| 5,567,584 | Sledziewski, et. al. |
| 5,618,678 | Kelly, et al. |
| 5,620,687 | Hart, et. al. |
| 5,648,076 | Ross, et. al. |
| 5,668,264 | Janjic, et. al. |
| 5,686,572 | Wolf, et. al. |
| 5,817,310 | Ramakrishnan. et. al. |
| 5,833,986 | LaRochelle, et. al. |
| 5,863,739 | LaRochelle, et. al. |
| 5,872,218 | Wolf, et. al. |
| 5,882,644 | Chang, et. al. |
| 5,891,652 | Wolf, et. al. |
| 5,976,534 | Hart, et. al. |
| 5,990,141 | Hirth, et. al. |
| 6,022,854 | Shuman |
| 6,043,211 | Williams, et. al. |
| 6,110,737 | Escobedo, et. al. |
| 6,207,816B1 | Gold, et. al. |
| 6,228,600B1 | Matsui, et. al. |
| 6,229,002B1 | Janjic, et. al. |
| 6,316,603B1 | McTigue, et. al. |
| 6,372,438B1 | Williams, et. al. |
| 6,403,769B1 | La Rochelle, et. al. |
| 6,440,44581 | Nowak, et. al. |
| 6,475,782B1 | Escobedo, et. al. |
| WO02/083849 | Rosen, et. al. |
| WO02/083704 | Rosen, et. al. |

(continued)

| | |
|---|---|
| WO02/081520 | Boesen, et. al. |
| WO02/079498 | Thomas, et. al. |
| WO02/070008 | Rockwell, et. al. |
| WO09959636 | Sato, et. al. |
| WO09946364 | Cao, et. al. |
| WO09940118 | Hanai, et. al. |
| WO9931238 | Yabana, et. al. |
| WO9929861 | Klagsbrun, et. al. |
| WO9858053 | Kendall, et. al. |
| WO9851344 | Maini, et. al. |
| WO9833917 | Alitalo, et. al. |
| WO9831794 | Matsumoto, et. al. |
| WO9816551 | Ferrara, et. al. |
| WO9813071 | Kendall, et al. |
| WO9811223 | Martiny-Baron, et. al. |
| WO9744453 | Chen, et. al. |
| WO9723510 | Plouet, et. al. |
| WO9715662 | Stinchcomb, et. al. |
| WO9708313 | Ferrara, et. al. |
| WO9639515 | Cao, et. al. |
| WO9623065 | Smith, et. al. |
| WO9606641 | Fleurbaaij, et. al. |
| WO9524473 | Cao, et. al. |
| WO9822316 | Kyowa |
| WO952186 | Rockwell, et. al. |
| WO02/060489 | Xia, et. al. |

**PDGFR-beta**

| | |
|---|---|
| EP0869177 | Matsui, et. al. |
| WO09010013 | Matsui, et. al. |
| WO9737029 | Matsui, et. al. |

**PDGFR-alpha**

| | |
|---|---|
| EP 1000617 | Lammers, et. al. |
| EP0869177 | Matsui, et al. |
| EP0811685 | Escobedo, et al. |

The following patents and publication relate to PDGF/PDGFR inhibition and their description of the disease states mediated by FGFR and assays to determine such activity.

| | |
|---|---|
| 5,191,067 | Lappi, et. al. |
| 5,288,855 | Bergonzoni, et. al. |
| 5,459,015 | Janjic, et. al. |
| 5,478,804 | Calabresi, et. al. |
| 5,576,288 | Lappi, et al. |

(continued)

| | |
|---|---|
| 5,639,868 | Janjic, et. al. |
| 5,648,076 | Ross, et. al. |
| 5,670,323 | Nova, et. al. |
| 5,676,637 | Lappi, et. al. |
| 5,707,632 | Williams, et. al. |
| 5,744,313 | . Williams, et. al. |
| 5,789,182 | Yayon, et. al. |
| 5,789,382 | Wellstein |
| 5,843,893 | Courtois |
| 5,891,655 | Ornitz |
| 5,965,132 | Thorpe, et. al. |
| 6,051,230 | Thorpe, et, al. |
| 6,350,593B1 | Williams, et. al. |

**Biochemistry and cellular examples**

**1. c-Raf (Raf-1) Biochemical Assay**

The c-Raf biochemical assay was performed with a c-Raf enzyme that was activated (phosphorylated) by Lck kinase. Lck-activated c-Raf (Lck/c-Raf) was produced in Sf9 insect cells by co-infecting cells with baculoviruses expressing, under the control of the polyhedrin promoter, GST-c-Raf (from amino acid 302 to amino acid 648) and Lck (full-length). Both baculoviruses were used at the multiplicity of infection of 2.5 and the cells were harvested 48 hours post infection.

MEK-1 protein was produced in Sf9 insect cells by infecting cells with the baculovirus expressing GST-MEK-1 (full-length) fusion protein at the multiplicity of infection of 5 and harvesting the cells 48 hours post infection. Similar purification procedure was used for GST-c-Raf 302-648 and GST-MEK-1.

Transfected cells were suspended at 100 mg of wet cell biomass per mL in a buffer containing 10 mM sodium phosphate, 140 mM sodium chloride pH 7.3, 0.5% Triton X-100 and the protease inhibitor cocktail. The cells were disrupted with Polytron homogenizer and centrifuged 30,000g for 30 minutes. The 30,000g supernatant was applied onto GSH-Sepharose. The resin was washed with a buffer containing 50 mM Tris, pH 8.0, 150 mM NaCl and 0.01% Triton X-100. The GST-tagged proteins were eluted with a solution containing 100 mM Glutathione, 50 mM Tris, pH 8.0, 150 mM NaCl and 0.01 % Triton X-100. The purified proteins were dialyzed into a buffer containing 20 mM Tris, pH 7.5,150 mM NaCl and 20% Glycerol.

Test compounds were serially diluted in DMSO using three-fold dilutions to stock concentrations ranging typically from 50 μM to 20 nM (final concentrations in the assay range from 1 μM to 0.4 nM). The c-Raf biochemical assay was performed as a radioactive filtermat assay in 96-well Costar polypropylene plates (Costar 3365). The plates were loaded with 75 μL solution containing 50 mM HEPES pH 7.5, 70 mM NaCl, 80 ng of Lck/c-Raf and 1 μg MEK-1. Subsequently, 2 μL of the serially diluted individual compounds were added to the reaction, prior to the addition of ATP. The reaction was initiated with 25 μL ATP solution containing 5μM ATP and 0.3 μCi [33P]-ATP. The plates were sealed and incubated at 32 °C for 1 hour. The reaction was quenched with the addition of 50 μl of 4% Phosphoric Acid and harvested onto P30 filtermats (PerkinElmer) using a Wallac Tomtec Harvester. Filtermats were washed with 1 % Phosphoric Acid first and deinonized $H_2O$ second. The filters were dried in a microwave, soaked in scintillation fluid and read in a Wallac 1205 Betaplate Counter (Wallac Inc., Atlanta, GA, U.S.A.). The results were expressed as percent inhibition.

$$\% \text{ Inhibition} = [100-(T_{ib}/T_i)] \times 100$$

where

$T_{ib}$ = (counts per minute with inhibitor)-(background)

$T_i$ = (counts per minute without inhibitor)-(background)

**2. Bio-Plex Phospho-ERK1/2 immunoassay.**

A 96-well phospho-ERK (pERK) immunoassay, using laser flow cytometry platform has been established to measure inhibition of basal pERK in cell lines. MDA-MB-231 cells were plated at 50,000 cells per well in 96-well microtitre plates in complete growth media. For effects of test compounds on basal pERK1/2 inhibition, the next day after plating, MDA-MB-231 cells were transferred to DMEM with 0.1% BSA and incubated with test compounds diluted 1:3 to a final concentration of 3 μM to 12 nM in 0.1% DMSO. Cells were incubated with test compounds for 2 h, washed, and lysed in Bio-Plex whole cell lysis buffer A. Samples are diluted with buffer B 1:1 (v/v) and directly transferred to assay plate or frozen at -80 C degrees until processed. 50 μL of diluted MDA-MB-231 cell lysates were incubated with about 2000 of 5 micron Bio-Plex beads conjugated with an anti-ERK1/2 antibody overnight on a shaker at room temperature. The next day, biotinylated phospho-ERK1/2 sandwich immunoassay was performed, beads are washed 3 times during each incubation and then 50 μL of PE-strepavidin was used as a developing reagent. The relative fluorescence units of pERK1/2 were detected by counting 25 beads with Bio-Plex flow cell (probe) at high sensitivity. The $IC_{50}$ was calculated by taking untreated cells as maximum and no cells (beads only) as background.

**3. Immunohistochemical staining of tumor sections with anti-pERK antibodies**

Activated Raf kinase phosphorylates and activates MEK (mitogen-activated protein kinase kinase), which in turn phosphorylates and activates ERK (extracellular signal-regulated kinase), which translocates to the nucleus and modifies gene expression. This pathway is often aberrantly activated in tumor cells due to the presence of activated ras, mutant BRAF, or elevation of growth factor receptors. A semi-quantitative immunohistochemical method was developed to detect phosphorylated ERK (pERK) in human tumor biopsies as a patient selection biomarker for determining whether a patient will be responsive to a compound of the present invention, and also to assess its efficacy. The antibody used was a polyclonal antibody (rabbit) that detects the phosphorylation status of p44 and p42 (phospho-p44/42 MAPK (Thr202/Tyr204)) MAP kinases (ERK1 and ERK2). The procedure was accomplished as follows:

1. Tumor samples were obtained from patients. Samples were embedded in paraffin and sectioned.
2. Tumor section slides were deparaffinized, and slides were incubated in 950 C citrate buffer for 35 min.
3. Slides (in buffer) were placed in a preheated steamer for 30 min.
4. When complete, slides in the heated buffer were allowed to acclimate to room temperature (RT) for 30 minutes.
5. Slides were washed in Wash Buffer and loaded onto staining racks in stainer, ensuring that all slides were moist with buffer at all times.
6. Slides were blocked in a 1.5 % Hydrogen Peroxide solution for 10 min and then with normal blocking serum (goat) for 20 min.
7. Slides were washed in buffer.
8. Slides were incubated with primary anti-pERK antibodies [phospho-p44/42 MAPK (Thr202/Tyr204)] at a dilution of 1:50 for 30 min. Negative control slides remained in blocking serum.
9. Slides were rinsed with buffer.
10. Slides were incubated in a biotinylated secondary antibody solution for 30 min.
11. Slides were rinsed with buffer.
12. Slides were exposed to preformed complex comprising horseradish peroxidase conjugated to avidin for 30 min.
13. Slides were rinsed with buffer.
14. A DAB substrate chromagen was applied to the slides for 1 min.
15. Slides were rinsed with distilled water.
16. Slides were counterstained slides with hematoxylin for 1 min.
17. Slides were rinsed with buffer.
18. Slides were rinsed with distilled water.
19. Slides were rehydrated, and coverslipped with permount.
20. Each tissue section was assessed using the Bioquant TCW98 image analysis system. Five non-overlapping fields/tissue were selected and captured electronically using an Olympus BX-60 microscope and an Optronics DEI-750 color digital camera connected to a PC-based computer running the Bioquant software.

A subject having a melanoma was treated with a compound in accordance with the present invention, and then a biopsy sample was assessed using the assay described above. Fig. 1 shows the semi-quantitative procedure utilized to analyze the tissue sections.

Fig. 2 shows data establishing that, prior to the administration of the compound, a subject having melanoma had high levels of phospho-ERK in tumor tissue as compared to stromal cells, indicating that the subject was a candidate for treatment with a compound of the present invention. The subj ect was then administered a 600 mg BID dose of the

compound.

A response to the compound was observed by comparing PET scans taken at baseline vs. the second cycle. The PET scan, which had shown significant metabolic activity at baseline, was reported to be "completely silent" after treatment. This response corresponds with a decrease in the percent of nuclei expressing pERK in the post-treatment biopsy. This subject continued to have stable disease based on CT scan measurements by RECIST through cycle 6 of treatment. Thus, not only did phospho-ERK (raf activity) predict that the subject would respond to the compound by showing measurable improvement, but the compound also reduced the levels of raf activity in the cells (as measured by phosphor-ERK). The compound utilized was N-[4-chloro-3-(trifluoromethyl)phenyl]-N'-{4-[2-N-methylcarbamoyl-4-pyridyloxy]phenyl} urea.

### 4. Flk-1 (murine VEGFR-2) Biochemical Assay

This assay was performed in 96-well opaque plates (Costar 3915) in the TR-FRET format. Reaction conditions are as follows: 10 μM ATP , 25 nM poly GT-biotin , 2 nM Eu-labelled phospho-Tyr Ab, 10 nM APC, 7 nM Flk-1 (kinase domain), 1% DMSO, 50 mM HEPES pH 7.5, 10 mM $MgCl_2$, 0.1 mM EDTA, 0.015% BRIJ, 0.1 mg/mL BSA, 0.1% mercapto-ethanol). Reaction is initiated upon addition of enzyme. Final reaction volume in each well is 100 μL. Plates are read at both 615 and 665 nM on a Perkin Elmer Victor V Multilabel counter at about 1.5- 2.0 hours after reaction initiation. Signal is calculated as a ratio: (665 nm / 615 nm) * 10000 for each well.

### 5. Murine PDGFR FRET biochemical assay

This assay was formatted in a 96-well black plate (Costar 3915). The following reagents (and their sources) are used: Europium-labeled anti-phosphotyrosine antibody pY20 and streptavidin-APC; poly GT-biotin, and mouse PDGFR within DRT. The reaction conditions are as follows: 1 nM mouse PDGFR is combined with 20 μM ATP, 7nM poly GT-biotin, 1 nM pY20 antibody, 5 nM streptavidin-APC, and 1% DMSO in assay buffer (50 mM HEPES pH 7.5, 10 mM $MgCl_2$, 0.1 mM EDTA, 0:015% BRIJ 35, 0.1 mg/mL BSA, 0.1% mercaptoethanol). Reaction is initiated upon addition of enzyme. Final reaction volume in each well is 100 μL. After 90 minutes, the reaction is stopped by addition of 10 μL/ well of 5 μM staurosporine. Plates are read at both 615 and 665 nm on a Perkin Elmer VictorV Multilabel counter at about 1 hour after the reaction is stopped. Signal is calculated as a ratio: (665 nm / 615 nm) * 10000 for each well.

For $IC_{50}$ generation for both PDGFR and Flk-1, compounds were added prior to the enzyme initiation. A 50-fold stock plate was made with compounds serially diluted 1:3 in a 50% DMSO/50% dH2O solution. A 2 μL addition of the stock to the assay gave final compound concentrations ranging from 10 μM - 4.56 nM in 1% DMSO. The data were expressed as percent inhibition: % inhibition = 100-((Signal with inhibitor-background)/(Signal without inhibitor - background)) *100

### 6. pPDGFR-b sandwich ELISA in AoSMC cells

100K P3-P6 Aortic SMC were plated in each well of 12-well cluster in 1000 μL volume/ well of SGM-2 using standard cell culture techniques. Next day, cells were rinsed with 1000 uL D-PBS (Gibco) once, then serum starved in 500 uL SBM (smooth muscle cell basal media) with 0.1% BSA (Sigma, Cat A9576) overnight. Compounds were diluted at a dose range from (10 uM to 1 nM in 10-fold dilution steps in DMSO. Final DMSO concentration 0.1%). Remove old media by inversion into the sink quickly then add 100 ul of each dilution to corresponding well of cells for 1 hr at 37 ˚C. Cells were then stimulated with 10 ng/mL PDGF BB ligand for 7 minutes at 37 ˚C. The media is decanted and 150 uL of isotonic lysis buffer with protease inhibitor tablet (Complete; EDTA-free) and 0.2 mM Na vanadate is added. Cells are lysed for 15 min at 4 ˚C on shaker in cold room. Lysates are put in eppendorf tubes to which 15 uL of agarose-conjugated anti-PDGFR-b antibody is added (Santa Cruz, sc-339) and incubated at 4 ˚C overnight. Next day, beads are rinsed in 50-volumes of PBS three times and boiled in 1x LDS sample buffer (Invitrogen) for 5 minutes. Samples were run on 3-8% gradient Tris-Acetate gels (Invitrogen) and transferred onto Nitrocellulose. Membranes were blocked in 1% BSA/TBS-T for 1 hr. before incubation in anti-phospho-PDGFR-b (Tyr-857) antibody in blocking buffer (1:1000 dilution) for 1 hour. After three washes in TBS-T, membranes were incubated in Goat anti-rabbit HRP IgG (Amersham, 1:25000 dilution) for 1 hr. Three more washes followed before addition of ECL substrate. Membranes were exposed to Hyperfum-ECL. Subsequently, membranes were stripped and reprobed with anti-PDGFR-b antibody (Santa Cruz, SC-339) for total PDGFR-b.

### 7. MDA-MB231 proliferation assay

Human breast carcinoma cells (MDA MB-231, NCI) were cultured in standard growth medium (DMEM) supplemented with 10% heat-inactivated FBS at 37˚C in 5% $CO_2$ (vol/ vol) in a humidified incubator. Cells were plated at

a density of 3000 cells per well in 90 μL growth medium in a 96 well culture dish. In order to determine $T_{0h}$ CTG values, 24 hours after plating, 100 μL of CellTiter-Glo Luminescent Reagent (Promega) was added to each well and incubated at room temperature for 30 minutes. Luminescence was recorded on a Wallac Victor II instrument. The CellTiter-Glo reagent results in cell lysis and generation of a luminescent signal proportional to the amount of ATP present, which, in turn is directly proportional to the number of cells present.

Test compounds are dissolved in 100% DMSO to prepare 10 mM stocks. Stocks were further diluted 1:400 in growth medium to yield working stocks of 25 μM test compound in 0.25% DMSO. Test compounds were serially diluted in growth medium containing 0.25% DMSO to maintain constant DMSO concentrations for all wells. 60 μL of diluted test compound were added to each culture well to give a final volume of 180 μL. The cells with and without individual test compounds were incubated for 72 hours at which time ATP dependent luminescence was measured, as described previously, to yield $T_{72h}$ values. Optionally, the $IC_{50}$ values can be determined with a least squares analysis program using compound concentration versus percent inhibition.

$$\% \text{ Inhibition} = [1-(T_{72h\,test}-T_{0h})/(T_{72h\,ctrl}-T_{0h})] \times 100,$$

where

$T_{72h\ test}$ = ATP dependent luminescence at 72 hours in the presence of test compound

$T_{72h\ ctrl}$ = ATP dependent luminescence at 72 hours in the absence of test compound

$T_{0h}$ = ATP dependent luminescence at Time Zero.

**8. Tumor treatment**

To test the efficacy of a compound of the present invention against a cancer, an N-[4-chloro-3-(trifluoromethyl) phenyl]-N'-{4-[2-N-methylcarbamoyl-4-pyridyloxy]phenyl} urea salt was administered to staged HCT 116 colon (mutant k-Ras), MIA PaCa-2 pancreatic (mutant k-Ras), NCI-H460 lung (mutant k-Ras) and SK-OV-3 ovarian (wt k-Ras) xenograft models. All treatments were administered p.o. on a qd x 14 schedule. Dosages of 10, 30 and 100 mg/kg/dose produced dose-dependent inhibition of HCT-116 tumor growth that ranged between 45% and 68%. Similarly, the growth of MIA PaCa-2 tumors was inhibited 44%, 66%, and 73% at dosages of 10, 30, and 100 mg/kg/dose, respectively. The growth of NCI-H460 tumors was inhibited 27% and 56% at dosages of 10 and 30 mg/kg/dose of this Raf kinase inhibitor. The SK-OV-3 model was slightly more sensitive, generating 45%-81% tumor growth inhibitions at dosages of 3 to 100 mg/kg/ dose.

The anti-tumor efficacy of longer duration was also evaluated in the HCT 116 model. The compound produced net tumor stasis at dosages of 30 and 100 mg/kg/dose when treatment was extended to 30 days duration.

HCT 116, SK-OV-3, and MIA PaCa-2 stock tumors were maintained as serial s.c. passages of fragments in CD-1 Nu/Nu female mice (HCT 116 and SK-OV-3) or CB17 SCID female mice (MIA-PaCa-2). Treatments were administered orally and were initiated against established tumors. Tumor dimensions were measured via calipers 2-3 times per week and were converted into tumor mass by the formula [L x (W2)]2, where L and W refer to the largest and smallest dimensions, respectively.

**9. P38 Kinase Assay**

The *in vitro* inhibitory properties of compounds were determined using a p38 kinase inhibition assay. P38 activity was detected using an *in vitro* kinase assay run in 96-well microtiter plates. Recombinant human p38 (0.5 □g/mL) was mixed with substrate (myelin basic protein, 5 □g/mL) in kinase buffer (25 mM Hepes, 20 mM $MgCl_2$ and 150 mM NaCl) and compound. One μCi/well of $^{33}$P-labeled ATP (10 μM) was added to a final volume of 100 μL. The reaction was run at 32 ˚C for 30 min. and stopped with a 1M HCl solution. The amount of radioactivity incorporated into the substrate was determined by trapping the labeled substrate onto negatively charged glass fiber filter paper using a I % phosphoric acid solution and read with a scintillation counter. Negative controls include substrate plus ATP alone.

Without further elaboration, it is believe that one skilled in the art can, using the preceding description, utilize the present invention to its fullest extent. The following preferred specific embodiments are, therefore, to be construed as merely illustrative, and not limitative of the remainder of the disclosure in any way whatsoever.

TABLE 8. BIOCHEMICAL ASSAY

| SIGNALING MOLECULE | IC50 (nM) |
|---|---|
| CRAF | 2 |
| BRAF-WT | 25 |
| BRAF V599E | 38 |
| HER1 | INACTIVE > 10,000 |
| HER2 | INACTIVE > 10,000 |
| mPDGFR | 57 |
| VEGFR2 | 4 |
| mVEGFR | 1 |
| FGFR | 580 |
| p38 | 38 |
| c-KIT | 68 |
| FLT3 | 58 |
| LCK | INACTIVE 2200 |
| BCR-ABL | INACTIVE 1350 |

TABLE 9. CELLULAR ASSAY

| Pathway/Signaling molecule | IC50 (nM) |
|---|---|
| Raf Pathway phospho-ERK | |
| Breast | 80 |
| Melanoma | 880 |
| HER 1 & 2 Pathway | |
| HER1 Receptor | INACTIVE |
| HER2 Receptor | INACTIVE |
| PDGFR Pathway | |
| PDGFR-beta receptor | 80 |
| VEGFR- 2 and -3 Pathway | |
| VEGFR-2 receptor | 30 |
| mVEGFR-3 receptor | 102 |
| c-KIT Pathway | |
| c-KIT phosphor-AKT | 402 |
| FLT3 Pathway | |
| FLT3 ITD Receptor (internal tandem duplication mutation) | 20 |

## Claims

1. A method of assessing the efficacy of a compound of formula I in treating a disease in a mammalian subject, or a cell derived therefrom, comprising:

measuring the expression or activity of VEGFR-2, VEGFR-3, PDGFR-beta, and/or Flt-3 in a sample obtained from said subject who has been treated with a compound of formula I, and
determining the effects of said compound on said expression or activity,
wherein said compound of formula I is:

$$\text{B-NH-C(O)-NR-L-M-L}^1\text{-(Q)}_{1\text{-}3} \qquad (I)$$

wherein B is

(i) phenyl, optionally substituted with 1-3 substituents independently selected from the group consisting of $R^1$, $OR^1$, $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, halogen, cyano, and nitro;
(ii) naphthyl, optionally substituted with 1-3 substituents independently selected from the group consisting of $R^1$, $OR^1$, $NR^1R^2$, $S(O)_qR^1$, $SO_2NR^1R^2$, $NR^1SO_2R^2$, $C(O)R^1$, $C(O)OR^1$, $C(O)N\text{-}R^1R^2$, $NR^1C(O)R^2$, $NR^1C(O)OR^2$, halogen, cyano, and nitro;
(iii) a 5 or 6 membered monocyclic heteroaryl group, having 1-3 heteroatoms independently selected from the group consisting of O, N and S, optionally substituted with 1-3 substituents independently selected from the group consisting of $R^1$, $OR^1$, $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, halogen, cyano, oxo, and nitro; or
(iv) an 8 to 10 membered bicyclic heteroaryl group in which the first ring is bonded to the NH of Figure I and contains 1-3 heteroatoms independently selected from the group consisting of O, N, and S, and the second ring is fused to the first ring using 3 to 4 carbon atoms. The bicyclic heteroaryl group is optionally substituted with 1-3 substituents independently selected from the group consisting of $R^1$, $OR^1$, $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, halogen, cyano, oxo, and nitro.

L is

(i) phenyl, optionally substituted with 1-3 substituents independently selected from the group consisting of $C_1$-$C_5$ linear or branched alkyl, $C_1$-$C_5$ linear or branched haloalkyl, $C_1$-$C_3$ alkoxy, hydroxy, amino, $C_1$-$C_3$ alkylamino, $C_1$-$C_6$ dialkylamino, halogen, cyano, and nitro;
(ii) naphthyl, optionally substituted with 1-3 substituents independently selected from the group consisting of $C_1$-$C_5$ linear or branched alkyl, $C_1$-$C_5$ linear or branched haloalkyl, $C_1$-$C_3$ alkoxy, hydroxy, amino, $C_1$-$C_3$ alkylamino, $C_1$-$C_6$ dialkylamino, halogen, cyano, and nitro;
(iii) a 5 or 6 membered monocyclic heteroaryl group, having 1-3 heteroatoms independently selected from the group consisting of O, N and S, optionally substituted with 1-3 substituents independently selected from the group consisting of $C_1$-$C_5$ linear or branched alkyl, $C_1$-$C_5$linear or branched haloalkyl, $C_{1\text{-}3}$ alkoxy, hydroxy, amino, $C_1C_3$ alkylamino, $C_1C_6$ dialkylamino, halogen, cyano, and nitro; or
(iv) an 8 to 10 membered bicyclic heteroaryl group having 1-6 heteroatoms independently selected from the group consisting of O, N and S, optionally substituted with 1-3 substituents independently selected from the group consisting of $C_1C_5$ linear or branched alkyl, $C_{1\text{-}5}$ linear or branched haloalkyl, $C_1C_3$ alkoxy, hydroxy, amino, $C_1C_3$ alkylamino, $C_1$-$C_6$ dialkylamino, halogen, cyano, and nitro.

M is

(a) $-(CH_2)_m-O-(CH_2)_1-$
(b) $-(CH_2)_m-(CH_2)_1-$,
(c) $-(CH_2)_m-C(O)-(CH_2)_1-$,
(d) $-(CH_2)_m-NR^3-(CH_2)_1-$,
(e) $-(CH_2)_m-NR^3C(O)-(CH_2)_1-$,
(f) $-(CH_2)_m-S-(CH_2)_1-$,
(g)$-(CH_2)_m-C(O)NR^3-(CH_2)_1-$,
(h) $-(CH_2)_m-CF_2-(CH_2)_1-$,
(i) $-(CH_2)_m-CCl_2-(CH_2)_1)-$,
(j) $-(CH_2)_m-CHF-(CH_2)_1-$,
(k) $-(CH_2)_m-CH(OH)-(CH_2)_1-$;
(l) $-(CH_2)_m-C{\equiv}C-(CH_2)_1-$;
(m) $-(CH_2)_m-C{=}C-(CH_2)_1$;

(n) $-(CH_2)_m-CR^4R^5-(CH_2)_l-$;
or
(o) a single bond, where m and 1 are 0;

wherein the variables m and I are integers independently selected from 0-4,
L' is

(i) phenyl, optionally substituted with 1-2 additional substituents other than Q, independently selected from the group consisting of $R^1$, $OR^1$, $NR^1R^2$, $S(O)_qR^1$ $SO_2NR^1R^2$, $NR^1SO_2R^2$, $NR^1C(O)R^2$, $NR^1C(O)OR^2$, halogen, cyano and nitro;
(ii) naphthyl, optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogen, cyano and nitro;
(iii) a 5 and 6 membered monocyclic heteroaryl group, having 1-3 heteroatoms independently selected from the group consisting of O, N and S, optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogen, cyano and nitro and also oxides (e.g. =O, $-O^-$ or -OH);
(iv) an 8 to 10 membered bicyclic heteroaryl group, having 1-6 heteroatoms independently selected from the group consisting of O, N and S, optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogen, cyano and nitro and also oxides (e.g. =O, $-O^-$ or-OH).
(v) a saturated and partially saturated $C_3$-$C_6$ monocyclic carbocyclic moiety optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogen, cyano and, nitro;
(vi) a saturated and partially saturated $C_8$-$C_{10}$ bicyclic carbocyclic moiety, optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogen, cyano and nitro;
(vii) a saturated and partially saturated 5 and 6 membered monocyclic heterocyclic moiety, having 1-3 heteroatoms independently selected from the group consisting of O, N and S, optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2N,R^1C(O)R^2$, $NR^1C(O)OR^2$, halogen, cyano and nitro, and also oxides (e.g. =O, $-O^-$ or -OH); or
(viii) a saturated and partially saturated 8 to 10 membered bicyclic heterocyclic moiety, having 1-6 heteroatoms independently selected from the group consisting of O, N and S, optionally substituted with 1-2 additional substituents other than Q, independently selected from the group consisting of $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogen, cyano and nitro, and also oxides (e.g. =O, $-O^-$ or-OH); and

each Q is independently $C(O)R^4$, $C(O)OR^4$ and $C(O)NR^4R^5$;
wherein each $R^1$ - $R^5$ is independently selected from:

(a) hydrogen,
(b) $C_1$-$C_5$ linear, branched, or cyclic alkyl,
(c) phenyl,
(d) $C_1$-$C_3$ alkyl-phenyl, wherein the alkyl moiety is optionally substituted with halogen up to per-halo;
(e) up to per-halo substituted $C_1$-$C_5$ linear or branched alkyl. Or
(f) $-(CH_2)_q$-X, where X is a 5 or 6 membered monocyclic heterocyclic ring, containing 1-4 atoms selected from oxygen, nitrogen and sulfur, which is saturated, partially saturated, or aromatic, or a 8-10 membered bicyclic heteroaryl having 1-4 heteroatoms selected from the group consisting of 0, N and S; and wherein said alkyl moiety is optionally substituted with halogen up to per-halo,

wherein each $R^1$-$R^5$, other than per-halo substituted $C_1$-$C_5$ linear or branched alkyl, is optionally substituted with 1-3 substituents independently selected from the group consisting of $C_1$-$C_5$ linear or branched alkyl, up to perhalo substituted $C_1$-$C_5$ linear or branched alkyl, $C_1$-$C_3$ alkoxy, hydroxy, carboxy, amino, $C_1$-$C_3$ alkylamino, $C_1$-$C_6$ dialkylamino, halogen, cyano, and nitro;
wherein the variable p is an integer selected from 0, 1, or 2 and the variable q is an integer selected from 0, 1, 2, 3, or 4.

**2.** A method of claim 1, wherein said measuring the expression is determining the amounts of mRNA corresponding

to VEGFR-2, VEGFR-3, PDGFR-beta, and/or Flt-3.

**3.** A method of claim 1, wherein said measuring the expression is determining the amounts of polypeptide corresponding to VEGFR-2, VEGFR-3, PDGFR-beta, and/or Flt-3.

**4.** A method of claim 3, wherein said measuring the activity is determining the amounts of phospho-ERK.

**5.** A method of claim 4, wherein said subject has cancer, and measuring the activity is determining the amounts of phospho-ERK in peripheral blood lymphocytes or a tissue biopsy of a cancer.

**6.** A method of any of claims 1-5, further comprising comparing the expression or activity in said sample to a normal control.

**7.** A method of any of claims 1-6, further comprising comparing the expression or activity in at least one sample before treating with said compound and in at least one sample after treating with said compound.

**8.** A method of any of claims 1-7, further comprising measuring expression in at least two different samples collected at different timepoints in the treatment regimen with said compound.

**9.** A method of any of claims 1-8, wherein a reduction in expression or activity indicates that said compound is effective in treating said disease.

**10.** A method of any of claims 1-9, wherein said disease is renal cell carcinoma or melanoma.

**11.** A method of any of claims 1-10, wherein said sample comprises tumor cells.

**12.** A method of any of claims 1-11, wherein said sample comprises peripheral blood cells.

**13.** A method of any of claims 1-11, further comprising administering a compound of formula I at a plurality of timepoints.

**14.** A method of selecting subjects having a disease for treatment with a compound of formula I, comprising:

measuring the expression or activity of VEGFR-2, VEGFR-3, PDGFR-beta, and/or Flt-3, in a sample obtained from a subject having a disease.

**15.** A method of selecting subjects having a disease for treatment with a compound of formula I, comprising:

determining the the presence of a VEGFR-2, VEGFR-3, PDGFR-beta, and/or Flt-3 gene mutation in a sample obtaiuned from a subject, wherein said mutation is associated with a disease.

**16.** A method of claim 15, wherein said mutation is in the BRAF gene.

**17.** A method of claim 16, wherein said BRAF mutation is at amino acid position 599 of the coding sequence of said gene.

**18.** A method of claim 17, wherein said BRAF mutation is V599E.

**19.** A method of any of claims 15-18, wherein said disease is melanoma.

**20.** A method as in claim 1 wherein for the compounds of formula (I):

L is phenyl, optionally substituted with 1-4 halogen,
M is -O-,
B is phenyl or pyridyl, optionally substituted with 1-6 substituents independently selected from the group consisting of $R^1$ and halogen, and
$L^1$ is optionally substituted phenyl or pyridinyl.

**21.** A method as in claim 20, wherein the substituents on the groups for B and L' are selected from the group consisting of: methyl, ethyl, propyl, butyl, pentyl, isopropyl, isobutyl, sec-butyl, and tert-butyl, methoxy, ethoxy, F, Cl, Br, and I.

**22.** A method as in claim 1 wherein the pharmaceutically acceptable salt of a compound of formula I is selected from the group consisting of

a) basic salts of organic acids and inorganic acids selected from the group consisting of hydrochloric acid, hydrobromic acid, sulphuric acid, phosphoric acid, methanesulphonic acid, trifluorosulphonic acid, benzenesulfonic acid, p-toluene sulphonic acid (tosylate salt), 1-napthalene sulfonic acid, 2-napthalene sulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid; and
b) acid salts of organic and inorganic bases containing cations selected from the group consisting of alkaline cations, alkaline earth cations, the ammonium cation, aliphatic substituted ammonium cations and aromatic substituted ammonium cations.

**23.** A method of assessing the efficacy of a compound in treating a disease in a mammalian subject, or a cell derived therefrom, comprising:

measuring the expression or activity of VEGFR-2, VEGFR-3, PDGFR-beta, and/or Flt-3 in a sample obtained from said subject who has been treated with said compound, and
determining the effects of said compound on said expression or activity,
wherein said compound is:

N-(4-chloro-3-(trifluoromethyl)phenyl)-N'-(4-(2-(N-methylcarbamoyl)-4-pyridyloxy)phenyl) urea,
N-(4-bromo-3-(trifluoromethyl)phenyl)-,N'-(4-(2-(N-methylcarbamoyl)-4-pyridyloxy)phenyl) urea,
N-(4-bromo-3-(trifluoromethyl)phenyl)-N'-(4-(2-(N-methylcarbamyl)-4-pyridyloxy)-2-chlorophenyl) urea,
N-(4-chloro-3-(tritluoromerhyl)phenyl)-N'-(4-(2-carbamoyl-4-pyridyloxy)phenyl) urea,
N-(4-chloro-3-(trifluoromethyl)phenyl)-N'-(4-(1-hydroxy-2-carbamoyl-4-pyridyloxy)phenyl) urea,
N-(4-chloro-3-(trifluoromethyl)phenyl)-N'-(4-(1-hydroxy-2-(N-methylcarbamoyl)-4-pyridyl oxy)phenyl) urea,
N-(4-chloro-3-(trifluoromethyl)phenyl)-N'-(4-(2-(N-methylcarbamoyl)-4-pyridyl oxy)-2-fluorophenyl) urea,
N-(4-bromo-3-(trifluoromethyl)phenyl)-N'-(4-(2-(N-methylcarbamoyl)-4-pyridyl oxy)-2-fluorophenyl) urea,
N-(4-fluoro-3-(trifluoromethyl)phenyl)-N'-(4-(2-(N-methylcarbamoyl)-4-pyridyl oxy)-2-fluorophenyl) urea,
N-(4-chloro-3-(trifluoromethyl)phenyl)-N'-(4-(2-(N-methylcarbamoyl)-4-pyridyl oxy)-2-chlorophenyl) urea,
N-(6-(2,2,4,4-tetrafluoro-4H-benzo[1,3]dioxinyl))-N'-(4-(2-cyano-4-pyridyloxy) phenyl) urea, or
N-(6-(2,2,4,4-tetrafluoro-4H-benzo[1,3]dioxinyl))-N'-(4-(2-cyano-4-pyridyloxy)-2-fluorophenyl) urea.

**24.** A method of claim 1 wherein the compound of formula I is of the formula X below,

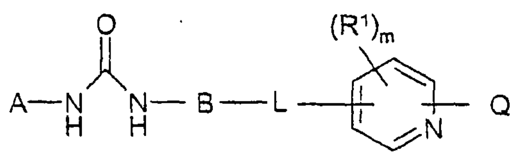

(X)

wherein:

A is phenyl, optionally substituted 1, 2 or 3 times by $R^3$ , wherein each $R^3$ is independently $C_1$-$C_5$ alkyl, $C_1$-$C_5$ haloalkyl, up to per-haloalkyl, $C_1$-$C_5$ alkoxy, $C_1$-$C_5$ haloalkoxy, up to per-haloalkoxy, halogen, cyano, or nitro; or A is a group of the formula:

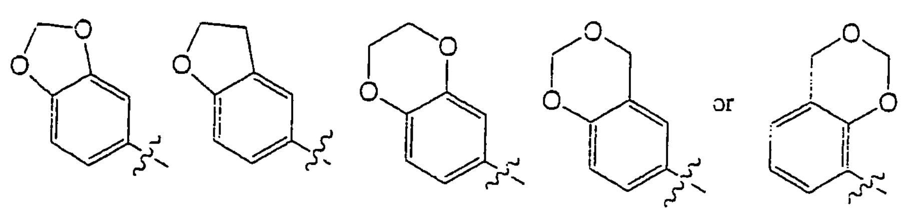

or

optionally substituted 1, 2, 3, 4, 5 or 6 times with $R^4$ wherein each $R^4$ is independently $C_1$-$C_5$ alkyl or halogen. B is phenylene, optionally substituted 1, 2 or 3 times by $R^2$, or naphthylene, optionally substituted optionally substituted 1, 2 or 3 times by $R^2$, wherein each $R^2$ is independently $C_1$-$C_5$ alkyl, $C_1$-$C_5$ haloalkyl, up to per-haloalkyl, $C_1$-$C_5$ alkoxy, $C_1$-$C_5$ haloalkoxy up to per-haloalkoxyl, halogen, cyano or nitro;
Q is cyano, -C(O)-$R^a$, or -C(O)-$NR^bR^c$, where each $R^a$, $R^b$ and $R^c$ is independently H or $C_1$-$C_5$ alkyl,
L is -O- or -S-,
m is an integer 0,1,2 or 3, and
each $R^1$ is independently halogen, $C_{1-5}$ alkyl, $C_{1-5}$ haloalkyl, up to per-haloalkyl, C $_{1-5}$ alkoxy, $C_{1-5}$ haloalkoxy, up to per-baloalkoxy, N-oxo or N-hydroxy.

**25.** A method of claim 24 wherein for the compound of formula (X), each $R^2$ is independently fluorine, chorine, bromine, methyl, ethyl, propyl, butyl, isopropyl, tert-butyl, trifluoromethyl, methoxy, CN or $NO_2$,

**26.** A method of claims 24 or 25 wherein for the compound of formula (X), each $R^3$ is independently fluorine, chorine, bromine, methyl, ethyl, propyl, butyl, pentyl, isopropyl, iso-butyl, sec-butyl, tert-butyl, trifluoromethyl, methoxy, ethoxy, trifluoromethoxy, CN or $NO_2$ and each $R^a$ is independently fluorine, chorine, bromine or methyl.

**27.** A method of any of claims 24-26 wherein for the compound of formula (X), each $R^1$ is independently methyl, ethyl, propyl, oxygen, cyano, n-oxo or n-hydroxy and each $R^a$, $R^b$ and $R^c$ is independently H or methyl.

**28.** A method of claims 24-27 wherein for the compound of formula (X), A is substituted phenyl.

**29.** A method of any of claims 24-27 wherein for the compound of formula (X), A is a group of the formula:

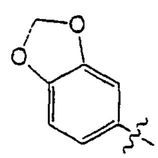
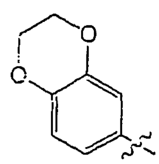
or
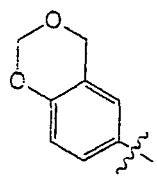

optionally substituted 1, 2, 3 or 4 times with $R^4$, wherein each $R^4$ is independently chlorine or fluorine.

**30.** A method of any of claims 24-29 wherein for the compound of formula (X), B is phenylene.

**31.** A method of any of claims 24-29 wherein for the compound of formula (X), B is naphthylene.

**32.** A method of any of claims 24-29 wherein for the compound of formula (X), B is phenylene substituted by at least one fluorine atom.

**33.** A method of any of claims 24-32 wherein for the compound of formula (X), L is oxygen.

**34.** A method of any of claims 24-33 wherein for the compound of formula (X), each $R^3$ is chlorine, bromine, tert-butyl, trifluoromethyl or methoxy.

**35.** A method of claim 24 wherein for the compound of formula (X),
A is 4-chloro-3-trifluoromethylphenyl, 4-fluoro-3-trifluoromethylpheny-1, 4-bromo-3-trifluoromethylphenyl, or 2,2,4,4-tetrafluoro-4H-benzo[1,3]dioxin-6-yl;
B is phenylene, chlorophenylene or fluorophenylene;
L is -O-;
and
Q is cyano, C(O)-$NH_2$, or C(O)-NHMe.

**36.** method of any of claims 24-35 wherein a pharmaceutically acceptable basic salt of an organic acid of a compound of formula (X) is used.

**37.** A method of claims 24-35 wherein a pharmaceutically acceptable basic salt of an organic acid of formula (X) is

administered, selected from hydrochloric acid, hydrobromic acid, sulfuric acid, phosphoric acid, methanesulfonic acid, trifluoromethanesulfonic acid, benzenesulfonic acid, p-toluene sulfonic acid (tosylate salt), 1-napthalene sulfonic acid, 2-napthalene sulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, or mandelic acid.

**38.** The method of claim 24 wherein the compound of formula X is a pharmaceutically acceptable hydrochloride, benzenesulfonate, or methanesulfonate salt of
N-(4-chloro-3-(trifluorometyl)phenyl)-N'-2-fluoro-(4-(2-(-N-methylcarbamoyl)-4-pyridyloxy)phenyl) urea or
N-(4-chloro-3-(trifluoromethyl)phenyl)-N'-(4-(2-(N-methylcarbamoyl)-4-pyridyloxy)phenyl) urea.

**39.** A method of assessing the efficacy of a compound in treating a treating a disease in a mammalian subject, or a cell derived therefrom, comprising:

measuring the expression or activity of VEGFR-2, VEGFR-3, PDGFR-beta, and/or Flt-3 in a sample obtained from said subject who has been treated with said compound, and
determining the effects of said compound on said expression or activity,
wherein said compound is
an aryl urea compound of formulae X, Y, ZA, ZB, ZC or ZD,
a salt form of a compound of formulae X, Y, ZA, ZB, ZC or ZD,
an isolated or mixed stereoisomer of a compound of formulae X, Y, ZA, ZB, ZC or ZD,
an ester of a compound of formulae X, Y, ZA, ZB, ZC or ZD,
a metabolite of a compound of formulae X, Y, ZA, ZB, ZC or ZD, or
a prodrug of a compound of formulae X, Y, ZA, ZB, ZC or ZD,

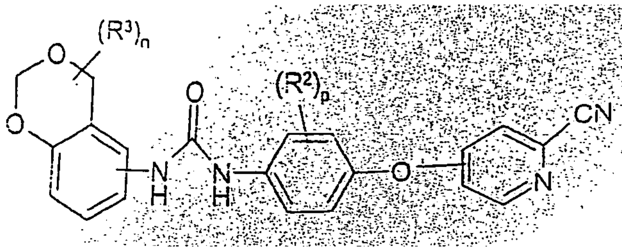
X

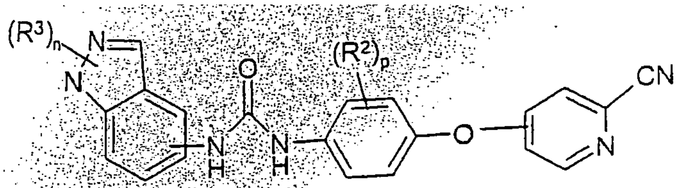
Y

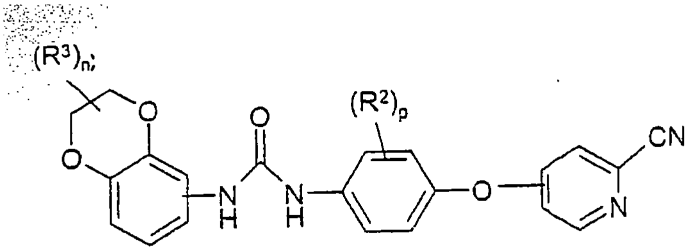
ZA

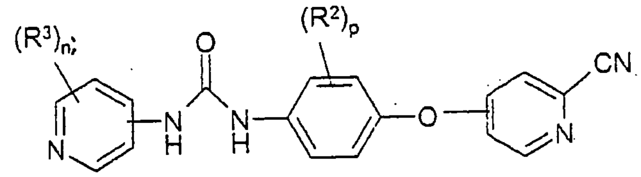

ZB

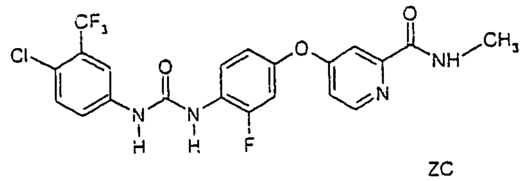

ZC

and

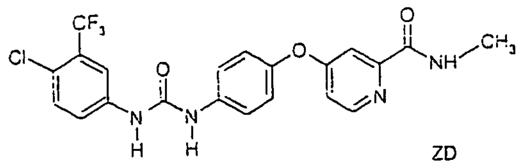

ZD

wherein
each $R^3$ is independently halogen or trifluoromethyl and
each $R^2$ is independently fluorine, chorine, bromine, ethyl, trifluoromethyl, methoxy, CN or $NO_2$.
the variable n is 0, 1, 2, 3 or 4 and
the variable p is 0, 1 or 2.

**Patentansprüche**

**1.** Verfahren zur Beurteilung der Wirksamkeit einer Verbindung der Formel I bei der Behandlung einer Krankheit in einem Säugetier-Subjekt oder einer davon abgeleiteten Zelle, umfassend:

das Messen der Expression oder Aktivität von VEGFR-2, VEGFR-3, PDGFR-beta und/oder Flt-3 in einer Probe, die von dem Subjekt, das mit der Verbindung der Formel I behandelt wurde, erhalten wurde und
das Bestimmen der Wirkungen dieser Verbindung auf die Expression oder Aktivität,

worin die Verbindung der Formel I Folgendes ist:

$$B\text{-}NH\text{-}C\ (O)\ \text{-}NH\text{-}L\text{-}M\text{-}L^1\text{-}\ (Q)\ 1\text{-}3 \qquad (I)$$

worin B Folgendes ist:

(i) Phenyl, optional substitiert mit 1-3 Substituenten, die unabhängig voneinander ausgewählt sind aus der Gruppe bestehend aus $R^1$, $OR^1$, $NR^1R^2$ $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, Halogen, Cyano und Nitro;
(ii) Naphthyl, optional substituiert mit 1-3 Substituenten, die unabhängig voneinander ausgewählt sind aus der

Gruppe bestehend aus $R^1$, $OR^1$, $NR^1R^2$ $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, Halogen, Cyano und Nitro;

(iii) eine 5- oder 6-gliedrige monozyklische Heteroarylgruppe, die 1-3 Heteroatome aufweist, die unabhängig voneinander ausgewählt sind aus der Gruppe bestehend aus O, N und S, optional substituiert mit 1-3 Substituenten, die unabhängig voneinander ausgewählt sind aus der Gruppe bestehend aus $R^1$, $OR^1$, $NR^1R^2$, $S(O)_qR^1$, $SO_ZNR^1R^2$, $NR^1SO_2R^2$, $C(O)R^1$, $C(O)OR^1$, $C(O)NR^1R^2$, $NR^1C(O)R^2$, $NR^1C(O)OR^2$, Halogen, Cyano, Oxo und Nitro; oder

(iv) eine 8- bis 10-gliedrige bizyklische Heteroarylgruppe, bei der der erste Ring an das NH der Figur I gebunden ist und 1-3 Heteroatome enthält, die unabhängig voneinander ausgewählt sind aus der Gruppe bestehend aus O, N und S und der zweite Ring an den ersten Ring unter Verwendung von 3 bis 4 Kohlenstoffatomen kondensiert ist.

Die bizyklische Heteroarylgruppe ist optional mit 1-3 Substituenten substituiert, die unabhängig voneinander ausgewählt sind aus der Gruppe bestehend aus $R^1$, $OR^1$, $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, Halogen, Cyano, Oxo und Nitro;

L ist

(i) Phenyl, optional substitiert mit 1-3 Substituenten, die unabhängig voneinander ausgewählt sind aus der Gruppe bestehend aus $C_1$-$C_5$ linearem oder verzweigtem Alkyl, $C_1$-$C_5$ linearem oder verzweigtem Haloalkyl, $C_1$-$C_3$ Alkoxy, Hydroxy, Amino, $C_1$-$C_3$ Alkylamino, $C_1$-$C_6$ Dialkylamino, Halogen, Cyano und Nitro;

(ii) Naphthyl, optional substituiert mit 1-3 Substituenten, die unabhängig voneinander ausgewählt sind aus der Gruppe bestehend aus $C_1$-$C_5$ linearem oder verzweigtem Alkyl, $C_1$-$C_5$ linearem oder verzweigtem Haloalkyl, $C_1$-$C_3$ Alkoxy, Hydroxy, Amino, $C_1$-$C_3$ Alkylamino, $C_1$-$C_6$ Dialkylamino, Halogen, Cyano und Nitro;

(iii) eine 5- oder 6-gliedrige monozyklische Heteroarylgruppe, die 1-3 Heteroatome aufweist, die unabhängig voneinander ausgewählt sind aus der Gruppe bestehend aus O, N und S, optional substituiert mit 1-3 Substituenten, die unabhängig voneinander ausgewählt sind aus der Gruppe bestehend aus $C_1$-$C_5$ linearem oder verzweigtem Alkyl, $C_1$-$C_5$ linearem oder verzweigtem Haloalkyl, $C_1$-$C_3$ Alkoxy, Hydroxy, Amino, $C_1$-$C_3$ Alkylamino, $C_1$-$C_6$ Dialkylamino, Halogen, Cyano und Nitro; oder

(iv) eine 8- bis 10-gliedrige bizyklische Heteroarylgruppe, die 1-6 Heteroatome aufweist, die unabhängig voneinander ausgewählt sind aus der Gruppe bestehend aus O, N und S, optional substituiert mit 1-3 Substituenten, die unabhängig voneinander ausgewählt sind aus der Gruppe bestehend aus $C_1$-$C_5$ linearem oder verzweigtem Alkyl, $C_1$-$C_5$ linearem oder verzweigtem Haloalkyl, $C_1$-$C_3$ Alkoxy, Hydroxy, Amino, $C_1$-$C_3$ Alkylamino, $C_1$-$C_6$ Dialkylamino, Halogen, Cyano und Nitro.

M ist

(a) $-(CH_2)_m-O-(CH_2)_l-$,
(b) $-(CH_2)_m-(CH_2)_l-$,
(c) $-(CH_2)_m-C(O)-(CH_2)_l-$,
(d) $-(CH2)_m-NR^3-(CH_2)_l-$,
(e) $-(CH_2)_m-NR^3C(O)-(CH_2)_l-$,
(f) $-(CH_2)_m-S-(CH_2)_l-$,
(g) $-(CH_2)_m-C(O)NR^3-(CH_2)_l-$,
(h) $-(CH_2)_m-CF_2-(CH_2)_l-$,
(i) $-(CH_2)_m-CCl_2-(CH_2)_l-$,
(j) $-(CH_2)_m-CHF-(CH_2)_l-$,
(k) $-(CH_2)_m-CH(OH)-(CH_2)_l-$;
(l) $-(CH_2)_m-C{\equiv}C-(CH_2)_l-$ ;
(m) $-(CH_2)_m-C=C-(CH_2)_l-$ ;
(n) $-(CH_2)_m-CR^4R^5-(CH_2)_l-$ ;
oder
(o) eine Einfachbindung, bei der m und 1 gleich 0 sind;

worin die Variablen m und 1 ganze Zahlen sind, die unabhängig voneinander ausgewählt sind aus 0-4,

L' ist

(i) Phenyl, optional substitiert mit 1-2 weiteren Substituenten, die von Q verschieden sind und die unabhängig voneinander ausgewählt sind aus der Gruppe bestehend aus $R^1$, $OR^1$, $NR^1R^2$, $S(O)_qR^1$, $SO_2NR^1R^2$,

$NR^1SO_2R^2$, $NR^1C(O)R^2$, $NR^1C(O)OR^2$, Halogen, Cyano und Nitro;

(ii) Naphthyl, optional substituiert mit 1-2 weiteren Substituenten, die von Q verschieden sind und die unabhängig voneinander ausgewählt sind aus der Gruppe bestehend aus $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, Halogen, Cyano und Nitro;

(iii) eine 5- oder 6-gliedriger monozyklische Heteroarylgruppe, die 1-3 Heteroatome aufweist, die unabhängig voneinander ausgewählt sind aus der Gruppe bestehend aus O, N und S, optional substituiert mit 1-3 Substituenten, die von Q verschieden sind und die unabhängig voneinander ausgewählt sind aus der Gruppe bestehend aus $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, Halogen, Cyano und Nitro und auch Oxide (beispielsweise =O, $-O^-$ oder -OH);

(iv) eine 8- bis 10-gliedrige bizyklische Heteroarylgruppe, die 1-6 Heteroatome aufweist, die unabhängig voneinander ausgewählt sind aus der Gruppe bestehend aus O, N und S und optional mit 1-2 weiteren Substituenten, die von Q verschieden sind substituiert ist und die unabhängig voneinander ausgewählt sind aus der Gruppe bestehend aus $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, Halogen, Cyano, Oxo und Nitro und auch Oxide (beispielsweise =O, $-O^-$ oder -OH);

(v) eine gesättigte und teilweise gesättigte $C_3$-$C_6$ monozyklische carbozyklische Einheit, die optional mit 1-2 weiteren Substituenten, die von Q verschieden sind substituiert ist und unabhängig voneinander ausgewählt sind aus der Gruppe bestehend aus $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, Halogen, Cyano und Nitro;

(vi) eine gesättigte und teilweise gesättigte $C_8$-$C_{10}$ bizyklische carbozyklische Einheit, die optional mit 1-2 weiteren Substituenten substituiert ist, die verschieden von Q sind und unabhängig voneinander ausgewählt sind aus der Gruppe bestehend aus $R^1$, $OR^1$, $NR^1R^2$, $S(O)_qR^1$, $SO_2NR^1R^2$, $NR^1SO_2R^2$, $NR^1R^2$, $NR^1C(O)R^2$, $NR^1C(O)OR^2$, Halogen, Cyano und Nitro;

(vii) eine gesättigte und teilweise gesättigte 5- und 6-gliedrige monozyklische heterozyklische Einheit, die 1-3 Heteroatome aufweist, die unabhängig voneinander ausgewählt sind aus der Gruppe bestehend aus O, N und S und optional mit 1-2 weiteren Substituenten substituiert ist, die von Q verschieden sind und unabhängig voneinander ausgewählt sind aus der Gruppe bestehend aus $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, Halogen, Cyano und Nitro und auch Oxide (beispielsweise =O, $-O^-$ oder -OH); oder

(viii) eine gesättigte und teilweise gesättigte 8- bis 10-gliedrige bizyklische heterozyklische Einheit, die 1-6 Heteroatome aufweist, die unabhängig voneinander ausgewählt sind aus der Gruppe bestehend aus O, N und S und optional mit 1-2 weiteren Substituenten substituiert ist, die von Q verschieden sind und unabhängig voneinander ausgewählt sind aus der Gruppe bestehend aus $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, Halogen, Cyano, Oxo und Nitro und auch Oxide (beispielsweise =O, $-O^-$ oder -OH); und

jedes Q ist unabhängig voneinander $C(O)R^4$, $C(O)OR^4$ und $C(O)NR^4R^5$;
worin jedes $R^1$ - $R^5$ unabhängig voneinander ausgewählt ist aus:

(a) Wasserstoff
(b) $C_1$-$C_5$ linearem, verzweigtem oder zyklischem Alkyl,
(c) Phenyl,
(d) $C_1$-$C_3$ Alkyl-Phenyl, worin die Alkyleinheit optional mit Halogen bis hin zu Per-Halogen substituiert ist,
(e) $C_1$-$C_5$ linearem oder verzweigtem Alkyl, das bis zu Per-Halogen substituiert ist, oder
(f) $-(CH_2)_q$-X, worin X ein 5- oder 6-gliedriger monozyklischer heterozyklischer Ring ist, der 1-4 Atome enthält, die ausgewählt sind aus Sauerstoff, Stickstoff und Schwefel, der gesättigt, teilweise gesättigt oder aromatisch ist oder ein 8- bis 10-gliedriger bizyklisches Heteroaryl ist, das 1-4 Heteroatome aufweist, die ausgewählt sind aus der Gruppe bestehend aus O, N und S; und worin die Alkyleinheit optional mit Halogen substituiert ist bis hin zu Per-Halogen,

worin jedes $R^1$-$R^5$, das von dem Per-Halogen substituierten $C_1$-$C_5$ linearem oder verzweigtem Alkyl verschieden ist, wahlweise mit 1-3 Substituenten substituiert ist, die unabhängig voneinander ausgewählt sind aus der Gruppe bestehend aus $C_1$-$C_5$ linearem oder verzweigtem Alkyl, bis zum Per-Halogen substituierten $C_1$-$C_5$ linearem oder verzweigtem Alkyl, $C_1$-$C_3$ Alkoxy, Hydroxy, Carboxy, Amino, $C_1$-$C_3$ Alkylamino, $C_1$-$C_6$ Dialkylamino, Halogen, Cyano und Nitro;

worin die Variable p eine ganze Zahl ist ausgewählt aus 0, 1 oder 2 und die Variable q eine ganze Zahl ist, die ausgewählt ist aus 0, 1, 2, 3 oder 4.

**2.** Verfahren nach Anspruch 1, worin das Bestimmen der Expression darin besteht, dass die Mengen an mRNA bestimmt werden, die VEGFR-2, VEGFR-3, PDGFR-beta und/oder Flt-3 entsprechen.

3. Verfahren nach Anspruch 1, worin das Messen der Expression darin besteht, dass die Mengen an Polypeptiden bestimmt werden, die VEGFR-2, VEGFR-3, PDGFR-beta und/oder Flt-3 entsprechen.

4. Verfahren nach Anspruch 3, worin das Messen der Aktivität darin besteht, dass die Menge an Phospho-ERK bestimmt wird.

5. Verfahren nach Anspruch 4, worin die Person Krebs hat und das Messen der Aktivität darin besteht, dass die Menge an Phospho-ERK in peripheren Blutlymphozyten oder einer Gewebebiopsie von Krebs bestimmt werden.

6. Verfahren nach einem der Ansprüche 1 bis 5, weiter umfassend das Vergleichen der Expression oder der Aktivität in der Probe mit einer Normalkontrolle.

7. Verfahren nach einem der Ansprüche 1 bis 6, weiter umfassend das Vergleichen der Expression oder der Aktivität in wenigstens einer Probe, bevor diese mit der Verbindung behandelt wird und in wenigstens einer Probe nach der Behandlung mit der Verbindung.

8. Verfahren nach einem der Ansprüche 1 bis 7, weiter umfassend das Messen der Expression in wenigstens zwei verschiedenen Proben, die zu verschiedenen Zeitpunkten während der Behandlung mit der Verbindung gesammelt wurden.

9. Verfahren nach einem der Ansprüche 1 bis 8, worin eine Reduktion in der Expression oder der Aktivität anzeigt, dass die Verbindung bei der Behandlung der Krankheit wirksam ist.

10. Verfahren nach einem der Ansprüche 1 bis 9, worin die Krankheit ein Nierenzellenkarzinom oder -melanom ist.

11. Verfahren nach einem der Ansprüche 1 bis 10, worin die Probe Tumorzellen umfasst.

12. Verfahren nach einem der Ansprüche 1 bis 11, worin die Probe periphere Blutzellen umfasst.

13. Verfahren nach einem der Ansprüche 1 bis 11, weiter umfassend das Verabreichen einer Verbindung der Formel I zu mehreren Zeitpunkten.

14. Verfahren zur Auswahl von Personen, die eine Krankheit haben zur Behandlung mit einer Verbindung der Formel I, umfassend:

das Messen der Expression oder der Aktivität von VEGFR-2, VEGFR-3, PDGFR-beta und/oder Flt-3 in einer Probe, die von einer Person erhalten wurde, die eine Krankheit hat.

15. Verfahren zur Auswahl von Personen, die eine Krankheit haben zur Behandlung mit einer Verbindung der Formel I umfassend:

das Bestimmen der Gegenwart einer VEGFR-2, VEGFR-3, PDGFR-beta und/oder Flt-3 Genmutation einer Probe, die von einer Person erhalten wurde und wobei die Mutation mit einer Krankheit verbunden ist.

16. Verfahren nach Anspruch 15, worin sich die Mutation im BRAF-Gen befindet.

17. Verfahren nach Anspruch 16, worin sich die BRAF-Mutation an der Aminosäurestelle 599 der kodierenden Sequenz des Gens befindet.

18. Verfahren nach Anspruch 17, worin die BRAF-Mutation V599E ist.

19. Verfahren nach einem der Ansprüche 15 bis 18, worin die Krankheit ein Melanom ist.

20. Verfahren nach Anspruch 1, worin für die Verbindungen der Formel I
L Phenyl ist, optional substituiert mit 1-4 Halogenen, M ist -O-,
B Phenyl oder Pyridyl ist, optional substituiert mit 1-6 Substituenten, die unabhängig voneinander ausgewählt sind aus der Gruppe bestehend aus $R^1$ und Halogen und $L^1$ optional substituiertes Phenyl oder Pyridinyl ist.

**21.** Verfahren nach Anspruch 20, worin die Substituenten an den Gruppen für B und L' ausgewählt sind aus der Gruppe bestehend aus Methyl, Ethyl, Propyl, Butyl, Pentyl, Isopropyl, Isobutyl, sec-Butyl und tert-Butyl, Methoxy, Ethoxy, F, Cl, Br und I.

**22.** Verfahren nach Anspruch 1, worin das pharmazeutisch verträgliche Salz einer Verbindung der Formel I ausgewählt ist aus der Gruppe bestehend aus

a) basischen Salzen von organischen Säuren und anorganischen Säuren, ausgewählt aus der Gruppe bestehend aus Salzsäure, Bromwasserstoffsäure, Schwefelsäure, Phosphorsäure, Methansulfonsäure, Trifluorosulfonsäure, Benzolsulfonsäure, Paratoluolsulfonsäure (Tosylatsalz), 1-Naphtholsulfonsäure, 2-Naphtholsulfonsäure, Essigsäure, Trifluoressigsäure, Äpfelsäure, Weinsäure, Zitronensäure, Milchsäure, Oxalsäure, Bernsteinsäure, Fumarsäure, Maleinsäure, Benzoesäure, Salicylsäure, Phenylessigsäure und Mandelsäure; und
b) sauren Salzen von organischen und anorganischen Basen, die Kationen enthalten, die ausgewählt sind aus der Gruppe bestehend aus Alkalikationen, Erdalkalikationen, den Ammoniumkationen, aliphatisch substituierten Ammoniumkationen und aromatisch substituierten Ammoniumkationen.

**23.** Verfahren zur Beurteilung der Wirksamkeit einer Verbindung bei der Behandlung einer Krankheit in einem Säugetier-Subjekt oder einer Zelle, die daraus abgeleitet ist, umfassend
das Messen der Expression oder Aktivität von VEGFR-2, VEGFR-3, PDGFR-beta und/oder Flt-3 in einer Probe, die von dem Subjekt erhalten wurde, das mit der Verbindung behandelt wurde und
das Bestimmen der Wirkungen der Verbindung auf die Expression oder Aktivität, worin die Verbindung folgendes ist :

N-(4-chloro-3-(trifluoromethyl)phenyl)-N'-(4-(2-(N-methylcarbamoyl)-4-pyridyloxy)phenyl)Harnstoff,
N-(4-bromo-3-(trifluoromethyl)phenyl)-N'-(4-(2-(N-methylcarbamoyl)-4-pyridyloxy)phenyl)Harnstoff,
N-(4-bromo-3-(trifluoromethyl)phenyl)-N'-(4-(2-(N-methylcarbamoyl)-4-pyridyloxy)-2-chlorophenyl)Harnstoff,
N-(4-chloro-3-(trifluoromethyl)phenyl)-N'-(4-(2-carbamoyl-4-pyridyloxy)phenyl)Harnstoff,
N-(4-chloro-3-(trifluoromethyl)phenyl)-N'-(4-(1-hydroxy-2-carbamoyl-4-pyridyloxy)phenyl)Harnstoff,
N-(4-chloro-3-(trifluoromethyl)phenyl)-N'-(4-(1-hydroxy-2-(N-methylcarbamoyl)-4-pyridyloxy)phenyl) Harnstoff,
N-(4-bromo-3-(trifluoromethyl)phenyl)-N'-(4-(2-(N-methylcarbamoyl)-4-pyridyloxy)phenyl)Harnstoff
N-(4-bromo-3-(trifluoromethyl)phenyl)-N'-(4-(2-(N-methylcarbamoyl)-4-pyridyloxy)-2-fluorophenyl)Harnstoff,
N-(4-fluoro-3-(trifluoromethyl)phenyl)-N'-(4-(2-(N-methylcarbamoyl)-4-pyridyloxy)-2-fluorophenyl)Harnstoff,
N-(4-chloro-3-(trifluoromethyl)phenyl)-N'-(4-(2-(N-methylcarbamoyl)-4-pyridyloxy)-2-chlorophenyl)Harnstoff
N-(6-(2,2,4,4-tetrafluoro-4H-benzol[1,3]dioxinyl))-N'-(4-(2-cyano-4-pyridyloxy)phenyl)Harnstoff oder
N-(6-(2,2,4,4-tetrafluoro-4H-benzol[1,3]dioxinyl))-N'-(4-(2-cyano-4-pyridyloxy)-2-cyano-4-pyridyloxy)-2-fluorophenyl)Harnstoff.

**24.** Verfahren nach Anspruch 1, worin die Verbindung der Formel I die untenstehende Formel X hat

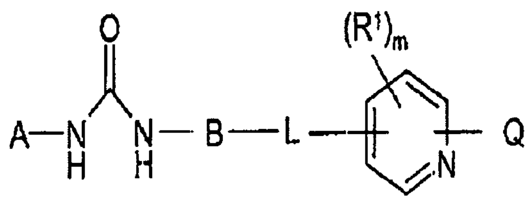

(X)

worin:

A Phenyl ist, optional 1, 2 oder 3 mal durch $R^3$ substituiert, worin jedes $R^3$ unabhängig voneinander $C_1$-$C_5$ Alkyl, $C_1$-$C_5$ Haloalkyl bis zu Per-Haloalkyl, $C_1$-$C_5$ Alkoxy, $C_1$-$C_5$ Haloalkoxy bis zu Per-Haloalkoxy, Halogen, Cyano oder Nitro ist;

oder A ist eine Gruppe der Formel

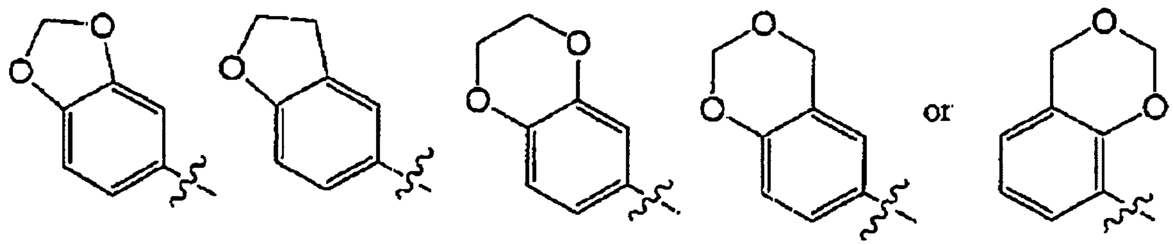

or

die optional 1, 2, 3, 4, 5 oder 6 mal mit $R^4$ substituiert ist, worin jedes $R^4$ unabhängig voneinander $C_1$-$C_5$ Alkyl oder Halogen ist;

B ist Phenylen, optional 1, 2 oder 3 mal mit $R^2$ substituiert, oder Naphthylen, optional 1, 2 oder 3 mal mit $R^2$ substituiert, worin jedes $R^2$ unabhängig voneinander $C_1$-$C_5$ Alkyl, $C_1$-$C_5$ Haloalkyl bis zu Per-Haloalkyl, $C_1$-$C_5$ Alkoxy, $C_1$-$C_5$ Haloalkoxy bis zu Per-Haloalkoxy, Halogen, Cyano oder Nitro ist;
Q ist Cyano, -C(O)-$R^8$ oder -C(O)-$NR^bR^c$, worin jedes $R^a$, $R^b$ und $R^c$ unabhängig voneinander H oder $C_1$-$C_5$ Alkyl ist,
L ist -O- oder -S-,
m ist eine ganze Zahl 0, 1, 2 oder 3 und

jedes $R^1$ ist unabhängig voneinander Halogen, $C_1$-$C_5$ Alkyl, $C_1$-$C_5$ Haloalkyl bis zu Per-Haloalkyl, $C_1$-$C_5$ Alkoxy, $C_1$-$C_5$ Haloalkoxy bis zu Per-Haloalkoxy, N-Oxo oder N-Hydroxy.

**25.** Verfahren nach Anspruch 24, worin für die Verbindung der Formel (X) jedes $R^2$ unabhängig voneinander Fluor, Chlor, Brom, Methyl, Ethyl, Propyl, Butyl, Isopropyl, tert-Butyl, Trifluoromethyl, Methoxy, CN oder $NO_2$ ist.

**26.** Verfahren nach den Ansprüchen 24 oder 25, worin für die Verbindung der Formel (X) jedes $R^3$ unabhängig voneinander Fluor, Chlor, Brom, Methyl, Ethyl, Propyl, Butyl, Penthyl, Isopropyl, Isobutyl, sec-Butyl, tert-Butyl, Trifluoromethyl, Methoxy, Ethoxy, Trifluoromethoxy, CN oder $NO_2$ ist und jedes $R^4$ ist unabhängig voneinander Fluor, Chlor, Brom oder Methyl.

**27.** Verfahren nach einem der Ansprüche 24 - 26, worin für die Verbindung der Formel (X) jedes $R^1$ unabhängig voneinander Methyl, Ethyl, Propyl, Sauerstoff, Cyano, n-Oxo oder n-Hydroxy ist und jedes $R^a$, $R^b$ und $R^c$ unabhängig voneinander H oder Methyl ist.

**28.** Verfahren nach einem der Ansprüche 24 - 27, worin für die Verbindung der Formel (X) A ein substituiertes Phenyl ist.

**29.** Verfahren nach einem der Ansprüche 24 - 27, worin für die Verbindung der Formel (X) A eine Gruppe der Formel

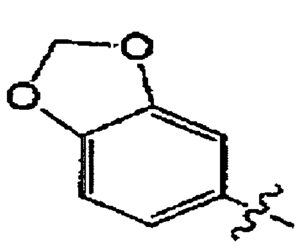

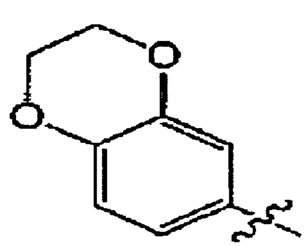

or

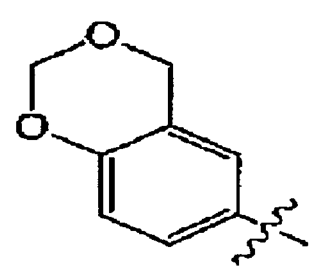

ist, die optional 1, 2, 3 oder 4 mal mit $R^4$ substituiert ist, worin $R^4$ unabhängig voneinander Chlor oder Fluor ist.

**30.** Verfahren nach einem der Ansprüche 24 - 29, worin für die Verbindung der Formel (X) B Phenylen ist.

**31.** Verfahren nach einem der Ansprüche 24 - 29, worin für die Verbindung der Formel (X) B Naphthylen ist.

**32.** Verfahren nach einem der Ansprüche 24 - 29, worin für die Verbindung der Formel (X) B ein Phenylen ist, das durch wenigstens ein Fluoratom substituiert ist.

**33.** Verfahren nach einem der Ansprüche 24 - 32, worin für die Verbindung der Formel (X) L Sauerstoff ist.

**34.** Verfahren nach einem der Ansprüche 24 - 33, worin für die Verbindung der Formel (X) jedes $R^3$ Chlor, Brom, tert-Butyl, Trifluoromethyl oder Methoxy ist.

**35.** Verfahren nach Anspruch 24, worin für die Verbindung der Formel (X)

A 4-chloro-3-trifluoromethylphenyl, 4-fluoro-3-trifluoromethylphenyl, 4-bromo-3-trifluoromethylphenyl oder 2,2,4,4-tetrafluoro-4H-benzo [1,3]dioxin-6-yl ist;
B Phenylen, Chlorophenylen oder Fluorophenylen ist;
L ist -O-;
und
Q ist Cyano, C(O)-$NH_2$ oder C(O)-NHMe.

**36.** Verfahren nach einem der Ansprüche 24 - 35, worin ein pharmazeutisch verträgliches Salz einer organischen Säure einer Verbindung der Formel (X) verwendet wird.

**37.** Verfahren nach einem der Ansprüche 24 - 35, worin ein pharmazeutisch verträgliches basisches Salz einer organischen Säure der Formel (X) verabreicht wird, welches ausgewählt ist aus Salzsäure, Bromwasserstoffsäure, Schwefelsäure, Phosphorsäure, Methansulfonsäure, Trifluoromethansulfonsäure, Benzolsulfonsäure, Paratoluolsulfonsäure (Tosylatsalz), 1-Naphtholsulfonsäure, 2-Naphtholsulfonsäure, Essigsäure, Trifluoressigsäure, Äpfelsäure, Weinsäure, Zitronensäure, Milchsäure, Oxalsäure, Bernsteinsäure, Fumarsäure, Maleinsäure, Benzoesäure, Salycylsäure, Phenylessigsäure oder Mandelsäure.

**38.** Verfahren nach Anspruch 24, worin die Verbindung der Formel (X) ein pharmazeutisch verträgliches Hydrochlorid-, Benzolsulfonat- oder Methansulfonat-Salz von

N-(4-chloro-3-(trifluoromethyl)phenyl)-N'-2-fluoro-(4-(2-(N-methylcarbamoyl)-4-pyridyloxy)phenyl)Harnstoff oder
N-(4-chloro-3-(trifluoromethyl)phenyl)-N'-(4-(2-(N-methylcarbamoyl)phenyl)Harnstoff ist.

**39.** Verfahren zur Beurteilung der Wirksamkeit einer Verbindung bei der Behandlung einer Krankheit in einem Säugetier-Subjekt oder einer daraus abgeleiteten Zelle umfassend:

das Messen der Expression oder Aktivität von VEGFR-2, VEGFR-3, PDGFR-beta und/oder Flt-3 in einer Probe, die von diesem Subjekt erhalten wurde, das mit dieser Verbindung behandelt wurde und
das Bestimmen der Wirkungen der Verbindung auf die Expression oder Aktivität,
worin die Verbindung folgendes ist

eine Aryl-Harnstoff-Verbindung der Formeln X, Y, ZA, ZB, ZC oder ZD,
eine Salzform einer Verbindung der Formeln X, Y, ZA, ZB, ZC oder ZD,
ein isoliertes oder gemischtes Stereoisomer einer Verbindung der Formel X, Y, ZA, ZB, ZC oder ZD,
ein Ester einer Verbindung der Formeln X, Y, ZA, ZB, ZC oder ZD,
ein Metaboliten einer Verbindung der Formel X, Y, ZA, ZB, ZC oder ZD oder
ein Vorläufermedikament einer Verbindung der Formel X, Y, ZA, ZB, ZC oder ZD,

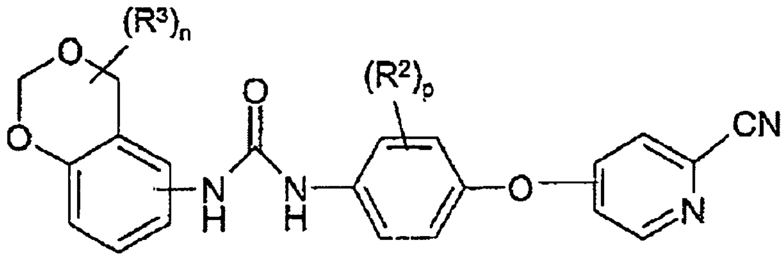

X

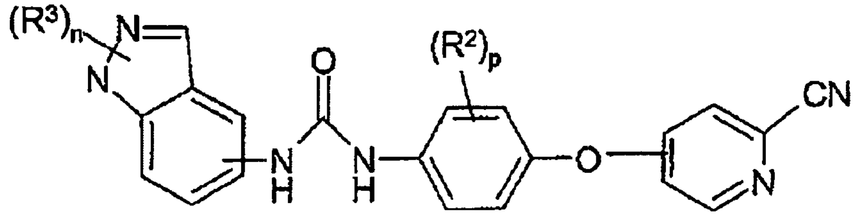

Y

,

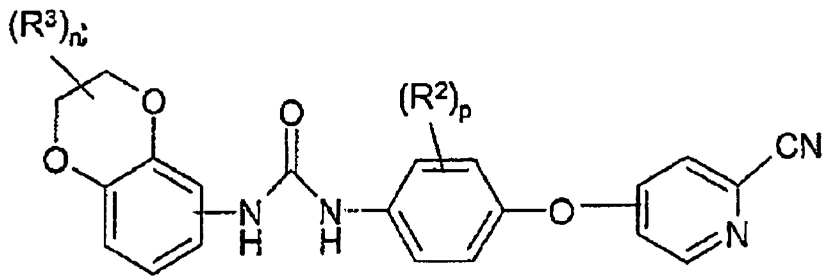

ZA

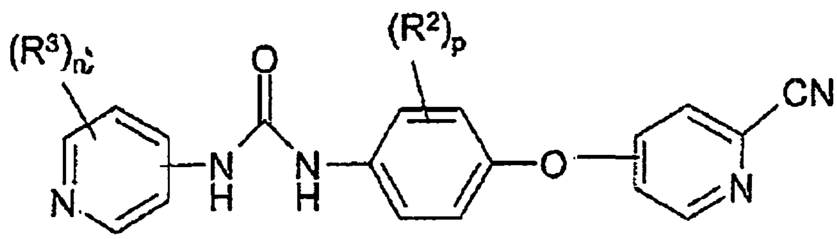

ZB

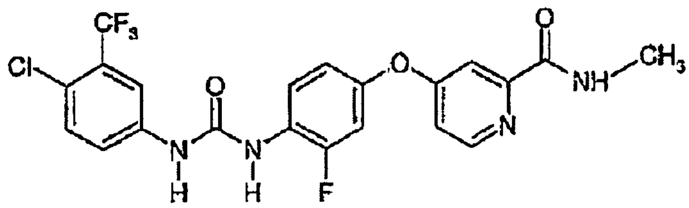

ZC

and

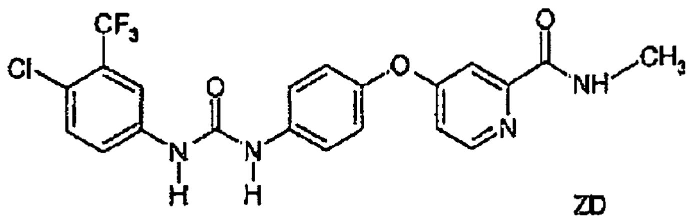

ZD

worin
jedes $R^3$ unabhängig voneinander Halogen oder Trifluoromethyl und
jedes $R^2$ unabhängig voneinander Fluor, Chlor, Brom, Methyl, Trifluormethyl, Methoxy, CN oder $NO_2$ ist ;
die Variable n ist 0,1,2,3 oder 4 ist und
die Variable p ist 0,1 oder 2.

**Revendications**

**1.** Procédé pour évaluer l'efficacité d'un composé de formule I dans le traitement d'une maladie chez un sujet mammifère, ou une cellule dérivée de celui-ci, comprenant :

la mesure de l'expression ou de l'activité de VEGFR-2, VEGFR-3, PDGFR-bêta et/ou Flt-3 dans un échantillon obtenu sur ledit sujet qui a été traité avec un composé de formule I, et
la détermination des effets dudit composé sur ladite expression ou activité,
dans lequel ledit composé de formule I est :

$$\mathbf{B\text{-}NH\text{-}C(O)\text{-}NH\text{-}L\text{-}M\text{-}L^1\text{-}(Q)_{1\text{-}3}} \qquad (I)$$

dans laquelle B est

(i) phényle, facultativement substitué avec 1-3 substituants choisis indépendamment dans le groupe consistant en $R^1$, $OR^1$ $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, halogène, cyano et nitro ;
(ii) naphtyle, facultativement substitué avec 1-3 substituants choisis indépendamment dans le groupe consistant en $R^1$, $OR^1$, $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, halogène, cyano et nitro ;
(iii) un groupe hétéroaryle monocyclique à 5 ou 6 éléments ayant 1-3 hétéroatomes indépendamment choisis dans le groupe O, N et S, facultativement substitué avec 1-3 substituants choisis indépendamment dans le groupe consistant en $R^1$, $OR^1$, $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR^2$, halogène, cyano, oxo et nitro ; ou
(iv) un groupe hétéroaryle bicyclique à 8 à 10 éléments dans lequel le premier cycle est lié à NH de la figure I et contient 1-3 hétéroatomes indépendamment choisis dans le groupe consistant en O, N et S, et le second cycle est fusionné au premier cycle en utilisant 3 à 4 atomes de carbone. Le groupe hétéroaryle bicyclique est

facultativement substitué avec 1-3 substituants choisis indépendamment dans le groupe consistant en $R^1$, $OR^1$, $NR^1R^2$, $S(O)_qR^1$, $SO_2NR^1R^2$, $NR^1SO_2R^2$, $C(O)R^1$, $C(O)OR^1$, $C(O)NR^1R^2$, $NR^1C(O)R^2$, $NR^1C(O)OR_2$, halogène, cyano, oxo et nitro.

L est

(i) phényle, facultativement substitué avec 1-3 substituants choisis indépendamment dans le groupe consistant en alkyle $C_1$-$C_5$ linéaire ou ramifié, haloalkyle $C_1$-$C_5$ linéaire ou ramifié, alcoxy $C_1$-$C_3$, hydroxy, amino, alkylamino $C_1$-$C_3$, dialkylamino $C_1$-$C_6$, halogène, cyano et nitro ;
(ii) naphtyle, facultativement substitué avec 1-3 substituants choisis indépendamment dans le groupe consistant en alkyle $C_1$-$C_5$ linéaire ou ramifié, haloalkyle $C_1$-$C_5$ linéaire ou ramifié, alcoxy $C_1$-$C_3$, hydroxy, amino, alkylamino $C_1$-$C_3$, dialkylamino $C_1$-$C_6$, halogène, cyano et nitro ;
(iii) un groupe hétéroaryle monocyclique à 5 ou 6 éléments, ayant 1-3 hétéroatomes choisis indépendamment dans le groupe consistant en O, N et S, facultativement substitué avec 1-3 substituants choisis indépendamment dans le groupe consistant en alkyle $C_1$-$C_5$ linéaire ou ramifié, haloalkyle $C_1$-$C_5$ linéaire ou ramifié, alcoxy $C_1$-$C_3$, hydroxy, amino, alkylamino $C_1$-$C_3$, dialkylamino $C_1$-$C_6$, halogène, cyano et nitro ; ou
(iv) un groupe hétéroaryle bicyclique à 8 à 10 éléments ayant 1-6 hétéroatomes choisis indépendamment dans le groupe consistant en O, N et S, facultativement substitué avec 1-3 substituants choisis indépendamment dans le groupe consistant en alkyle $C_1$-$C_5$ linéaire ou ramifié, haloalkyle $C_1$-$C_5$ linéaire ou ramifié, alcoxy $C_1$-$C_3$, hydroxy, amino, alkylamino $C_1$-$C_3$, dialkylamino $C_1$-$C_6$, halogène, cyano et nitro.

M est

(a) $-(CH_2)_m-O-(CH_2)_1-$ ;
(b) $-(CH_2)_m-(CH_2)_1-$ ;
(c) $-(CH_2)_m-C(O)-(CH_2)_1-$ ;
(d) $-(CH_2)_m-NR^3-(CH_2)_1-$ ;
(e) $-(CH_2)_m-NR^3C(O)-(CH_2)_1-$ ;
(f) $-(CH_2)_m-S-(CH_2)_1-$ ;
(g) $-(CH_2)_m-C(O)NR^3-(CH_2)_1-$ ;
(h) $-(CH_2)_m-CF_2-(CH_2)_1-$ ;
(i) $-(CH_2)_m-CCl_2-(CH_2)_1-$ ;
(j) $-(CH_2)_m-CHF-(CH_2)_1-$ ;
(k) $-(CH_2)_m-CH(OH)-(CH_2)_1-$
(l) $-(CH_2)_m-C{\equiv}C-(CH_2)_1-$ ;
(m) $-(CH_2)_m-C=C-(CH_2)_1-$ ;
(n) $-(CH_2)_m-CR^4R^5-(CH_2)_1-$ ;
ou
(o) une liaison simple, où m et 1 sont 0 ;

les variables m et 1 étant des nombres entiers choisis indépendamment parmi 0-4,
L' est

(i) phényle, facultativement substitué avec 1-2 substituants supplémentaires autres que Q, indépendamment choisis dans le groupe consistant en $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogène, cyano et nitro ;
(ii) naphtyle, facultativement substitué avec 1-2 substituants supplémentaires autres que Q, indépendamment choisis dans le groupe consistant en $R^1$, $OR^1$, $NR^1R^2$, $S(O)_qR^1$, $SO_2NR^1R^2$, $NR^1SO_2R2$, $NR^1C(O)R^2$, $NR^1C(O)OR^2$, halogène, cyano et nitro ;
(iii) un groupe hétéroaryle monocyclique à 5 et 6 éléments, ayant 1-3 hétéroatomes indépendamment choisis dans le groupe consistant en O, N et S, facultativement substitué avec 1-2 substituants supplémentaires autres que Q, indépendamment choisis dans le groupe consistant en $R^1$, $OR^1$, $NR^1R^2$, $S(O)_qR^1$, $SO_2NR^1R^2$, $NR^1SO_2R^2$ $NR^1C(O)R^2$, $NR^1C(O)OR^2$, halogène, cyano et nitro et aussi oxydes (par exemple =O, $-O^-$ ou -OH) ;
(iv) un groupe hétéroaryle bicyclique à 8 à 10 éléments ayant 1-6 hétéroatomes choisis indépendamment dans le groupe consistant en O, N, S, facultativement substitué avec 1-2 substituants supplémentaires autres que Q, indépendamment choisis dans le groupe consistant en $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogène, cyano et nitro et aussi oxydes (par exemple =O, $-O^-$ ou -OH) ;
(v) un groupement carbocyclique monocyclique $C_3$-$C_6$ saturé et partiellement saturé facultativement substitué

avec 1-2 substituants supplémentaires autres que Q, indépendamment choisis dans le groupe consistant en $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogène, cyano et nitro ;
(vi) un groupement carbocyclique bicyclique $C_8$-$C_{10}$ saturé et partiellement saturé, facultativement substitué avec 1-2 substituants supplémentaires autres que Q, indépendamment choisis dans le groupe consistant en $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogène, cyano et nitro ;
(vii) un groupement hétérocyclique monocyclique à 5 et 6 éléments saturé et partiellement saturé, ayant 1-3 hétéroatomes indépendamment choisis dans le groupe consistant en O, N et S, facultativement substitué avec 1-2 substituants supplémentaires autres que Q, indépendamment choisis dans le groupe consistant en $R^1$, $OR^1$, $NR^1R^2$, $S(O)_qR^1$, $SO_2NR^1R^2$, $NR^1SO_2R^2$, $NR^1C(O)R^2$, $NR^1C(O)OR^2$, halogène, cyano et nitro et aussi oxydes (par exemple =O, $-O^-$ ou -OH) ; ou
(viii) un groupement hétérocyclique bicyclique à 8 à 10 éléments saturé et partiellement saturé, ayant 1-6 hétéroatomes indépendamment choisis dans le groupe consistant en O, N et S, facultativement substitué avec 1-2 substituants supplémentaires autres que Q, indépendamment choisis dans le groupe consistant en $R^1$, $OR^1$, $NR^1R^2$, $S(O)_qRSO_2NR^1R^2$, $NR^1SO_2R^2$, $NR^1C(O)R^2$, $NR^1C(O)OR^2$, halogène, cyano et nitro et aussi oxydes (par exemple =O, $-O^-$ ou -OH) ; et

chaque Q est indépendamment $C(O)R^4$, $C(O)OR^4$ et $C(O)NR^4R^5$ ;
où chaque $R^1$-$R^5$ est indépendamment choisi parmi :

(a) hydrogène,
(b) alkyle $C_1$-$C_5$ linéaire, ramifié ou cyclique,
(c) phényle,
(d) phényle-alkyle $C_1$-$C_3$, où le groupement alkyle est facultativement substitué avec halogène jusqu'à perhalogénation ;
(e) alkyle linéaire ou ramifié $C_1$-$C_5$ substitué jusqu'à perhalogénation, ou
(f) $-(CH_2)_q$-X, où X est un cycle hétérocyclique monocyclique à 5 ou 6 éléments, contenant 1 à 4 atomes choisis parmi oxygène, azote et soufre, qui est saturé, partiellement saturé ou aromatique, ou un hétéroaryle bicyclique à 8-10 éléments ayant 1-4 hétéroatomes choisis dans le groupe consistant en O, N et S ; et où ledit groupement alkyle est facultativement substitué avec halogène jusqu'à perhalogénation,

où chaque $R^1$ - $R^5$, autre que alkyle $C_1$-$C_5$ linéaire ou ramifié substitué jusqu'à perhalogénation, est facultativement substitué avec 1-3 substituants indépendamment choisis dans le groupe consistant en alkyle linéaire ou ramifié $C_1$-$C_5$, alkyle $C_1$-$C_5$ linéaire ou ramifié substitué jusqu'à perhalogénation, alcoxy $C_1$-$C_3$, hydroxy, carboxy, amino, alkylamino $C_1$-$C_3$, dialkylamino $C_1$-$C_6$, halogène, cyano et nitro ;
où la variable p est un entier choisi parmi 0, 1 ou 2 et la variable q est un nombre entier choisi parmi 0, 1, 2, 3 ou 4.

**2.** Procédé selon la revendication 1, dans lequel ladite mesure de l'expression détermine les quantités d'ARNm correspondant à VEGFR-2, VEGFR-3, PDGFR-bêta, et/ou Flt-3.

**3.** Procédé selon la revendication 1, dans lequel ladite mesure de l'expression détermine les quantités de polypeptide correspondant à VEGFR-2, VEGFR-3, PDGFR-bêta, et/ou Flt-3.

**4.** Procédé selon la revendication 3, dans lequel ladite mesure de l'activité détermine les quantités de phospho-ERK.

**5.** Procédé selon la revendication 4, dans lequel ledit sujet est atteint d'un cancer, et la mesure de l'activité détermine les quantités de phospho-ERK dans les lymphocytes du sang périphérique ou un tissu cancéreux obtenu par biopsie.

**6.** Procédé selon l'une quelconque des revendications 1-5, comprenant en outre la comparaison de l'expression ou de l'activité dans ledit échantillon à un contrôle normal.

**7.** Procédé selon l'une quelconque des revendications 1-6, comprenant en outre la comparaison de l'expression ou de l'activité dans au moins un échantillon avant le traitement avec ledit composé et dans au moins un échantillon après le traitement avec ledit composé.

**8.** Procédé selon l'une quelconque des revendications 1-7, comprenant en outre la mesure de l'expression dans au moins deux échantillons différents collectés à des moments différents dans le régime thérapeutique avec ledit composé.

**9.** Procédé selon l'une quelconque des revendications 1-8, dans lequel une réduction de l'expression ou de l'activité indique que ledit composé est efficace pour traiter ladite maladie.

**10.** Procédé selon l'une quelconque des revendications 1-9, dans lequel ladite maladie est un carcinome des cellules rénales ou un mélanome.

**11.** Procédé selon l'une quelconque des revendications 1-10 dans lequel ledit échantillon comprend des cellules tumorales.

**12.** Procédé selon l'une quelconque des revendications 1-11, dans lequel ledit échantillon comprend des cellules du sang périphérique.

**13.** Procédé selon l'une quelconque des revendications 1-11, comprenant en outre l'administration d'un composé de formule I en une pluralité de moments dans le temps.

**14.** Procédé de sélection de sujets ayant une maladie pour traitement avec un composé de formule I, comprenant :

la mesure de l'expression ou de l'activité de VEGFR-2, VEGFR-3, PDGFR-bêta, et/ou Flt-3, dans un échantillon obtenu sur un sujet ayant une maladie.

**15.** Procédé de sélection de sujets ayant une maladie pour traitement avec un composé de formule I, comprenant :

la détermination de la présence d'une mutation du gène VEGFR-2, VEGFR-3, PDGFR-bêta, et/ou Flt-3 dans un échantillon obtenu sur un sujet, ladite mutation étant associée à une maladie.

**16.** Procédé selon la revendication 15, dans lequel ladite mutation est dans le gène BRAF.

**17.** Procédé selon la revendication 16, dans lequel ladite mutation BRAF est en position d'acide aminé 599 de la séquence codante dudit gène.

**18.** Procédé selon la revendication 17, dans lequel ladite mutation BRAF est V599E.

**19.** Procédé selon l'une quelconque des revendications 15-18, dans lequel ladite maladie est le mélanome.

**20.** Procédé selon la revendication 1, dans lequel, pour les composés de formule (I) :

L est phényle, facultativement substitué avec 1-4 halogènes,
M est -O-,
B est phényle ou pyridyle, facultativement substitué avec 1-6 substituants indépendamment choisis dans le groupe consistant en $R^1$ et halogène, et
$L^1$ est phényle ou pyridinyle facultativement substitués.

**21.** Procédé selon la revendication 20, dans lequel les substituants sur les groupes pour B et L' sont choisis dans le groupe consistant en : méthyle, éthyle, propyle, butyle, pentyle, isopropyle, isobutyle, sec-butyle, et *tert*-butyle, méthoxy, éthoxy, F, Cl, Br et I.

**22.** Procédé selon la revendication 1 dans lequel le sel pharmaceutiquement acceptable d'un composé de formule I est choisi dans le groupe consistant en

a) des sels basiques d'acides organiques et d'acides inorganiques choisis dans le groupe consistant en l'acide chlorhydrique, l'acide bromhydrique, l'acide sulfurique, l'acide phosphorique, l'acide méthanesulfonique, l'acide trifluorosulfonique, l'acide benzènesulfonique, l'acide p-toluène sulfonique (sel tolysate), l'acide 1-naphtalène sulfonique, l'acide 2-naphtalène sulfonique, l'acide acétique, l'acide trifluoroacétique, l'acide malique, l'acide tartarique, l'acide citrique, l'acide lactique, l'acide oxalique, l'acide succinique, l'acide fumarique, l'acide maléique, l'acide benzoïque, l'acide salicylique, l'acide phénylacétique et l'acide mandélique ; et
b) des sels acides de bases organiques et inorganiques contenant des cations choisis dans le groupe consistant en des cations alcalins, des cations alcalino-terreux, le cation ammonium, les cations ammonium aliphatiques substitués et les cations ammonium aromatiques substitués.

**23.** Procédé pour évaluer l'efficacité d'un composé pour traiter une maladie chez un sujet mammifère, ou une cellule dérivée de ce mammifère, comprenant :

la mesure de l'expression ou de l'activité de VEGFR-2, VEGFR-3, PDGFR-bêta, et/ou Flt-3 dans un échantillon prélevé sur ledit sujet qui a été traité avec ledit composé, et
la détermination des effets dudit composé sur ladite expression ou activité,
dans lequel ledit composé est :

N-(4-chloro-3-(trifluorométhyl)phényl)-N'-(4-(2-(N-méthylcarbamoyl)-4-pyridyloxy)phényl)urée,
N-(4-bromo-3-(trifluorométhyl)phényl)-N'-(4(2-(N-méthylcarbamoyl)-4-pyridyloxy)phényl)urée,
N-(4-bromo-3-(trifluorométhyl)phényl)-N'-(4-(2-(N-méthylcarbamoyl)-4-pyridyloxy)-2-chlorophényl) urée,
N-(4-chloro-3-(trifluorométhyl)phényl)-N'-(4-(2-carbamoyl-4-pyridyloxy)phényl)urée,
N-(4-chloro-3-(trifluométhyl)phényl)-N'-(4-(1 hydroxy-2-carbamoyl-4-pyridyloxy)phényl)urée,
N-(4-chloro-3-(trifluorométhyl)phényl)-N'-(4-(1-hydroxy-2-(N-méthylcarbamoyl)-4-pyridyloxy)phényl)urée,
N-(4-chloro-3-(trifluorométhyl)phényl)-N'-(4-(2-(N-méthylcarbamoyl)-4-pyridyloxy)-2-fluorophényl) urée,
N-(4-bromo-3-(trifluorométhyl)phényl)-N'-(4-(2-(N-méthylcarbamoyl)-4-pyridyloxy)-2-fluorophényl)urée,
N-(4-fluoro-3-(trifluorométhyl)phényl)-N'-(4-(2-(N méthylcarbamoyl)-4-pyridyloxy)-2-fluorophényl)urée,
N-(4-chloro-3-(trifluorométhyl)phényl)-N'-(4-(2-(N méthylcarbamoyl)-4-pyridyloxy)-2-chlorophényl)urée,
N-(6-(2,2,4,4-tétrafluoro-4H-benzo[1,3]dioxinyl))-N'-(4-(2-cyano-4-pyridyloxy)phényl)urée, ou
N-(6-(2,2,4,4-tetrafluoro-4H-benzo[1,3]dioxinyl))-N'-(4-(2-cyano)-4-pyridyloxy)-2-fluorophenyl) urée.

**24.** Procédé selon la revendication 1, dans lequel le composé de formule I est de formule X ci-dessous,

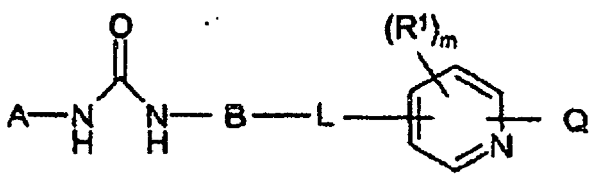

(X)

dans laquelle :

A est phényle, facultativement substitué 1, 2 ou 3 fois par $R^3$, où chaque $R^3$ est indépendamment alkyle $C_1$-$C_5$, haloalkyle $C_1$-$C_5$, jusqu'à perhaloalkyle, alcoxy $C_1$-$C_5$, haloalcoxy $C_1$-$C_5$, jusqu'à perhaloalcoxy, halogène, cyano ou nitro ; ou A est un groupe de formule :

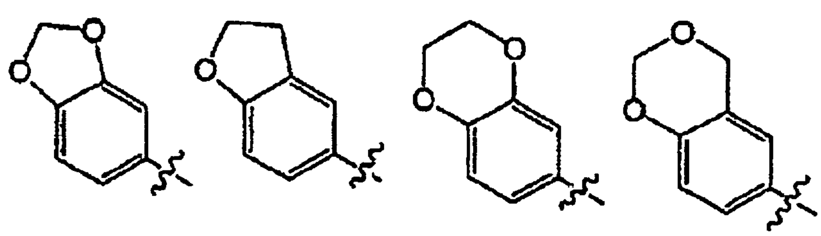

ou

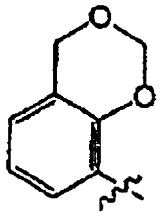

facultativement substitué 1, 2, 3, 4, 5 ou 6 fois avec $R^4$ où chaque $R^4$ est indépendamment alkyle $C_1$-$C_5$ ou halogène ;
B est phénylène, facultativement substitué 1, 2 ou 3 fois par $R^2$, ou naphtylène, facultativement substitué 1, 2 ou 3 fois par $R^2$, où $R^2$ est indépendamment alkyle $C_1$-$C_5$, haloalkyle $C_1$-$C_5$, jusqu'à perhaloalkyle, alcoxy $C_1$-$C_5$, haloalcoxy $C_1$-$C_5$ jusqu'à perhaloalcoxyle, halogène, cyano ou nitro ;
Q est cyano, -C(O)-$R^a$, ou -C(O)-$NR^bR^c$, où $R^a$, $R^b$ et $R^c$ sont chacun indépendamment H ou alkyle $C_1$-$C_5$,
L est -O- ou -S-,
m est 0, 1, 2 ou 3, et

chaque $R^1$ est indépendamment halogène, alkyle $C_1$-$C_5$, haloalkyle $C_1$-$C_5$, jusqu'à perhaloalkyle, alcoxy $C_1$-$C_5$, haloalcoxy $C_1$-$C_5$, jusqu'à perhaloalcoxy, N-oxo ou N-hydroxy.

**25.** Procédé selon la revendication 24 dans lequel, pour le composé de formule (X), chaque $R^2$ est indépendamment fluorure, chlorure, bromure, méthyle, éthyle, propyle, butyle, isopropyle, tert-butyle, trifluorométhyle, méthoxy, CN ou $NO_2$.

**26.** Procédé selon les revendications 24 ou 25 dans lequel, pour le composé de formule (X), chaque $R^3$ est indépendamment fluorure, chlorure, bromure, méthyle, éthyle, propyle, butyle, pentyl, isopropyle, iso-butyle, sec-butyle, tert-butyle, trifluorométhyle, méthoxy, éthoxy, trifluorométhoxy, CN ou $NO_2$ et chaque $R^4$ est indépendamment fluorure, chlorure, bromure ou méthyle.

**27.** Procédé selon l'une quelconque des revendications 24 - 26 dans lequel, pour le composé de formule (X), chaque $R^1$ est indépendamment méthyle, éthyle, propyle, oxygène, cyano, n-oxo ou n-hydroxy et chaque $R^a$, $R^b$ et $R^c$ est indépendamment H ou méthyle.

**28.** Procédé selon les revendications 24 - 27 dans lequel, pour le composé de formule (X), A est phényle substitué.

**29.** Procédé selon l'une quelconque des revendications 24 - 27 dans lequel, pour le composé de formule (X), A est un groupe de formule :

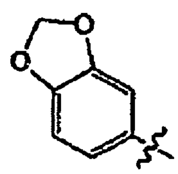

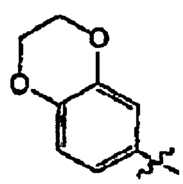

ou

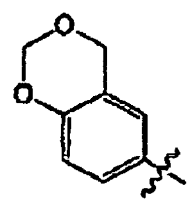

facultativement substitué 1, 2, 3 ou 4 fois avec $R^4$, où chaque $R^4$ est indépendamment chlorure ou fluorure.

**30.** Procédé selon l'une quelconque des revendications 24 - 29 dans lequel, pour le composé de formule (X), B est phénylène.

**31.** Procédé selon l'une quelconque des revendications 24 - 29 dans lequel, pour le composé de formule (X), B est naphtylène.

**32.** Procédé selon l'une quelconque des revendications 24 - 29 dans lequel, pour le composé de formule (X), B est phénylène substitué par au moins un atome fluorure.

**33.** Procédé selon l'une quelconque des revendications 24 - 32 dans lequel, pour le composé de formule (X), L est oxygène.

**34.** Procédé selon l'une quelconque des revendications 24 - 33 dans lequel, pour le composé de formule (X), chaque $R^3$ est chlorure, bromure, tert-butyle, trifluorométhyle ou méthoxy.

**35.** Procédé selon la revendication 24, dans lequel, pour le composé de formule (X), A est 4-chloro-3-trifluorométhylphényle, 4-fluoro-3-trifluorométhylphényle, 4-bromo-3-trifluorométhylphényle, ou 2,2,4,4-tétrafluoro-4H-benzo[1,3] dioxin-6-yle ;
B est phénylène, chlorophénylène ou fluorophénylène ;
L est -O- ;
et
Q est cyano, C(O)-$NH_2$, ou C(O)-NHMe.

**36.** Procédé selon l'une quelconque des revendications 24 - 35 dans lequel un sel basique pharmaceutiquement acceptable d'un acide organique d'un composé de formule (X) est utilisé.

**37.** Procédé selon les revendications 24 - 35 dans lequel un sel basique pharmaceutiquement acceptable d'un acide organique de formule (X) est administré, choisi parmi l'acide chlorhydrique, l'acide bromhydrique, l'acide sulfurique, l'acide phosphorique, l'acide méthanesulfonique, l'acide trifluorométhanesulfonique, l'acide benzènesulfonique, l'acide p-toluène sulfonique (sel tolysate), l'acide 1-naphtalène sulfonique, l'acide 2-naphtalène sulfonique, l'acide acétique, l'acide trifluoroacétique, l'acide malique, l'acide tartarique, l'acide citrique, l'acide lactique, l'acide oxalique, l'acide succinique, l'acide fumarique, l'acide maléique, l'acide benzoïque, l'acide salicylique, l'acide phénylacétique et l'acide mandélique.

**38.** Procédé selon la revendication 24 dans lequel le composé de formule X est un sel chlorhydrate, benzènesulfonate ou méthanesulfonate pharmaceutiquement acceptable de la
N-(4-chloro-3-(trifluorométhyl)phényl)-N'-2-fluoro-(4-(2-(N-méthylcarbamoyl)-4-pyridyloxy)phényl)urée ou de la
N-(4-chloro-3-(trifluorométhyl)phényl)-N'-(4-(2-(N-méthylcarbamoyl)-4-pyridyloxy)phényl)urée.

**39.** Procédé pour évaluer l'efficacité d'un composé dans le traitement d'une maladie chez un sujet mammifère, ou une cellule dérivée de celui-ci, comprenant :

la mesure de l'expression ou de l'activité de VEGFR-2, VEGFR-3, PDGFR-bêta, et/ou Flt-3 dans un échantillon obtenu sur ledit sujet traité avec ledit composé, et
la détermination des effets dudit composé sur ladite expression ou activité,
dans lequel ledit composé est
un composé aryle urée de formules X, Y, ZA, ZB, ZC ou ZD,
une forme de sel d'un composé de formules X, Y, ZA, ZB, ZC ou ZD,
un stéréoisomère isolé ou en mélange d'un composé de formules X, Y, ZA, ZB, ZC ou ZD,
un ester d'un composé de formules X, Y, ZA, ZB, ZC ou ZD,
un métabolite d'un composé de formules X, Y, ZA, ZB, ZC ou ZD, ou
un promédicament d'un composé de formules X, Y, ZA, ZB, ZC ou ZD,

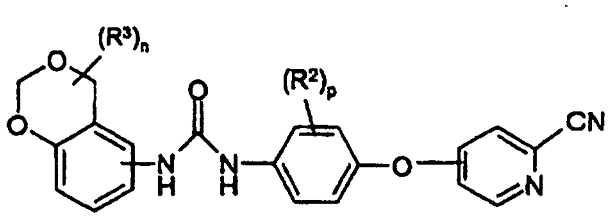

X

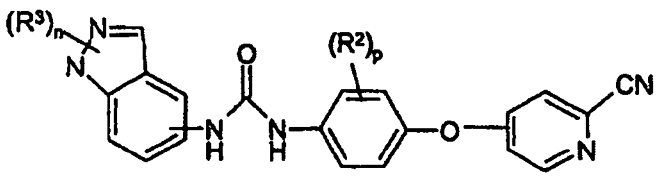

Y

,

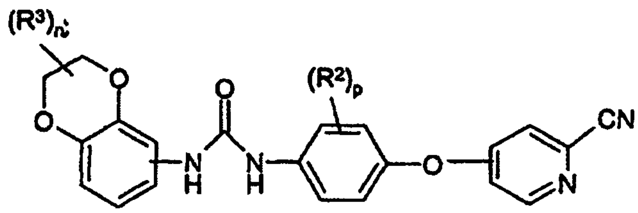

ZA

,

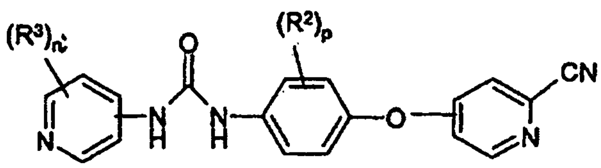

ZB

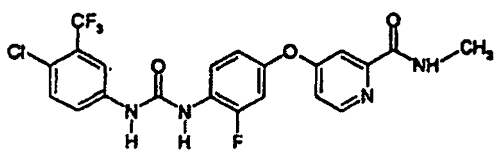

ZC

et

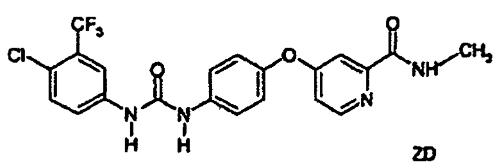

ZD

où

chaque $R^3$ est indépendamment halogène ou trifluorométhyle et

chaque $R^2$ est indépendamment fluorure, chlorure, bromure, méthyle, trifluorométhyle, méthoxy, CN ou $NO_2$,

la variable n est 0, 1, 2, 3 ou 4 et

la variable p est 0, 1 ou 2.

Fig. 1

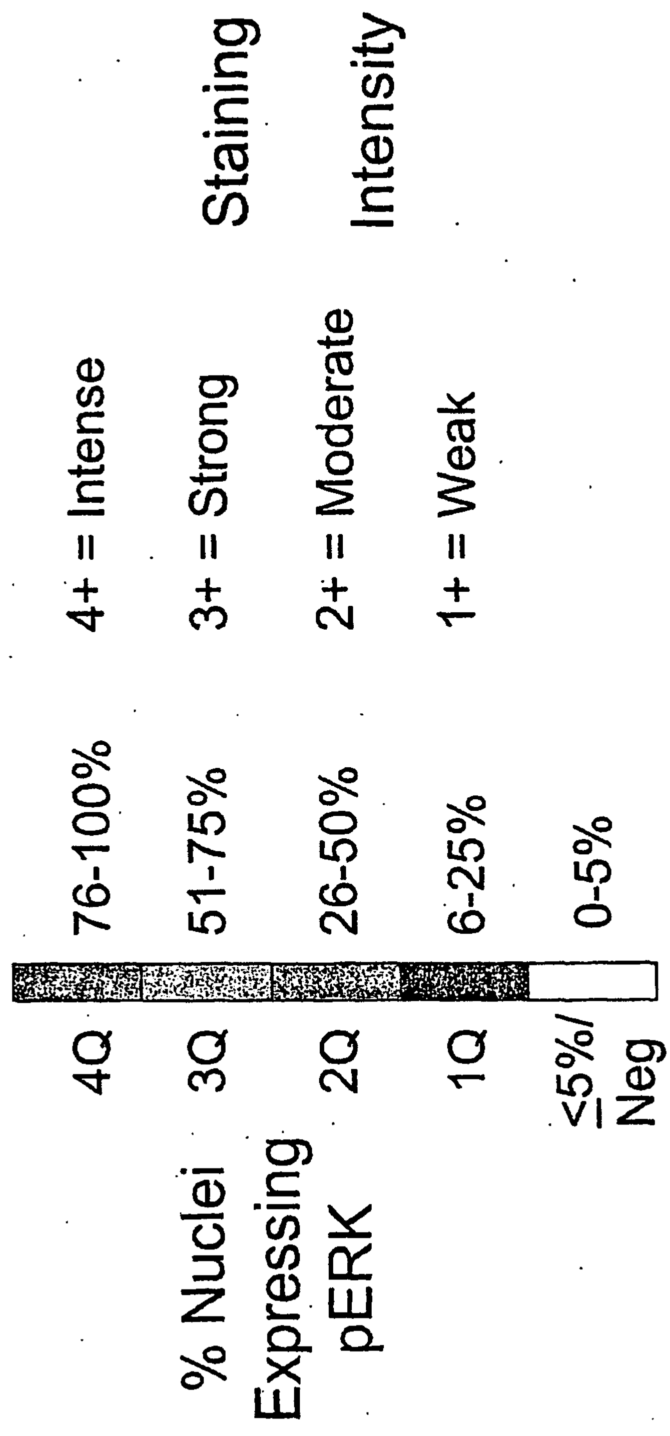
% Nuclei Expressing pERK
4Q 76-100%
3Q 51-75%
2Q 26-50%
1Q 6-25%
≤5%/ Neg 0-5%
4+ = Intense
3+ = Strong
2+ = Moderate
1+ = Weak
Staining Intensity

Fig. 2

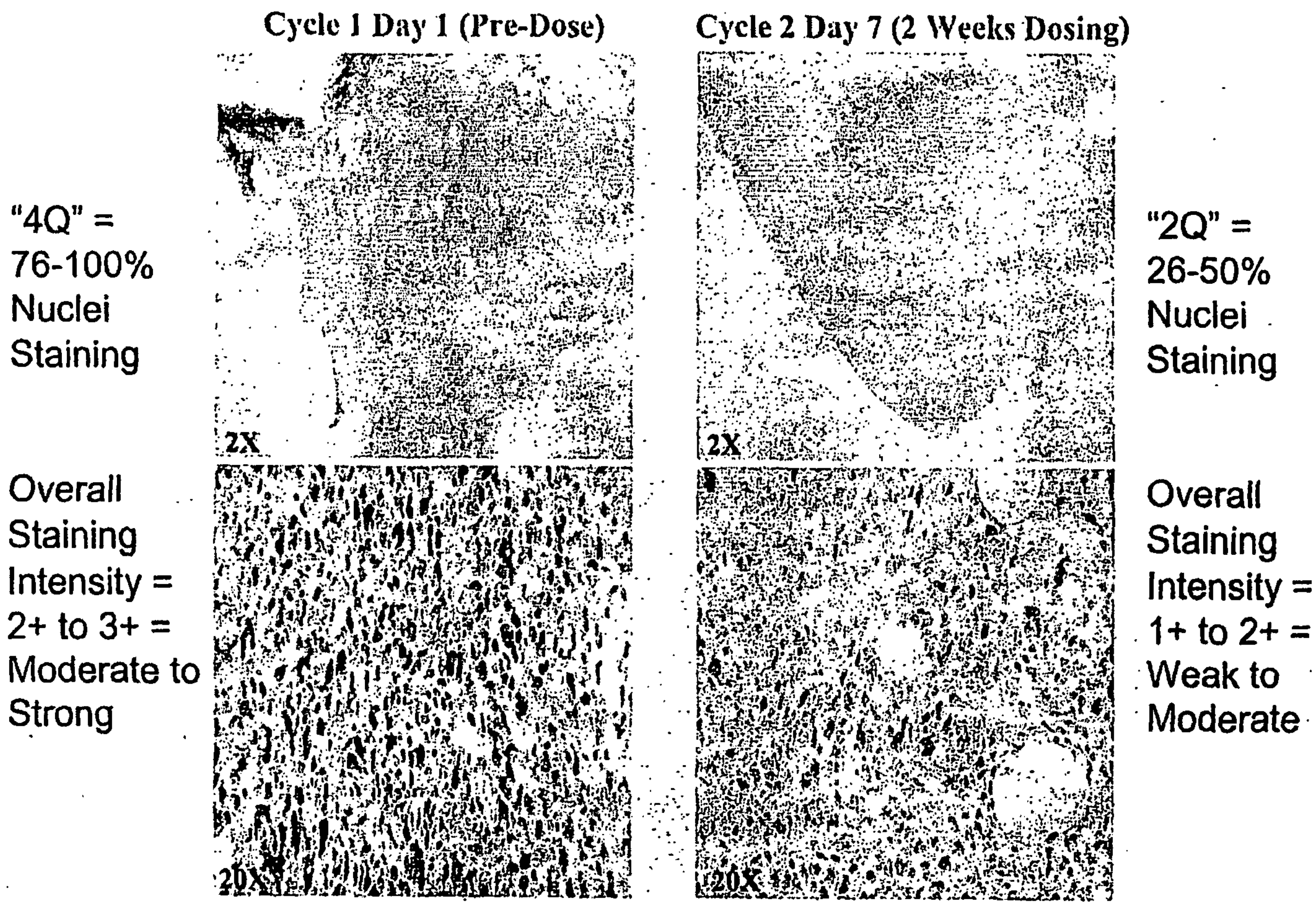
Cycle 1 Day 1 (Pre-Dose)
Cycle 2 Day 7 (2 Weeks Dosing)
"4Q" = 76-100% Nuclei Staining
Overall Staining Intensity = 2+ to 3+ = Moderate to Strong
"2Q" = 26-50% Nuclei Staining
Overall Staining Intensity = 1+ to 2+ = Weak to Moderate
2X
2X
20X
20X

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 55606204 P
- US 60520399 B
- US 60471735 B
- US 6316479 B
- US 5192744 A
- US 6024688 A
- US 6060449 A
- US 6017949 A
- US 4683195 A
- US 4683202 A
- US 6040166 A
- EP 320308 A
- US 5508169 A
- US 5262311 A
- US 5599672 A
- US 5965409 A
- WO 9718454 A
- US 6010850 A
- US 5712126 A
- US 5487985 A
- US 5409818 A
- US 5554527 A
- WO 8810315 A
- US 5143854 A
- US 5424186 A
- US 5700637 A
- US 5874219 A
- US 6054270 A
- WO 9210092 A
- WO 9015070 A
- US 8700880 W
- US 5747251 A
- US 5871918 A
- US 5210015 A
- US 5994063 A
- US 5928907 A
- US 5348853 A
- US 5532129 A
- US 5565322 A
- US 6030787 A
- US 6117635 A
- US 5723290 A
- US 6214556 B
- WO 9404157 A
- US 5023252 A
- US 5011472 A
- WO 0042012 A
- WO 0041698 A
- US 09425228 B
- US 09722418 B
- US 09758547 B
- US 09838285 B
- US 09838286 B
- WO 9800402 A
- EP 542609 A
- DE 2436108
- JP 42025067 B
- US 09948915 B
- US 20020042517 A
- WO 200041698 A
- WO 200285859 A
- WO 200285857 A
- WO 9533772 A
- WO 9533050 A
- WO 9639421 A
- WO 9833917 A
- WO 02057299 A
- WO 02060950 A
- WO 02081520 A
- EP 0882799 A
- EP 1167384 A
- EP 1086705 A
- EP 11300032 A
- EP 1166798 A
- EP 1166799 A
- EP 1170017 A
- EP 1203827 A
- WO 02083850 A
- EP 0627487 A
- WO 9846750 A
- WO 9818923 A
- WO 9428391 A
- WO 9426891 A
- WO 02083849 A
- WO 02083704 A
- WO 02079498 A
- WO 02070008 A
- WO 09959636 A
- WO 09946364 A
- WO 09940118 A
- WO 9931238 A
- WO 9929861 A
- WO 9858053 A
- WO 9851344 A
- WO 9831794 A
- WO 9816551 A
- WO 9813071 A
- WO 9811223 A

- WO 9744453 A
- WO 9723510 A
- WO 9715662 A
- WO 9708313 A
- WO 9639515 A
- WO 9623065 A
- WO 9606641 A
- WO 9524473 A
- WO 9822316 A
- WO 952186 A
- WO 02060489 A
- EP 0869177 A
- WO 09010013 A
- WO 9737029 A
- EP 1000617 A
- EP 0811685 A

**Non-patent literature cited in the description**


- **Daum et al.** *Trends Biochem. Sci.,* 1994, vol. 19, 474-80
- **Fridman et al.** *J. Biol. Chem.,* 1994, vol. 269, 30105-8
- **Kolch et al.** *Nature,* 1991, vol. 349, 426-28
- **Naumann, U. ; Eisenmann-Tappe, I. ; Rapp, U. R.** *Recent Results Cancer Res.,* 1997, vol. 143, 237
- **Monia, B. P. ; Johnston, J. F. ; Geiger, T. ; Muller, M. ; Fabbro, D.** *Nature Medicine,* 1996, vol. 2, 668
- **Folkman, J.** *Semin Oncol,* 2002, vol. 29 (16), 15-8
- **Ostman, A. ; C.H. Heldin.** *Adv Cancer Res,* 2001, vol. 80, 1-38
- **Kourembanas, S. et al.** *Kidney Int,* 1997, vol. 51 (2), 438-43
- **Heldin, C.H. ; A. Ostman ; L. Ronnstrand.** *Biochim Biophys Acta,* vol. 1378 (1), 79-113
- **Heldin, C.H. et al.** *Embo J,* 1988, vol. 7 (5), 1387-93
- **Soskic, V. et al.** *Biochemistry,* 1999, vol. 38 (6), 1757-64
- **Baker, E.A. ; D.J. Leaper.** *Wound Repair Regen,* 2000, vol. 8 (5), 392-8
- **Yu, J., A. Moon ; H.R. Kim.** *Biochem Biophys Res Commun,* 2001, vol. 282 (3), 697-700
- **Pietras, K et al.** *Cancer Res,* 2002, vol. 62 (19), 5476-84
- **Pietras, K. et al.** *Cancer Res,* 2001, vol. 61 (7), 2929-34
- **Forsberg, K. et al.** *Proc Natl Acad Sci USA.,* 1993, vol. 90 (2), 393-7
- **Skobe, M. ; N.E. Fusenig.** *Proc Natl Acad Sci USA,* 1998, vol. 95 (3), 1050-5
- **Bhardwaj, B. et al.** *Clin Cancer Res,* 1996, vol. 2 (4), 773-82
- **Nakanishi, K. et al.** *Mod Pathol,* 1997, vol. 10 (4), 341-7
- **Sundberg, C. et al.** *Am J Pathol,* 1997, vol. 151 (2), 479-92
- **Lindmark, G. et al.** *Lab Invest,* 1993, vol. 69 (6), 682-9
- **Vignaud, J.M. et al.** *Cancer Res,* 1994, vol. 54 (20), 5455-63
- **Fleming, T.P. et al.** *Cancer Res,* 1992, vol. 52 (16), 4550-3
- **Wang, J. ; M.D. Coltrera ; A.M. Gown.** *Cancer Res,* 1994, vol. 54 (2), 560-4
- **Henriksen, R. et al.** *Cancer Res,* 1993, vol. 53 (19), 4550-4
- **Fudge, K. ; C.Y. Wang ; M.E. Stearns.** *Mod Pathol,* 1994, vol. 7 (5), 549-54
- **Funa, K. et al.** *Cancer Res,* 1990, vol. 50 (3), 748-53
- **Antoniades, H.N. et al.** *Proc Natl Acad Sci USA,* 1992, vol. 89 (9), 3942-6
- **Heinrich; M.C. et al.** *Science,* 2003, vol. 9, 9
- **Farrara et al.** *Endocr. Rev.,* 1992, vol. 13, 18
- **Neufield et al.** *FASEB J.,* 1999, vol. 13, 9
- **Shweiki et al.** *Nature,* 1992, vol. 359, 843
- **Mustonen et al.** *J. Cell Biol.,* 1995, vol. 129, 895
- **Waltenberger et al.** *J. Biol. Chem.,* 1994, vol. 269, 26988
- **Park et al.** *Oncogene,* 1995, vol. 10, 13
- **Shweiki et al.** *Proc. Nat'l. Acad. Sci.,* 1995, vol. 92, 768
- **Grugel et al.** *J. Biol. Chem.,* 1995, vol. 270, 25915
- **Rak et al.** *Cancer Res.,* 1995, vol. 55, 4575
- **Mattern et al.** *Br. J. Cancer,* 1996, vol. 73, 931
- **Viglietto et al.** *Oncogene,* 1995, vol. 11, 1569
- **Brown et al.** *Human Pathol.,* 1995, vol. 26, 86
- **Brown et al.** *Cancer Res.,* 1993, vol. 53, 4727
- **Suzuki et al.** *Cancer Res.,* 1996, vol. 56, 3004
- **Brown et al.** *Am. J. Pathol.,* 1993, vol. 1431, 1255
- **Olson et al.** *Cancer Res.,* 1994, vol. 54, 1255
- **Guidi et al.** *J. Nat'l Cancer Inst.,* 1995, vol. 87, 12137
- **Hashimoto et al.** *Lab. Invest.,* 1995, vol. 73, 859
- **Plate et al.** *Nature,* 1992, vol. 359, 845
- **Phillips et al.** *Int. J. Oncol.,* 1993, vol. 2, 913
- **Berkman et al.** *J. Clin. Invest.,* 1993, vol. 91, 153
- **Kim et al.** *Nature,* 1993, vol. 362, 841
- **Rockwell et al.** *Mol. Cell. Differ.,* 1995, vol. 3, 315

- **Aiello et al.** *New Engl. J. Med.,* 1994, vol. 331, 1480
- **Peer et al.** *Lab. Invest.,* 1995, vol. 72, 638
- **Lopez et al.** *Invest. Opththalmol. Vis. Sci.,* 1996, vol. 37, 855
- **Koch et al.** *J. Immunol.,* 1994, vol. 152, 4149
- **Peacock et al.** *J. Exper. Med.,* 1992, vol. 175, 1135
- **McMahon, G.** *Oncologist,* 2000, vol. 5 (1), 3-10
- **McDonald, N.Q. ; Hendrickson, W.A.** *Cell,* 1993, vol. 73, 421-424
- **Skobe, M. et al.** *Nature Med.,* 2001, vol. 7 (2), 192-198
- **Stacker, S.A. et al.** *Nature Med.,* 2001, vol. 7 (2), 186-191
- **Makinen, T. et al.** *Nature Med.,* 2001, vol. 7 (2), 199-205
- **Mandriota, S.J. et al.** *EMBO J.,* 2001, vol. 20 (4), 672-82
- **Karpanen, T. et al.** *Cancer Res.,* 2001, vol. 61 (5), 1786-90
- **Kubo, H. et al.** *Blood,* 2000, vol. 96 (2), 546-53
- **Blaschke, F. et al.** *Biochem. Biophys. Res. Commun.,* 2002, vol. 296, 890-896
- **Shemirani, B. et al.** *Oral Oncology,* 2002, vol. 38, 251-257
- **Laferriere, J. et al.** *J. Biol. Chem.,* 2001, vol. 276, 33762-33772
- **Westermarck, J. et al.** *Cancer Res.,* 2000, vol. 60, 7156-7162
- **Huang, S. et al.** *J. Biol. Chem.,* 2000, vol. 275, 12266-12272
- **Simon, C. et al.** *Exp. Cell Res.,* 2001, vol. 271, 344-355
- **Redman et al.** *Bioorg. Med. Chem. Lett.,* 2001, vol. 11, 9-12
- **Smith et al.** *Bioorg. Med. Chem. Lett.,* 2001, vol. 11, 2775-2778
- **Dumas et al.** *Bioorg. Med. Chem. Lett.,* 2000, vol. 10, 2047-2050
- **Dumas et al.** *Bioorg. Med. Chem. Lett.,* 2000, vol. 10, 2051-2054
- **Ranges et al.** Book of Abstracts, 220th ACS National Meeting
- **Dumas et al.** *Bioorg. Med. Chem. Lett.,* 2002, vol. 12, 1559-1562
- **Lowinger et al.** *Clin. Cancer Res.,* 2000, vol. 6, 335
- **Lyons et al.** *Endocr.-Relat. Cancer*
- **Riedl et al.** Book of Abstracts, 92nd AACR Meeting
- **Khire et al.** Book of Abstracts, 93rdAACR Meeting
- **Lowinger et al.** *Curr. Pharm. Design,* 2002, vol. 8, 99-110
- **Regan et al.** *J. Med. Chem.,* 2002, vol. 45, 2994-3008
- **Pargellis et al.** *Nature,* 2002, vol. 9 (4), 268-272
- **Carter et al.** Book of Abstracts ; 92ndAACR Meeting
- **Vincent et al.** Book of Abstracts, 38th ASCO Meeting
- **Hilger et al.** Book of Abstracts, 38th ASCO Meeting
- **Moore et al.** Book of Abstracts, 38th ASCO Meeting
- **Strumberg et al.** Book ofAbstracts, 38th ASCO Meeting
- **Madwed JB.** Book of Abstracts, Protein Kinases: Novel Target Identification and Validation for Therapeutic Development
- **Roberts et al.** Book ofAbstracts, 38th ASCO Meeting
- **Tolcher et al.** Book ofAbstracts, 38th ASCO Meeting
- **Karp et al.** Book of Abstracts, 38th AACR Meeting
- **S. M. Berge et al.** Pharmaceutical Salts. *J. Pharm. Sci.,* 1977, vol. 66, 1-19
- Pharmaceutical Dosage Form and Drug Delivery Systems. Williams & Wilkins, 1995, 27-29
- **Goodman ; Gilman's et al.** The Pharmacological Basis of Therapeutics. McGraw-Hill, 1996, 11-13
- **Manley et al.** *J. Med. Chem.,* 2002, vol. 45 (26), 5687-93
- **Stahl et al.** *Angew Chem. Int. Ed. Engl.,* 2002, vol. 41 (7), 1174-8
- **Yee et al.** *Blood,* 2002, vol. 100 (8), 2941-9
- **Kelly et al.** *Cancer Cell,* 2002, vol. 1 (5), 421-32
- **Hamrah.** *Am. J. Path.,* 2003, vol. 163, 57-68
- **Cursiefen et al.** *Cornea,* 2003, vol. 22, 273-81
- **Taylor ; Folkman.** *Nature,* 1982, vol. 297, 307-312
- **Eliceiri et al.** *J. Cell Biol.,* 1998, vol. 140, 1255-1263
- **Polverini, P. J. et al.** *Methods Enzymol.,* 1991, vol. 198, 440-450
- **Szuba et al.** *FASEB J.,* 2002, vol. 16 (14), 1985-7
- **Penfold et al.** *Br. J. Oral and Maxill. Surg.,* vol. 34, 37-41
- **Dellas et al.** *Gyn. Oncol,* 1997, vol. 67, 27-33
- **Xiang et al.** *Dev. Bio.,* 1999, vol. 206, 123-141
- **Sato et al.** *Nature,* 1995, vol. 376, 70-74
- **Saiki et al.** *Science,* 1988, vol. 241, 53
- **Innis et al.** PCR Protocols: A Guide to Methods and Applications. Academic Press, 1990
- **Schaefer.** *Gene Cloning and Analysis: Current Innovations,* 1997, 99-115

- **Ohara et al.** *Proc. Natl. Acad. Sci.,* 1989, vol. 86, 5673-5677
- **Liang et al.** *Nucl. Acid. Res.,* 1993, vol. 21, 3269-3275
- **Prashar ; Weissman.** *Proc. Natl. Acad. Sci.,* vol. 93, 659-663
- **Welsh et al.** *Nucleic Acid Res.,* 1992, vol. 20, 4965-4970
- **Holland et al.** *Proc. Natl. Acad, Sci.,* 1991, vol. 88, 7276-7280
- **Tyagi ; Kramer.** *Nature Biotech.,* 1996, vol. 14, 303-309
- **Brady et al.** *Methods Mol. & Cell. Biol.,* 1990, vol. 2, 17-25
- **Eberwine et al.** *Proc. Natl. Acad. Sci.,* 1992, vol. 89, 3010-3014
- Goodman and Gilman's The Pharmacological Basis of Therapeutics. McGraw-Hill, 1996, 1225-1287
- **J. March.** Advanced Organic Chemistry. John Wiley, 1992
- **R.C. Larock.** Comprehensive Organic Transformations. Wiley-VCH, 1999
- **F.A. Carey ; R.J. Sundberg.** Advanced Organic Chemistry. Plenum Press, 1984
- **T.W. Greene ; P.G.M. Wuts.** Protective Groups in Organic Synthesis. John Wiley, 1999
- **L.S. Hegedus.** Transition Metals in the Synthesis of Complex Organic Molecules. University Science Books, 1994
- The Encyclopedia of Reagents for Organic Synthesis. John Wiley, 1994
- Comprehensive Organic Functional Group Transformations. Pergamon Press, 1995
- Comprehensive Organometallic Chemistry. Pergamon Press, 1982
- **B.M. Trost ; I. Fleming.** Comprehensive Organic Synthesis. Pergamon Press, 1991
- Comprehensive Heterocylic Chemistry. Pergamon Press, 1984
- Comprehensive Heterocylic Chemistry II. Pergamon Press, 1996
- Comprehensive Medicinal Chemistry. Pergamon Press, 1990
- Organic Reactions. John Wiley
- Organic Syntheses. John Wiley
- Reagents for Organic Synthesis. John Wiley
- The Total Synthesis of Natural Products. John Wiley
- The Organic Chemistry of Drug Synthesis. John Wiley
- Annual Reports in Organic Synthesis. Academic Press
- Methoden der Organischen Chemie. Thieme
- **March.** Advanced Organic Chemistry. John Wiley, 1985
- **Larock.** Comprehensive Organic Transformations. VCH Publishers, 1989
- **Rylander.** Hydrogenation Methods. Academic Press, 1985
- **Seyden-Penne.** Reductions by the Alumino- and Borohydrides in Organic Synthesis. VCH Publishers, 1991
- **Chien.** Transdermal Controlled Systemic Medications. Marcel Dekker, Inc, 1987
- **Powell, M.F. et al.** Compendium of Excipients for Parenteral Formulations. *PDA Journal of Pharmaceutical Science & Technology,* 1998, vol. 52 (5), 238-311
- **Strickley, R.G.** Parenteral Formulations of Small Molecule Therapeutics Marketed in the United States (1999)-Part-1. *PDA Journal of Pharmaceutical Science & Technology,* 1999, vol. 53 (6), 324-349
- **Nema, S. et al.** Excipients and Their Use in Injectable Products. *PDA Journal of Pharmaceutical Science & Technology,* 1997, vol. 51 (4), 166-171
- **Powell, M.F. et al.** *PDA Journal of Pharmaceutical Science & Technology,* 1998, vol. 52 (5), 238-311
- *IBID Bioorg. Med Chem.,* 1995, vol. 3, 129
- **K. Ikawa.** *Yakugaku Zasshi,* 1959, vol. 79, 760
- *CHEMICAL ABSTRACTS,* 53
- *IBID Kogyo Kagaku Zasshi,* 1967, vol. 70, 491
- *General Methods for Synthesis of Substituted Anilines,* 18-43
- *Synthesis of Urea Precursors,* 43
- *Methods of Urea Formation,* 44-51
- *Interconversion of Ureas,* 52-56